(19)

Europäisches
Patentamt
European
Patent Office
Office européen
des brevets

(11)     **EP 2 399 129 B1**

(12)     **EUROPEAN PATENT SPECIFICATION**

(45) Date of publication and mention
of the grant of the patent:
**25.11.2015 Bulletin 2015/48**

(21) Application number: **10744303.8**

(22) Date of filing: **18.02.2010**

(51) Int Cl.:
***G01N 33/574*** *(2006.01)*

(86) International application number:
**PCT/US2010/024606**

(87) International publication number:
**WO 2010/096574 (26.08.2010 Gazette 2010/34)**

(54) **A METHOD OF DIAGNOSIS OR PROGNOSIS OF A NEOPLASM COMPRISING DETERMINING THE LEVEL OF EXPRESSION OF A PROTEIN IN STROMAL CELLS ADJACENT TO THE NEOPLASM**

VERFAHREN ZUR DIAGNOSE BZW. PROGNOSE EINES NEOPLASMAS EINSCHLIESSLICH DER BESTIMMUNG DES EXPRESSIONSGRADS EINES PROTEINS IN STROMAZELLEN NEBEN DEM NEOPLASMA

PROCÉDÉ DE DIAGNOSTIC OU DE PRONOSTIC D'UN NÉOPLASME COMPRENANT LA DÉTERMINATION DU TAUX D'EXPRESSION D'UNE PROTÉINE DANS DES CELLULES STROMALES ADJACENTES AU NÉOPLASME

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **20.02.2009 US 154187 P**

(43) Date of publication of application:
**28.12.2011 Bulletin 2011/52**

(73) Proprietors:
 • **Lisanti, Michael, P.**
   **Philadelphia, Pennsylvania 19147 (US)**
 • **Sotgia, Federica**
   **Philadelphia, Pennsylvania 19147 (US)**
 • **Pestell, Richard G.**
   **Philadelphia, Pennsylvania 19147 (US)**

(72) Inventors:
 • **Lisanti, Michael, P.**
   **Philadelphia, Pennsylvania 19147 (US)**
 • **Sotgia, Federica**
   **Philadelphia, Pennsylvania 19147 (US)**
 • **Pestell, Richard G.**
   **Philadelphia, Pennsylvania 19147 (US)**

(74) Representative: **Vossius & Partner**
**Patentanwälte Rechtsanwälte mbB**
**Siebertstrasse 3**
**81675 München (DE)**

(56) References cited:
**WO-A2-02/30973     WO-A2-2008/067065**

 • **ISABELLE MERCIER ET AL: "Human breast cancer-associated fibroblasts (CAFs) show caveolin-1 down-regulation and RB tumor suppressor functional inactivation: Implications for the response to hormonal therapy", CANCER BIOLOGY & THERAPY, vol. 7, no. 8, 1 August 2008 (2008-08-01), pages 1212-1225, XP55014714, ISSN: 1538-4047, DOI: 10.4161/cbt.7.8.6220**
 • **WITKIEWICZ AGNIESZKA K ET AL: "An Absence of Stromal Caveolin-1 Expression Predicts Early Tumor Recurrence and Poor Clinical Outcome in Human Breast Cancers", AMERICAN JOURNAL OF PATHOLOGY; [10640], AMERICAN SOCIETY FOR INVESTIGATIVE PATHOLOGY, US, vol. 174, no. 6, 1 June 2009 (2009-06-01), pages 2023-2034, XP009154807, ISSN: 0002-9440, DOI: 10.2353/AJPATH.2009.080873**
 • **Agnieszka K Witkiewicz ET AL: "Towards a new "stromal-based" classification system for human breast cancer prognosis and therapy", Cell Cycle, 1 June 2009 (2009-06-01), pages 1654-1658, XP55028335, Retrieved from the Internet: URL:http://www.landesbioscience.com/journa ls/cc/WitkiewiczCC8-11.pdf [retrieved on 2012-05-29]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

**EP 2 399 129 B1**

- MERCIER, 1. ET AL.: 'Human breast cancer-associated fibroblasts (CAFs) show caveolin-1 downregulation and RB tumor suppressor functional inactivation' CANCER BIOLOGY & THERAPY vol. 7, no. 8, 2008, pages 1212 - 1225, XP055014714
- WITKIEWICZ, A. K. ET AL.: 'Loss of Caveolin-1 Expression in Breast Cancer Associated Fibroblasts Correlates with Tumor Aggressiveness' LABORATORY INVESTIGATION vol. 89, no. SUPP., January 2009, pages 74A - 75A
- ANDERSON, R. L. ET AL.: 'Stromal expression of caveolin-1 regulates breast cancer progression' JOURNAL OF BONE AND MINERAL RESEARCH vol. 19, no. 9, 2004, page 1580
- WITKIEWICZ, A. K. ET AL.: 'An Absence of Stromal Caveolin-1 Expression Predicts Early Tumor Recurrence and Poor Clinical Outcome in Human Breast Cancers' THE AMERICAN JOURNAL OF PATHOLOGY vol. 174, no. 6, June 2009, pages 2023 - 2034, XP009154807
- SLOAN, E. K. ET AL.: 'Stromal Cell Expression of Caveolin-1 Predicts Outcome in Breast Cancer' THE AMERICAN JOURNAL OF PATHOLOGY vol. 174, no. 6, June 2009, pages 2035 - 2043, XP002673739
- WITKIEWICZ, A. K. ET AL.: 'Towards a new "stromal-based" classification system for human breast cancer prognosis and therapy' CELL CYCLE vol. 8, no. 11, June 2009, pages 1654 - 1658, XP055028335
- DI VIZIO, D. ET AL.: 'An absence of stromal caveolin-1 is associated with advanced prostate cancer, metastatic disease and epithelial Akt activation' CELL CYCLE vol. 8, no. 15, August 2009, pages 2420 - 2424, XP055070023
- PAVLIDES, S. ET AL.: 'The reverse Warburg effect: Aerobic glycolysis in cancer associated fibroblasts and the tumor stroma' CELL CYCLE vol. 8, no. 23, December 2009, pages 3984 - 4001, XP009158918
- WITKIEWICZ, A. K. ET AL.: 'Stromal caveolin-1 levels predict early DCIS progression to invasive breast cancer' CANCER BIOLOGY & THERAPY vol. 8, no. 11, June 2009, pages 1071 - 1079, XP055070024
- KAWASE, A. ET AL.: 'Podoplanin expression by cancer associated fibroblasts predicts poor prognosis of lung adenocarcinoma' INT. J. CANCER vol. 123, 2008, pages 1053 - 1059, XP055070025

**Description**

BACKGROUND OF THE INVENTION

1. FIELD OF INVENTION

This invention relates to stromal caveolin-1 and/or caveolin-2 that serves as a new cancer biomarker that can be used to predict early tumor recurrence and clinical outcome across many different "subclasses" of cancer. Thus, the status of the tumor stroma is a primary determinant of disease recurrence and poor clinical outcome in cancer patients.

ISABELLE MERCIER ET AL: "Human breast cancer-associated fibroblasts (CAFs) show caveolin-1 down-regulation and RB tumor suppressor functional inactivation: Implications for the response to hormonal therapy", CANCER BIOLOGY & THERAPY, vol. 7, no. 8, 1 August 2008 (2008-08-01), pages 1212-1225,

2. DESCRIPTION OF RELATED ART

Carcinoma cells grow in a complex tumor micro-environment composed of (i) nonepithelial cells (including fibroblasts, pericytes, endothelial, and inflammatory cells), (ii) extracellular matrix, and (iii) secreted diffusible growth factors/cytokines. Although under normal physiologic conditions the stroma serves as an important barrier to malignant transformation, its role changes during neoplastic transformation. Instead, the stroma plays a key role in driving cancer cell invasiveness and progression. Recently, it was demonstrated that fibroblasts isolated from tumor stroma can promote tumor growth. This population of tissue fibroblasts termed "cancer associated fibroblasts" (CAFs) is characterized by a hyper-proliferative phenotype and these cells secrete increased amounts of growth factors, extracellular matrix components, and matrix metalloproteinases (MMPs). CAFs also show an ability to prevent cancer cell apoptosis, induce cancer cell proliferation, as well as stimulate tumor angiogenesis. In vitro studies of breast carcinomas showed that CAFs mixed with epithelial carcinoma cells are more proficient than normal fibroblasts in enhancing tumor-growth and give rise to highly vascularized tumors. To date, the mechanisms that govern the conversion of benign mammary stromal fibroblasts to tumor-associated fibroblasts are poorly understood and their relationship to disease outcome has not been addressed.

Down-regulation of caveolin-1 (Cav-1) and/or caveolin-2 (Cav-2) is one of the mechanisms implicated in the oncogenic transformation of fibroblasts. Caveolins are the principal protein component of caveolae, which are located at the ceU surface in most cell types. One of the caveolins, Cav-1, plays a major role in tumorigenesis through its various functions such as lipid transport, membrane trafficking, gene regulation, and signal transduction. In cell culture, the transformation of NIH-3T3 fibroblasts with various activated oncogenes, such as H-Ras (G12V), Bcr-Abl or v-Abl, causes dramatic reductions in Cav-1 protein expression.

Furthermore, knock-down of endogenous Cav-1 in NIH-3T3 fibroblasts promotes anchorage-independent growth in soft agar and tumor formation in nude mice, which could be reversed by Cav-1 re-expression. Finally, Cav-1 (-/-) null fibroblasts have a hyper-proliferative phenotype (similar to CAFs) and Cav-1 re-expression drives their arrest in the G0/G1 phase of the cell cycle. Taken together, these data suggest that loss of Cav-1 leads to the oncogenic transformation of fibroblasts, where Cav-1 normally functions as a transformation suppressor that prevents cell cycle progression. Using primary cell cultures established from surgically excised breast tumors, we recently demonstrated that Cav-1 is down-regulated in human breast cancer-associated fibroblasts (CAF) when compared to matching normal fibroblasts isolated from the same patient. In addition, orthotopic transplantation of Cav-1 (+/+) tumor tissue into the mammary stroma of Cav-1 (-/-) null mice results in up to a ~2-fold increase in tumor mass, functionally demonstrating that the mammary stroma of Cav-1 (-/-) mice behaves as a tumor promoter. However, to date, there is no study addressing the clinical significance of stromal Cav-1 expression in invasive carcinoma of the breast *in vivo*.

The Inventors evaluated the *in vivo* stromal expression of Cav-1 in a large series of invasive breast carcinomas and to examine the association between stromal Cav-1 expression, clinico-pathological variables, and patient outcome. Our results indicate that loss of stromal caveolin-1 is a novel.breast cancer biomarker that predicts early disease recurrence, metastasis, survival, and tamoxifen-resistance. Clinical outcome in HER2(+) and triple-negative (ER-/PR-/HER2-) patients was also strictly dependent on stromal Cav-1 levels. Remarkably, in lymph node-positive (LN(+)) patients, an absence of stromal Cav-1 was associated with an ~11.5-fold reduction in 5-year progression-free survival. As such, Cav-1 functions as a critical tumor/metastasis suppressor in the mammary stromal compartment.

Previously, we showed that caveolin-1 (Cav-1) expression is down-regulated in human breast cancer-associated fibroblasts. However, it remains unknown whether loss of Cav-1 expression occurs in the breast tumor stroma in vivo. Here, we immunostained a well-annotated breast cancer tissue microarray with antibodies directed against Cav-1, and scored its stromal expression. An absence of stromal Cav-1 immunostaining was associated with early disease recurrence, advanced tumor stage, and lymph node metastasis, resulting in an ~3.6-fold reduction in progression-free survival. When tamoxifen-treated patients were selected, an absence of stromal Cav-1 was a strong predictor of poor clinical outcome, suggestive of tamoxifen-resistance. Interestingly, in lymph-node positive patients, an absence of stromal Cav-1 predicted an ~11.5-fold reduction in 5-year progression-free survival. Clinical outcome in HER2(+) and triple negative (ER-/PR-/HER2-) patients was also strictly dependent on stromal Cav-1 levels. When our results were adjusted for tumor and nodal staging using Cox regression modeling, an absence of stromal Cav-1 remained an independent predictor of poor outcome. Thus, stromal Cav-1 expression can be used to stratify human breast cancer patients into low-risk and high-risk groups, and to predict their risk of early disease recurrence at diagnosis. Based on related mechanistic studies, we suggest that breast cancer patients lacking stromal Cav-1 might benefit from anti-angiogenic therapy, in addition to standard regimens. As such, Cav-1 may function as a tumor suppressor in the stromal micro-environment.

The tumor microenvironment plays a previously unrecognized role in human breast cancer onset and progression. Although the mammary microenvironment is composed of a host of cell types, tissue fibroblasts are an integral part of the mammary stromal and are thought to become "activated" or hyper-proliferative during tumor formation (known as the desmoplastic reaction). These cancer-associated fibroblasts (CAFs) take on the characteristics of myofibroblasts often observed during the process of wound healing. Little is known about the molecular events that govern the conversion of mammary stromal fibroblasts to tumor associated fibroblasts. During wound healing, this process is known to be driven by activation of the TGF-β signaling cascade. In addition, CAFs have been shown to secrete important growth factors, such as transforming growth factors (TGF)-β, platelet-derived growth factors (PDGF), hepatocyte growth factor (HGF), suggesting a role in tumor cell invasion.

Recently, we isolated cancer-associated fibroblasts (CAFs) from human breast cancer lesions and studied their properties, as compared with normal mammary fibroblasts (NFs) isolated from the same patient. Interestingly, we demonstrated that 8 out of 11 CAFs show dramatic down-regulation of caveolin-1 (Cav-1) protein expression; Cav-1 is a well-established marker that is normally decreased during the oncogenic transformation of fibroblasts. We also performed gene expression profiling studies (DNA mircoarray) and established a new CAF gene expression signature. Interestingly, the expression signature associated with CAFs includes a large number of genes that are regulated via the RB-pathway. This CAF-associated RB/E2F gene signature is also predictive of poor clinical outcome in breast cancer patients that were treated with tamoxifen mono-therapy, indicating that CAFs may be useful for predicting the response to hormonal therapy. In direct support of these findings, implantation of mammary tumor tissue in the mammary fat pads of Cav-1 (-/-) null mice results in up to a ~2-fold increase in tumor growth, indicating that the mammary stroma of Cav-1 (-/-) null mice has tumor promoting properties 7. However, it remains unknown whether loss of Cav-1 is sufficient to confer RB functional inactivation in mammary stromal fibroblasts (MSFs).

In addition, we have now employed a genetic approach using Cav-1 (-/-) null mice. Importantly, we show that the Cav-1 (-/-) MSF transcriptome significantly overlaps with that of human CAFs; both show a nearly identical profile of RB/E2F regulated genes that are upregulated, consistent with RB functional inactivation. Thus, Cav-1 (-/-) MSFs represents the first molecular genetic model for dissecting the activated signaling networks that govern the phenotypic behavior of human breast CAFs.

All references cited herein are incorporated herein by reference in their entireties.

## BRIEF SUMMARY OF THE INVENTION

The invention provides a method for making a prognosis of disease course in a human neoplastic disease patient, the method comprising the steps of: (a) obtaining a sample of stromal cells adjacent to a neoplasm; (b) determining the level of caveolin-1 and/or caveolin-2 protein expression in the stromal cells of the sample; wherein said prognosis is predicted from considering a likelihood of further neoplastic disease which is made when the level of caveolin-1 and/or caveolin-2 protein expression in the stromal cells of the sample is lower than the level of caveolin-1 and/or caveolin-2 protein expression in a control. The invention further provides a method wherein the human neoplastic disease patient has a neoplasm selected from the group consisting of breast, skin, kidney, lung, pancreas, rectum and colon, prostate, bladder, epithelial, non-epithelial, lymphomas, sarcomas, melanomas, and the like. The invention further provides a method wherein the human neoplastic disease patient has a breast neoplasm subtype selected from the group consisting of ER(+), PR(+), HER2(+), triple-negative (ER(-)/PR(-)/HER2(-)), ER(-), PR(-), all tumor and nodal stages, and all tumor grades. The invention further provides a method wherein the level of caveolin-1 and/or caveolin-2 stromal expression is determined by immunohistochemical staining. The invention further provides a method wherein the prognosis of disease course includes a risk for metastasis, recurrence and relapse of neoplastic disease. The invention further provides a method wherein loss of stromal caveolin-1 and/or caveolin-2 predicts early disease recurrence, metastasis, survival, and tamoxifen-resistance at diagnosis. The invention further provides a method wherein loss of stromal caveolin-1 and/or caveolin-2 predicts the prognosis of lymph-node positive (LN(+)) patients. The invention further provides a method wherein loss or absence of stromal caveolin-1 and/or caveolin-2 is associated with a poor prognosis. The invention further provides a method wherein the up-regulation or presence of stromal caveolin-1 and/or caveolin-2 is associated with a good prognosis. The invention further provides a method wherein epithelial caveolin-1 expression is not predictive in any of the sub-types of breast neoplasm. The invention further provides a method wherein the neoplasm is a pre-malignant lesions selected from the group consisting of ductal carcinoma in situ (DCIS) of the breast and myelodysplastic syndrome of the bone marrow. The invention further provides a method wherein the prognosis of disease course includes staging malignant disease in a human neoplastic disease patient. The invention further provides a method wherein loss or absence of stromal caveolin-1 and/or caveolin-2 is a surrogate marker for stromal RB tumor suppressor functional inactivation by RB hyper-phosphorylation.

The invention provides a method for determining the likelihood that a carcinoma is of a grade likely to become an invasive carcinoma comprising: (a) obtaining a sample of stromal cells adjacent to a neoplasm from a neoplastic disease patient; (b) determining the labeling level of caveolin-1 and/or caveolin-2 protein expression in the stromal cells of the sample; and (c) correlating an elevated amount of labeling signal in the test sample with a control, wherein the carcinoma is of a grade likely to become invasive when the level of caveolin-1 and/or caveolin-2 protein expression in the stromal cells of the sample is lower than the level of caveolin-1 and/or caveolin-2 protein expression in a control.

The invention further provides a method wherein the carcinoma is a carcinoma of the

breast. The invention further provides a method wherein the carcinoma is selected from the group consisting of carcinoma of the breast, skin, kidney, parotid gland, lung, bladder and prostate. The invention further provides a method wherein the detection reagent is a labeled antibody capable of binding to human caveolin-1 and/or caveolin-2. The invention further provides a method wherein the amount of labeling signal is measured by a technique selected from the group consisting of emulsion autoradiography, phosphorimaging, light microscopy, confocal microscopy, multi-photon microscopy, and fluorescence microscopy. The invention further provides a method wherein the amount of labeling signal is measured by autoradiography and a lowered signal intensity in a test sample compared to a control prepared using the same steps as the test sample is used to diagnose a high grade carcinoma possessing a high probability the carcinoma will progress to an invasive carcinoma.

The invention provides a kit for making a prognosis of disease course in a human neoplastic disease patient, comprising: (a) a label that labels caveolin-1 and/or caveolin-2; and (b) a usage instruction for performing a screening of a sample of said subject with said label such as that an amount of caveolin-1 and/or caveolin-2 present in the sample is determined. The invention further provides a kit wherein the subject is a mammal. The invention further provides a kit wherein the subject is a human. The invention further provides a kit wherein the caveolin-1 and/or caveolin-2 being labeled is cell surface caveolin-1 and/or caveolin-2. The invention further provides a kit wherein the caveolin-1 and/or caveolin-2 being labeled is systemic caveolin-1 and/or caveolin-2. The invention further provides a kit wherein the label comprises an antibody that specifically binds to caveolin-1 and/or caveolin-2. The invention further provides a kit wherein the antibody is a monoclonal antibody. The invention further provides a kit wherein the antibody is a polyclonal antibody.

The invention provides a kit comprising: a panel of antibodies comprising: an antibody or binding fragment thereof specific for caveolin-1 and/or caveolin-2 whose binding with stromal cells adjacent to a neoplasm has been correlated with breast cancer treatment outcome or patient prognosis; at least one additional antibody or binding fragment thereof specific for a protein whose expression is correlated with breast cancer treatment outcome or patient prognosis, reagents to perform a binding assay; a computer algorithm, residing on a computer, operating, in consideration of all antibodies of the panel historically analyzed to bind to samples, to interpolate, from the aggregation of all specific antibodies of the panel found bound to the stromal cells adjacent to a neoplasm sample, a prediction of treatment outcome for a specific treatment for breast cancer or a future risk of breast cancer progression for the subject The invention further provides a kit wherein the anti-caveolin-1 and/or caveolin-2 antibody comprises: a poly- or monoclonal antibody specific for caveolin-1 and/or caveolin-2 protein or protein fragment thereof correlated with breast cancer treatment outcome or patient prognosis. The invention further provides a kit wherein the panel of antibodies further comprises:a number of immunohistochemistry assays equal to the number of antibodies within the panel of antibodies. The invention further provides a kit wherein the antibodies of the panel of antibodies further comprise: antibodies specific to ER, PR, and/or HER-2. The invention further provides a kit wherein the treatment outcome predicted comprises the response to anti-neoplastic therapy or chemotherapy.

The invention provides a method for making a prognosis of disease course in a human patient by detecting differential expression of at least one marker in ductal carcinoma in situ (DCIS) pre-invasive cancerous breast tissue, said method comprising the steps of: (a) obtaining a sample of DCIS breast tissue and surrounding stromal cells from a human neoplastic disease patient; (b) determining the level of caveolin-1 and/or caveolin-2 protein expression in the stromal cells of the sample as the at least one marker and comparing the level of caveolin-1 and/or caveolin-2 protein expression in the stromal cells of the sample with the level of caveolin-1 and/or caveolih-2 protein expression in a control; wherein said prognosis of further progression is made when the level of caveolin-1 and/or caveolin-2 protein expression in the stromal cells of the sample is lower than the level of caveolin-1 and/or caveolin-2 protein expression in the control. The invention further provides a method wherein the size of said abnormal tissue sample substantially conforms to an isolatable tissue structure wherein only cells exhibiting abnormal cytological or histological characteristics are collected. The invention further provides a method further comprising confirming said differential expression of said marker in said normal tissue sample and in said abnormal tissue sample by using an immunological technique. The invention further provides a method wherein said immunological technique is selected from the group consisting of radioimmunoassay (RIA), EIA, ELISA, and immunofluorescence assays. The invention further provides a method wherein said abnormal breast tissue cells are non-comedo ductal carcinoma in situ cells.

The invention provides a method for making a prognosis of disease course in a human neoplastic disease patient; the method comprising the steps of: (a) obtaining a sample of a stromal cells adjacent to a neoplasm; (b) determining the level of the protein expression of a protein selected from the group consisting of vimentin, calponin2, tropomyosin, gelsolin, prolyl 4-hydroxylase alpha, EF-1-delta, and M2-isoform of pyruvate kinase in the stromal cells of the sample and comparing the level of the protein expression of a protein selected from the group consisting of vimentin, calponin2, tropomyosin, gelsolin, prolyl 4-hydroxylase alpha, EF-1-delta, and M2-isoform of pyruvate kinase in the stromal cells of the sample with the level of the protein expression of a protein selected from the group consisting of vimentin, calponin2, tropomyosin, gelsolin, prolyl 4-hydroxylase alpha, EF-1-delta, and M2-isoform of pyruvate kinase in a control; wherein said prognosis is predicted from considering a likelihood of further neoplastic disease which is made when the level of the protein expression of a protein selected from the group consisting of vimentin, calponin2, tropomyosin, gelsolin, prolyl 4-hydroxylase alpha, EF-1-delta, and M2-isoform of pyruvate kinase in the stromal cells of the sample is higher than the level of the protein expression of a protein selected from the group consisting of vimentin, calponin2, tropomyosin, gelsolin, prolyl 4-hydroxylase alpha, EF-1-delta, and M2-isoform of pyruvate kinase in the control. The invention further provides a method wherein the human neoplastic disease patient has a neoplasm selected from the group consisting of breast, skin, kidney, lung, pancreas, rectum and colon, prostate, bladder, epithelial, non-epithelial, lymphomas, sarcomas, melanomas, and the like. The invention further provides a method wherein the human neoplastic disease patient has a breast neoplasm subtype selected from the group consisting of ER(+), PR(+), HER2(+), triple-negative (ER(-)/PR(-)/HER2(-)), ER(-), PR(-), all tumor and nodal stages, and all tumor grades. The invention further provides a method wherein the level of a protein selected from the group consisting of vimentin, calponin2, tropomyosin, gelsolin, prolyl 4-hydroxylase alpha, EF-1-delta, and M2-isoform of pyruvate kinase stromal expression is determined by immunohistochemical staining. The invention further provides a method wherein the prognosis of disease course includes a risk for metastasis, recurrence and relapse of neoplastic disease. The invention further provides a method wherein increase of stromal protein selected from the group consisting of vimentin, calponin2, tropomyosin, gelsolin, prolyl 4-hydroxylase alpha, EF-1-delta, and M2-isoform of pyruvate kinase predicts early disease recurrence, metastasis, survival, and tamoxifen-resistance at diagnosis. The invention further provides a method wherein increase of stromal protein selected from the group consisting of vimentin, calponin2, tropomyosin, gelsolin, prolyl 4-hydroxylase alpha, EF-1-delta, and M2-isoform of pyruvate kinase predicts the prognosis of lymph-node positive (LN(+)) patients. The invention further provides a method wherein increase of stromal protein selected from the group consisting of vimentin, calponin2, tropomyosin, gelsolin, prolyl 4-hydroxylase alpha, EF-1-delta, and M2-isoform of pyruvate kinase is associated with a poor prognosis. The invention further provides a method wherein the neoplasm is a pre-malignant lesions selected from the group consisting of ductal carcinoma in situ (DCIS) of the breast and myelodysplastic syndrome of the bone marrow. The invention further provides a method wherein the prognosis of disease course includes staging malignant disease in a human neoplastic disease patient.

The invention provides a method for determining the likelihood that a carcinoma is of a grade likely to become an invasive carcinoma comprising: (a) obtaining a sample of stromal cells adjacent to a neoplasm from the human neoplastic disease patient; (b) determining the labeling level of the protein expression of a protein selected from the group consisting of vimentin, calponin2, tropomyosin, gelsolin, prolyl 4-hydroxylase alpha, EF-1-delta, and M2-isoform of pyruvate kinase in the stromal cells of the sample and comparing the labeling level of the protein expression of a protein selected from the group consisting of vimentin, calponin2, tropomyosin, gelsolin, prolyl 4-hydroxylase alpha, EF-1-delta, and M2-isoform of pyruvate kinase in the stromal cells of the sample with the labeling level of the protein expression of a protein selected from the group consisting of vimentin, calponin2, tropomyosin, gelsolin, prolyl 4-hydroxylase alpha, EF-1-delta, and M2-isoform of pyruvate kinase in a control; and (c) correlating an elevated amount of labeling signal in the test sample with a determination that the carcinoma is of a grade likely to become invasive. The invention further provides a method wherein the carcinoma is a carcinoma of the breast. The invention further provides a method wherein the carcinoma is selected from the group consisting of carcinoma of the breast, skin, kidney, parotid gland, lung, bladder and prostate. The invention further provides a method wherein the detection reagent is a labeled antibody capable of binding to a protein selected from the group consisting of vimentin, calponin2, tropomyosin, gelsolin, prolyl 4-hydroxylase alpha, EF-1-delta, and M2-isoform of pyruvate kinase: The invention further provides a method wherein the amount of labeling signal is measured by a technique selected from the group consisting of emulsion autoradiography, phosphorimaging, light microscopy, confocal microscopy, multi-photon microscopy, and fluorescence microscopy. The invention further provides a method wherein the amount of labeling signal is measured by autoradiography and an elevated signal intensity in a test sample compared to a non-high grade carcinoma control prepared using the same steps as the test sample is used to diagnose a high grade carcinoma possessing a high probability the carcinoma will progress to an invasive carcinoma.

The invention provides a kit for making a prognosis of disease course in a human neoplastic disease patient, comprising: (a) a label that labels the protein expression of a protein selected from the group consisting of vimentin, calponin2, tropomyosin, gelsolin, propyl 4-hydroxylase alpha, EF-1-delta, and M2-isoform of pyruvate kinase; and (b) a usage instruction for performing a screening of a sample of said subject with said label such as that an amount of the protein expression of a protein selected from the group consisting of vimentin, calponin2, tropomyosin, gelsolin, prolyl 4-hydroxylase alpha, EF-1-delta, and M2-isoform of pyruvate kinase present in the sample is determined. The invention further provides a kit wherein the subject is a mammal. The invention further provides a kit wherein the subject is a human. The invention further provides a kit wherein the label comprises an antibody that specifically binds to a protein selected from the group consisting of vimentin, calponin2, tropomyosin, gelsolin, prolyl 4-hydroxylase alpha, EF-1-delta, and M2-isoform of pyruvate kinase. The invention further provides a kit wherein the antibody is a monoclonal antibody. The invention further provides a kit wherein the antibody is a polyclonal antibody.

The invention provides a method for treating neoplastic disease in a patient, comprising the steps of: (a) obtaining a sample of stromal cells adjacent to a neoplasm from the neoplastic disease patient; (b) determining the level of caveolin-1 and/or caveolin-2 a protein expression in the stromal cells of the sample and comparing the level of caveolin-1 and/or caveolin-2 protein expression in the stromal cells of the sample with the level of caveolin-1 and/or caveolin-2 protein expression in a control; (c) predicting if the neoplasm will respond effectively to treatment with an anti-angiogenic agent, wherein said prediction is made when the level of caveolin-1 and/or caveolin-2 protein expression in the stromal cells of the sample is lower than the level of caveolin-1 and/or caveolin-2 protein expression in the control; and administering to said patient a therapeutically effective amount of an ami-angiogenic agent. The invention further provides a method wherein the anti-angiogenic agent comprises an agent selected from the group consisting of angiostatin, bevacizumab, arresten, canstatin, combretastatin, endostatin, NM-3, thrombospondin, tumstatin, 2-methoxyestradiol, Vitaxin, Getfitinib, ZD6474, erlotinib, CI1033, PKI1666, cetuximab, PTK787, SU6668, SU11248, trastuzumab, Marimastat, COL-3, Neovastat, 2-ME, SU6668, anti-VEGF antibody, Medi-522 (Vitaxin II), tumstatin, arrestin, recombinant EPO, troponin I, EMD121974, IFN-alpha, celecoxib, PD0332991, and thalidomide. The invention further provides a method wherein one or more additional anti-neoplastic agents are co-administered simultaneously or sequentially with the anti-angiogenic agent. The invention further provides a method wherein the at least one or more additional anti-neoplastic agent comprises a proteasome inhibitor. The invention further provides a method wherein the proteasome inhibitor is bortezomib. The invention further provides a method wherein the human neoplastic disease patient has a breast neoplasm subtype selected from the group consisting of ER(+), PR(+), HER2(+), triple-negative (ER(-)/PR(-)/HER2(-)), ER(-), PR(-), all neoplasm and nodal stages, and all neoplasm grades. The invention further provides a method wherein the human neoplastic disease patient has a neoplasm selected from the group consisting of breast, skin, kidney, lung, pancreas, rectum and colon, prostate, bladder, epithelial, non-epithelial, lymphomas, sarcomas, melanomas, and the like. The invention further provides a method wherein the neoplasm is a pre-malignant lesion selected from the group consisting of ductal carcinoma in situ (DCIS) of the breast and myelodysplastic syndrome of the bone marrow.

The invention provides a diagnostic kit for assaying the individual sensitivity of target cells towards angiogenesis inhibitors, comprising: (a) a molecule that specifically binds to caveolin-1 and/or caveolin-2; and (b) a pharmaceutically acceptable carrier: The invention further provides a kit wherein the angiogenesis inhibitor is selected from the group consisting of angiostatin, bevacizumab, arresten, canstatin, combretastatin, endostatin, NM-3. thrombospondin, tumstatin, 2-methoxyestradiol, Vitaxin, Getfitinib, ZD6474, erlotinib, CI1033, PKI1666, cetuximab, PTK787, SU6668, SU11248, trastuzumab, Marimastat, COL-3, Neovastat, 2-ME, SU6668, anti-VEGF antibody, Medi-522 (Vitaxin II), tumstatin, arrestin, recombinant EPO, troponin I, EMD121974, IFN-alpha, celecoxib, PD0332991, and thalidomide. The invention further provides a kit wherein the target cell is a cancer cell.

The invention provides a method of predicting whether a neoplastic disease patient is afflicted with a neoplasm that will respond effectively to treatment with an anti-angiogenic agent, comprising: (a) obtaining a sample of a stromal cells adjacent to a neoplasm from the neoplastic disease patient; (b) determining the level of caveolin-1 and/or caveolin-2 protein expression in the stromal cells of the sample and comparing the level of caveolin-1 and/or caveolin-2 protein expression in the stromal cells of the sample with the level of caveolin-1 and/or caveolin-2 protein expression in a control;(c) predicting if the neoplasm will respond effectively to treatment with an anti-angiogenic agent, wherein low expression levels of caveolin-1 and/or caveolin-2 protein expression in the stromal layers relative to caveolin-1 and/or caveolin-2 expression levels in the control correlate with a neoplasm that will respond effectively to treatment with an anti-angiogenic agent. The invention further provides a method wherein the anti-angiogenic agent comprises an agent selected from the group consisting of angiostatin, bevacizumab, arresten, canstatin, combretastatin, endostatin, NM-3, thrombospondin, tumstatin, 2-methoxyestradiol, Vitaxin, Getfitinib, ZD6474, erlotinib, CI1033, PKI1666, cetuximab, PTK787, SU6668, SU 11248, trastuzumab, Marimastat, COL-3, Neovastat, 2-ME, SU6668, anti-VEGF antibody, Medi-522 (Vitaxin II), tumstatin, arrestin, recombinant EPO, troponin I, EMD121974, IFN-alpha, celecoxib, PD0332991, and thalidomide.

The invention provides a method of predicting the sensitivity of neoplasm cell growth to inhibition by an anti-neoplastic agent, comprising: (a) obtaining a sample of stromal cells adjacent to a neoplasm from a neoplastic disease patient; (b) determining a level of caveolin-1 and/or caveolin-2 protein expression in the stromal cells of the sample and comparing the level of caveolin-1 and/or caveolin-2 protein expression in the stromal cells of the sample with the level of caveolin-1 and/or caveolin-2 protein expression in a control; and (c) predicting the sensitivity of neoplasm cell growth to inhibition by an anti-neoplastic agent, wherein low expression levels of the stromal cell caveolin-1 and/or caveolin-2 protein expression compared the level of caveolin-1 and/or caveolin-2 expression in a control correlates with high sensitivity to inhibition by anti-neoplastic agent.

The invention further provides a method wherein the anti-angiogenic agent comprises an agent selected from the group consisting of angiostatin, bevacizumab, arresten, canstatin, combretastatin, endostatin, NM-3, thrombospondin, tumstatin, 2-methoxyestradiol, Vitaxin, Getfitmib, ZD6474, erlotinib, CI1033, PKI1666, cetuximab, PTK787, SU6668, SU11248, trastuzumab, Marimastat, COL-3, Neovastat, 2-ME, SU6668, anti-VEGF antibody, Medi-522 (Vitaxin II), tumstatin, arrestin, recombinant EPO, troponin I, EMD121974, IFN-alpha, celecoxib, PD0332991, and thalidomide.

The invention provides a diagnostic kit for determining the target cancer cells susceptible to anti-angiogenesis inhibitor treatment, comprising: (a) an antibody which specifically binds caveolin-1 and/or caveolin-2; and (b) a pharmaceutically acceptable carrier. The invention further provides a kit wherein the antibody is a polyclonal antibody. The invention further provides a kit wherein the antibody is a monoclonal antibody.

The invention provides a method for treating neoplastic disease in a patient, comprising the steps of: (a) obtaining a sample of stromal cells adjacent to a neoplasm from the patient; (b) determining the level of caveolin-1 and/or caveolin-2 protein expression in the stromal cells of the sample and comparing the level of caveolin-1 and/or caveolin-2 protein expression in the stromal cells of the sample with the level of caveolin-1 and/or caveolin-2 protein expression in a control; (c) predicting if the neoplasm will respond effectively to treatment with a lactate transporter inhibitor, wherein low expression levels of the stromal cell caveolin-1 and/or caveolin-2 protein expression compared the level of caveolin-1 and/or caveolin-2 expression in a control correlates with high sensitivity to treatment with a lactate transporter inhibitor, and (d) administering to said patient a therapeutically effective amount of a lactate transporter inhibitor. The invention further provides a method wherein the lactate transporter inhibitor comprises an agent which inhibits an enzyme selected from the group consisting of triose-phosphate isomerase, fructose 1,6 bisphosphate aldolase, glycero-3-phosphate dehydrogenase, phosphoglycerate kinase, phosphoglycerate mutase, enolase, pyruvate kinase, lactate dehydrogenase. The invention further provides a method wherein one or more additional anti-neoplastic agents are co-administered simultaneously or sequentially with the lactate transporter inhibitor. The invention further provides a method wherein the human neoplastic disease patient has a breast neoplasm subtype selected from the group consisting of ER(+), PR(+), HER2(+), triple-negative (ER(-)/PR(-)/HER2(-)), ER(-), PR(-), all neoplasm and nodal stages, and all neoplasm grades. The invention further provides a method wherein the human neoplastic disease patient has a neoplasm selected from the group consisting of breast, skin, kidney, lung, pancreas, rectum and colon, prostate, bladder, epithelial, non-epithelial, lymphomas, sarcomas, melanomas, and the like. The invention further provides a method wherein the neoplasm is a pre-malignant lesion selected from the group consisting of ductal carcinoma in situ (DCIS) of the breast and myelodysplastic syndrome of the bone marrow.

The invention provides a method of predicting the sensitivity of neoplasm cell growth to inhibition by a lactate transporter inhibitor, comprising: (a) obtaining a sample of stromal cells adjacent to a neoplasm from a neoplastic disease patient; (b) determining a level of caveolin-1 and/or caveolin-2 protein expression in the stromal cells of the sample and comparing the level of caveolin-1 and/or caveolin-2 protein expression in the stromal cells of the sample with the level of caveolin-1 and/or caveolin-2 protein expression in a control; and (c) predicting the sensitivity of neoplasm cell growth to inhibition by a.lactate transporter inhibitor, wherein low expression levels of the stromal cell caveolin-1 and/or caveolin-2 protein expression compared the level of caveolin-1 and/or caveolin-2 expression in a control correlates with high sensitivity to inhibition by a lactate transporter inhibitor. The invention further provides a method wherein the lactate transporter inhibitor comprises an agent which inhibits an enzyme selected from the group consisting of triose-phosphate isomerase, fructose 1,6 bisphosphate aldolase, glycero-3-phosphate dehydrogenase, phosphoglycerate kinase, phosphoglycerate mutate, enolase, pyruvate kinase, lactate dehydrogenase.

The invention provides a method of identifying a potential therapeutic agent that treats stromal caveolin-1 and/or caveolin-2 deficient neoplasms comprising: providing a wild-type mouse injected with mouse mammary neoplasm cells in the mammary fat pad as a control mouse; providing a caveolin-1 and/or caveolin-2 deficient mouse injected with mouse mammary neoplasm cells in the mammary fat pad as a lest mouse; providing a potential therapeutic agent; injecting a placebo into a test mouse; injecting a placebo into a control mouse; treating both a test mouse and a control mouse with the potential therapeutic agent; measuring vascularization of the resulting neoplasm in the test mouse and the control mouse in the presence of placebo; measuring vascularization of the resulting neoplasm in the test mouse and the control mouse in the presence of the potential therapeutic agent; comparing vascularization in the test subject mouse with the vascularization in the control mouse, in the presence of either placebo or the potential therapeutic agent, wherein a decrease in vascularization in the test mouse injected with the potential therapeutic agent identifies a therapeutic agent which treats stromal caveolin-1 and/or caveolin-2 deficient neoplasms. The invention further provides a method wherein the mouse mammary neoplasm cells are Met-1 cells. The invention further provides a method wherein the caveolin-1 and/or caveolin-2 deficient mouse is a knockout mouse.

The invention provides a method of screening for anticancer activity of a potential therapeutic agent comprising: (a) providing a cell deficient in expression of caveolin-1 and/or caveolin-2, or fragment thereof; (b) contacting a tissue sample derived from a cancer cell with potential therapeutic agent; and (c) monitoring an effect of the potential therapeutic agent on an expression of the caveolin-1 and/or caveolin-2 in the tissue sample. The invention further provides a method further comprising: (d) comparing the level of expression in the absence of said potential therapeutic agent to the level of expression in the presence of the drug candidate.

The invention provides a method for screening for potential therapeutic agent capable of modulating the activity of caveolin-1 and/or caveolin-2, said method comprising: a) combining said caveolin-1 and/or caveolin-2 and a candidate bioactive agent; and b) determining the effect of the potential therapeutic agent on the bioactivity of said caveolin-1 and/or caveolin-2. The invention further provides a method wherein the potential therapeutic agent affects the expression of the caveolin-1 and/or caveolin-2.

The invention provides a method for treating neoplastic disease in a patient, comprising the steps of: (a) obtaining a sample of stromal cells surrounding a neoplasm from a neoplastic disease patient; (b) determining the level of caveolin-1 and/or caveolin-2 a protein expression in the stromal cells of the sample and comparing the level of caveolin-1 and/or caveolin-2 protein expression in the stromal cells of the sample with the level of caveolin-1 and/or caveolin-2 protein expression in a control; (c) predicting if the neoplasm will respond effectively to treatment with a therapeutic agent, wherein low expression levels of the stromal cell caveolin-1 and/or caveolin-2 protein expression compared the level of caveolin-1 and/or caveolin-2 expression in a control correlates with high sensitivity to inhibition by a therapeutic agent; and administering to said patient a therapeutically effective amount of a therapeutic agent.

The invention further provides a method wherein the therapeutic agent comprises an agent selected from the group consisting of 17-AAG, Apatinib, Ascomycin, Axitinib, Bexarotene, Bortezomib, Bosutinib, Bryostatin 1, Bryostatin 2, Canertinib, Carboplatin, Cediranib, Cisplatin, Cyclopamine, Dasatinib, 17-DMAG, Docetaxel, Doramapimod, Dovitinib, Erlotinib, Everolimus, Gefitinib, Geldanamycin, Gemcitabine, Imatinib, Imiquimod, Ingenol 3-Angelate, Ingenol 3-Angelate 20-Acetate, Irinotecan, Lapatinib, Lestaurtinib, Nedaplatin, Masitinib, Mubritinib, Nilotinib, NVP-BEZ235, OSU-03012, Oxaliplatin, Paclitaxel, Pazopanib, Picoplatin, Pimecrolimus, PKC412, Rapamycin, Satraplatin, Sorafenib, Sunitinib, Tandutinib, Tivozanib, Thalidomide, Temsirolimus, Tozasertib, Vandetanib, Vargatef, Vatalanib, Zotarolimus, ZSTK474, Bevacizumab (Avasti), Cetuximab, Herceptin, Rituximab, Trastuzumab, Apatinib, Axitinib, Bisindolylmaleimide I, Bisindolylmaleimide. I, Bosutinib, Canertinib, Cediranib, Chelerythrine, CP690550, Dasatinib, Dovitinib, Erlotinib, Fasudil, Gefitinib, Genistein, Gö 6976, H-89, HA-1077, Imatinib, K252a, K252c, Lapatinib, Di-p-Toluenesulfonate, Lestaurtinib, LY 294002, Masitinib, Mubritinib, Nilotinib, OSU-03012, Pazopanib, PD 98059, PKC412, Roscovitine, SB 202190, SB 203580, Sorafenib, SP600125, Staurosporine, Sunitinib, Tandutinib, Tivozanib, Tozasertib, Tyrphostin AG 490, Tyrphostin AG 1478, U0126, Vandetanib, Vargatef, Vatalanib, Wortmannin, ZSTK474, Cyclopamine, Carboplatin, Cisplatin, Eptaplatin, Nedaplatin, Oxaliplatin, Picoplatin, Satraplatin, Bortezomib (Velcade), Metformin, Halofuginone. Metformin, N-acetyl-cysteine (NAC), RTA 402 (Bardoxolone methyl), Auranofin, BMS-345541, IMD-0354, PS-1145, TPCA-1, Wedelolactone, Echinomycin, 2-deoxy-D-glucose (2-DG), 2-bromo-D-glucose, 2-fluoro-D-glucose, and 2-iodo-D-glucose, dichloro-acetate (DCA), 3-chloro-pyruvate, 3-Bromo-pyruvate (3-BrPA), 3-Bromo-2-oxopropionate, Oxamate, LY 294002, NVP-BEZ235, Rapamycin, Wortmannin, Quercetin, Resveratrol, N-acetyl-cysteine (NAC), N-acetyl-cysteine amide (NACA), Ascomycin, CP690550, Cyclosporin A, Everolimus, Fingolimod, FK-506, Mycophenolic Acid, Pimecrolimus, Rapamycin, Temsirolimus, Zotarolimus, Roscovitine, PD 0332991 (CDK4/6 inhibitor), Chloroquine, BSI-201, Olaparib, DR 2313, and NU 1025.

## BRIEF DESCRIPTION OF SEVERAL VIEWS OF THE DRAWINGS

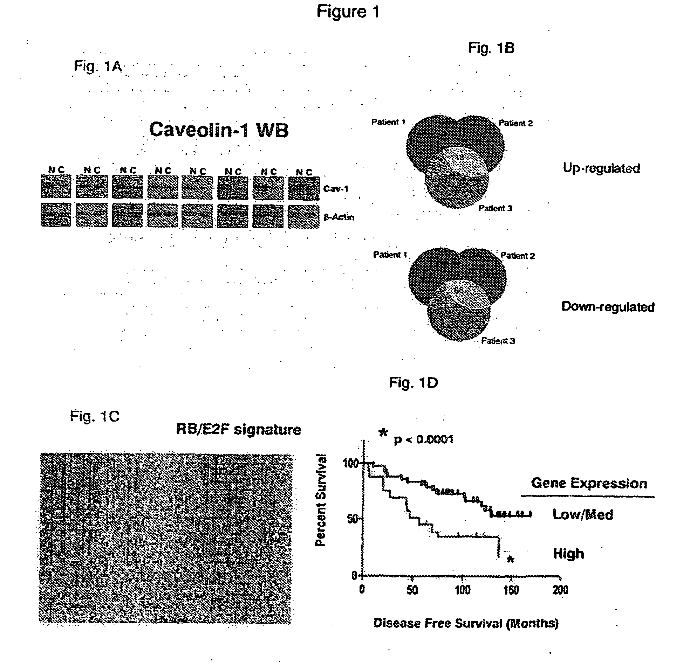

Figure 1. High expression of the breast CAF gene signature is associated with poor clinical outcome in breast cancer patients treated with tamoxifen mono-therapy. (Fig. 1A) Western blot (WB) analysis shows the down-regulation of Cav-1 protein expression (-2.5 fold on average) in the CAFs (N versus C) from 8 different patients. Expression of beta-actin is shown as a control for equal protein loading. N, normal mammary fibroblasts; and C, breast cancer-associated fibroblasts from the same patients. (Fig. 1B) Venn diagrams summarizing how the 2 gene signatures were derived by comparing and intersecting the gene sets from matched NFs (N) and CAFs C) from 3 different patients. (Fig. 1C) Gene expression data from 60 ER -positive human breast tumors that were both micro- and macrodissected were analyzed for the expression patter of 118 genes upregulated in CAFs. A core of proliferation-associated genes that are regulated by the kB/E2F pathway (marked in red)strongly co-segregated in this analysis. (Fig. 1D) A Kaplan-Meier survival analysis was conducted, wherein the recurrence of those tumors in the highest quartile of overall expression was compared against the remainder of the cohort (p<0.001). Patients in the High CAF gene expression group bad a poor prognosis on Tamoxifen mono-therapy, with greater than a 3.8-fold reduction in recurrence-free survival.

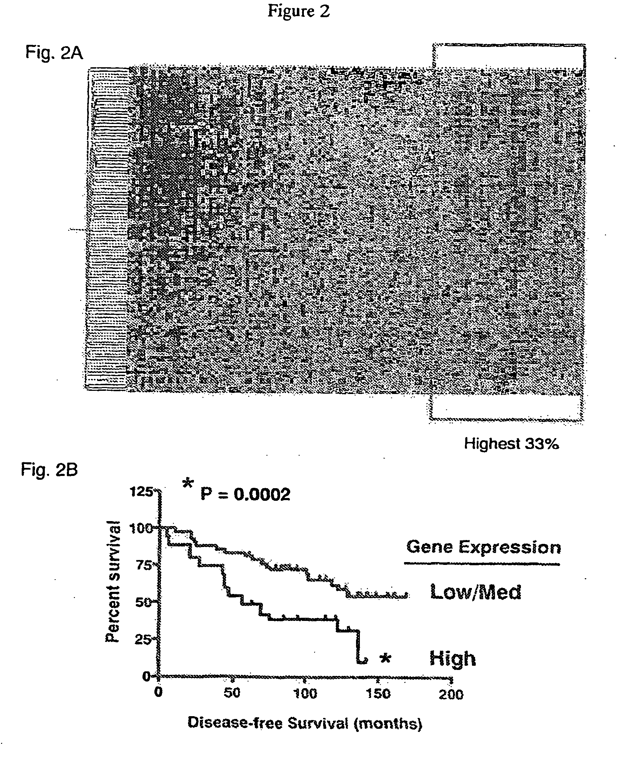

Figure 2. High expression of the Cav-1 (-/-) MSF gene signature is associated with poor clinical outcome in breast cancer patients treated with tamoxifen mono-therapy. (Fig. 2A) Gene expression data from 60 ER-positive human breast tumors that were both micro- and macrodissected were analyzed for the expression pattern of proliferative genes upregulated in Cav-1 (-/-) MSFs. A core of proliferation associated genes that are regulated by the RB/E2F pathway strongly co-segregated in this analyses. (Fig. 2B) A Kaplan-Meier survival analysis was conducted, wherein the recurrence of those tumors in the highest one-third of overall expression was compared against the remainder of the cohort (p<0.0002). Patients in the High Cav-1 (-/-) MSFs gene expression group had a poor prognosis on Tamoxifen mono-therapy, with greater than a ~2.6-fold reduction in disease-free survival.

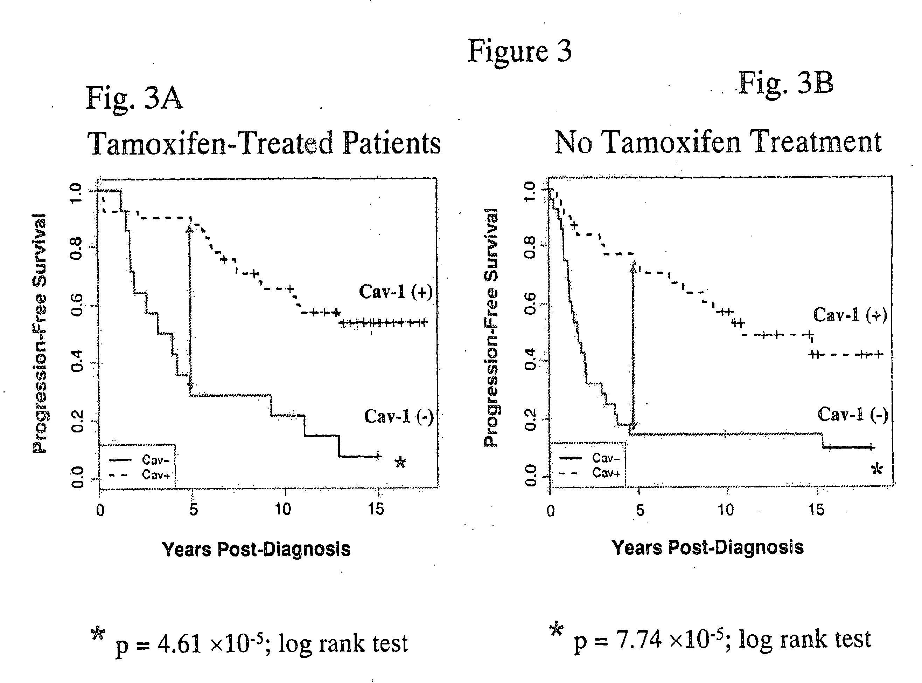

Figure 3. Kaplan-Meier curves of Progression-Free Survival for Patients with and without Tamoxifen-Treatment. (Fig. 3A): Note that an absence of stromal Cav-1 immunostaining predicts poor clinical outcome in Tamoxifen-treated patients, suggestive of tamoxifen resistance. (Fig. 3B): Virtually identical results were obtained with patients that did not receive Tamoxifen. In both panels, 5-year PFS is indicated by an arrow. Tamoxifen-Treated (p=4.61 x $10^{-5}$, log-rank test); No Tamoxifen (p = 7.74 x $10^{-5}$, log-rank test).

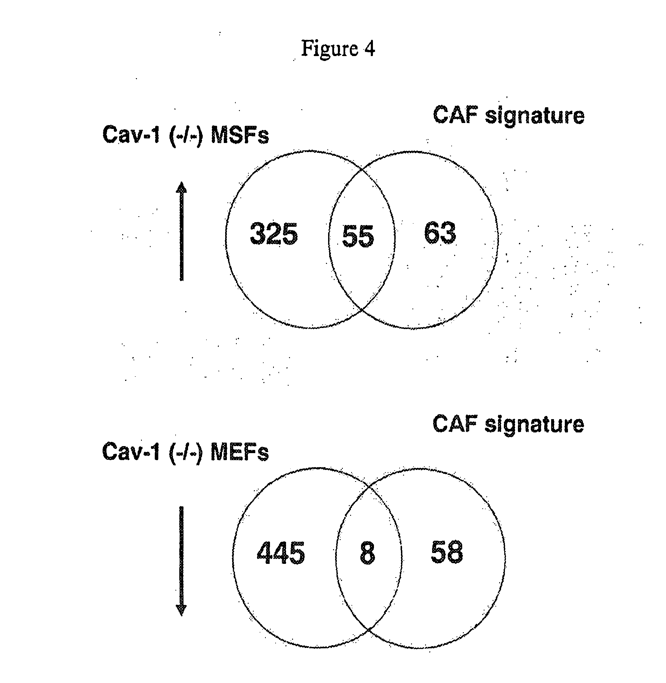

Figure 4. CAF versus Cav-1 (-/-) MSF Gene Signatures. Venn diagrams summarizing the similarities and differences between gene transcript changes in human breast CAFs and Cav-1 (-/-) MSFs.

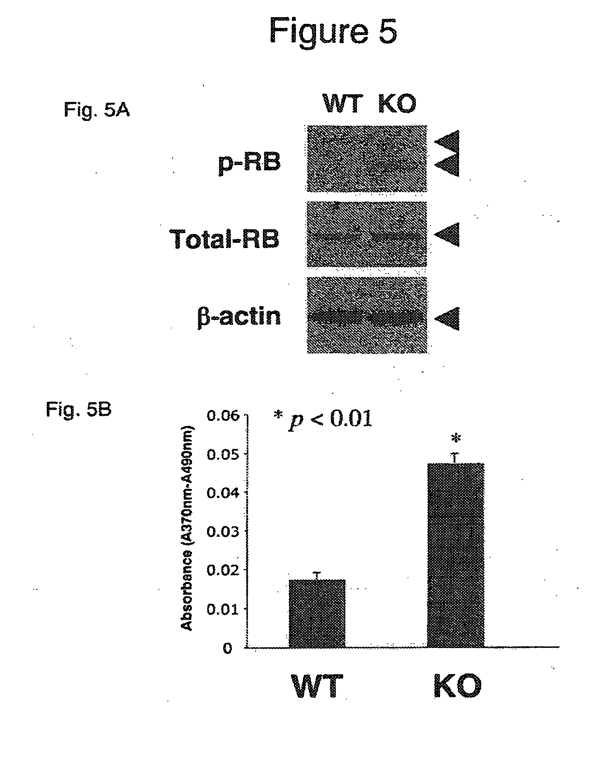

Figure 5: Cav-1 (-/-) MSFs show Increased Levels of Phosphorvlated RB (p-RB). (Fig. 5A) Virtually identical results were obtained by Western blot analysis. Total RB levels and (β-actin levels are shown for comparison. (Fig. 5B) Note also that Cav-1 (-/-) MSFs show a significant increase in BrdU incorporation, consistent with cell cycle progression (* p < 0.01). WT, wild-type; KO, Cav-1 (-/-).

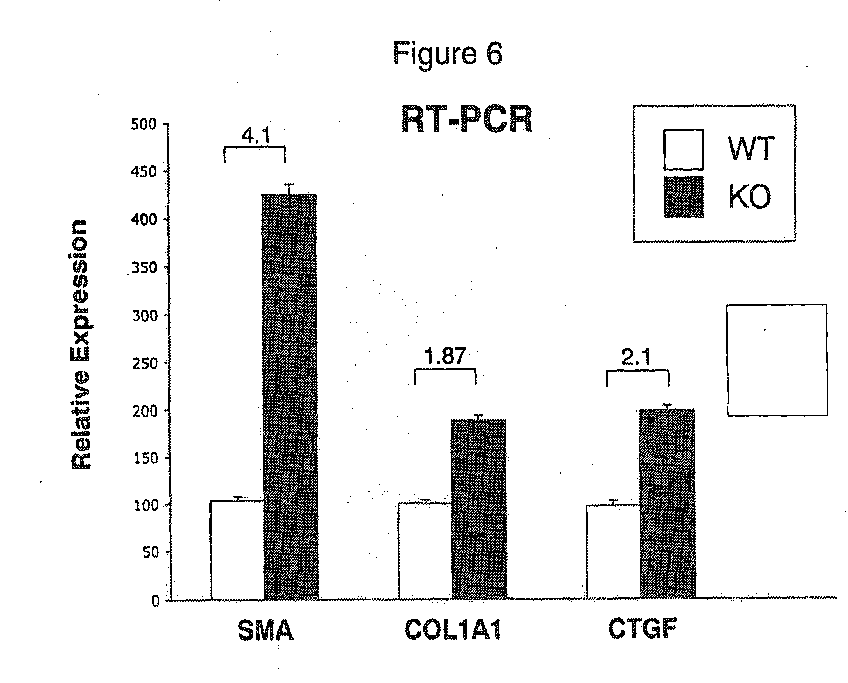

Figure 6. Cav-1 (-/-) MSFs Show Increased Levels of TGF-beta/Smad Responsive Genes. Relative quantification of samples was assessed by arbitrarily setting the control cDNA value at 100, and changes in transcript levels of a sample were expressed as a multiple thereof (relative expression). The differences in the number of mRNA copies in each PCR reactions was corrected for using mouse 18S rRNA endogenous control transcript levels. (P < 0.05). WT, wild-type; KO, Cav-1 (-/-).

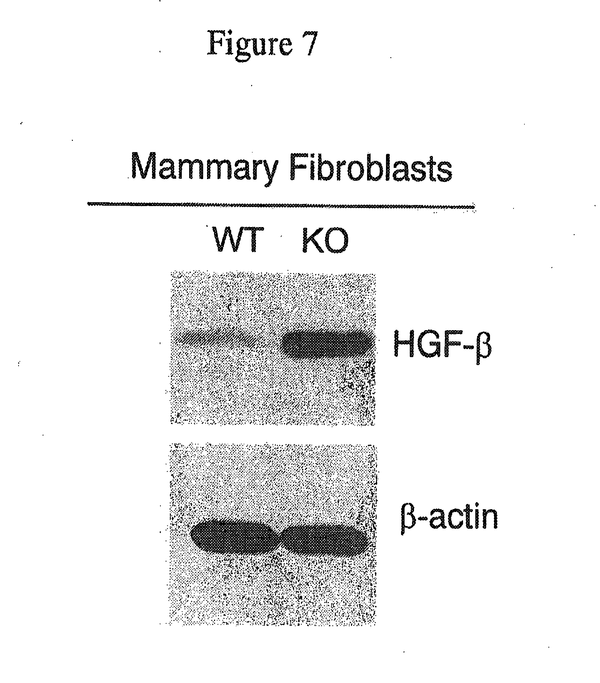

Figure 7. Cav-1 (-/-) MSFs Express Higher Levels of HGF, a Key Epithelial Morphogen. Lysates from WT and Cav-1 (-/-) MSFs were subjected to immunoblot analysis with an antibody directed against the (3-chain of HGF. Immunoblotting with (β-actin is shown as an equal loading control.

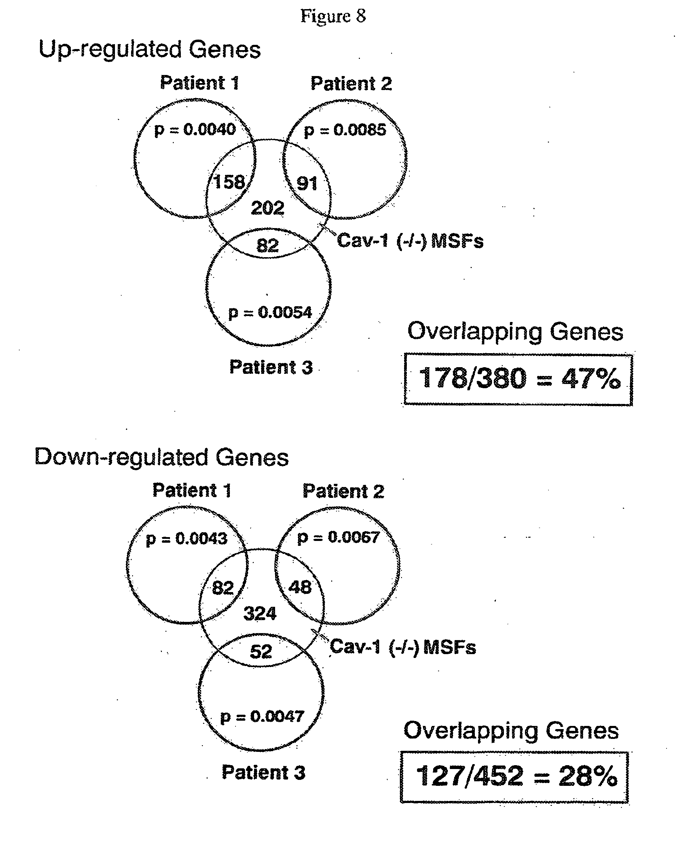

Figure 8. Transcriptional Comparison of Cav-1 (-/-) MSFs with CAFs Isolated from Individual Patients. We previously isolated 11 sets of CAFs, with corresponding NFs, from the same patients[6]. Three of these sets were randomly selected for genomewide transcriptional profiling. These primary cells (CAFs vs. NFs) were then compared pairwise to identify genes up-regulated or down-regulated in CAFs from breast cancer patients 1, 2, and 3. These 3 gene sets were then individually compared with the gene changes observed in WT vs. Cav-1 (-I-) MSFs. Note that intersection of the up-regulated CAF genes from patients 1, 2, and 3, with up-regulated Cav-1 (-/-) MSF genes, revealed significant overlap. Patients 1, 2, and 3 shared 158, 91, and 82 upregulated genes with Cav-1 (-/-) MSFs, respectively. These 3 gene lists were then compiled to yield a master list of 178 unique genes that were up-regulated in both CAFs and Cav-1 (-/-) MSFs. Thus, 178 of the 380 genes that were up-regulated in Cav-1 (-/-) were also upregulated in CAFs, yielding an overlap of 47%. However, 202 up-regulated Cav-1 (-/-) MSF genes did not show overlap with up-regulated CAFs genes. Identical comparisons were also made for the down-regulated gene sets. Patients 1, 2, and 3 shared 82, 48, and 52 down-regulated genes with Cav-1 (-/-) MSFs, respectively. These 3 gene lists were then compiled to yield a master list of 127 unique genes that were down-regulated in both CAFs and Cav-1 (-/-) MSFs. Thus, 127 of the 452 genes that were down-regulated in Cav-1 (-/-) were also down-regulated in CAFs, yielding an overlap of 28%. However, 324 down-regulated Cav-1 (-/-) MSF genes did not show overlap with downregulated CAFs genes. Thus, overall Cav-1 (-/-) MSFs were most closely related to CAFs from patient 1. CAFs, breast cancer-associated fibroblasts; NFs, normal mammary fibroblasts.

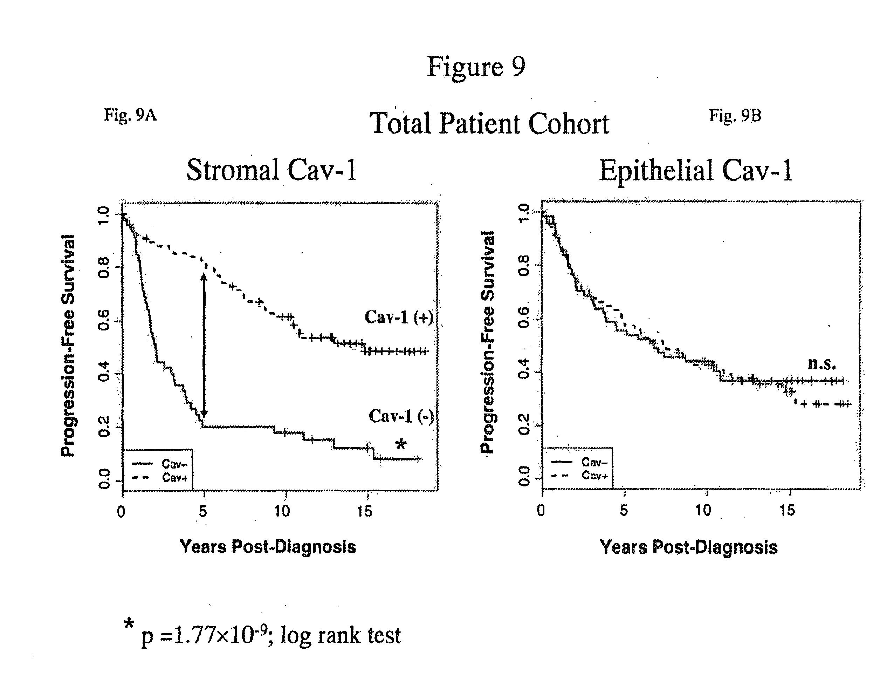

Figure 9. Epithelial Cav-1 Expression is not a Predictor of Progression-Free Survival. The status of stromal (Fig. 9A) and epithelial Cav-1 (Fig. 9B) was independently scored in the same total patient population for direct comparison. Note that only stromal Cav-1 is a predictor of clinical outcome (p=1.77 x $10^{-9}$, log-rank test), in a total population of 125 breast cancer patients. 5-year PFS is indicated by an arrow. The status of epithelial Cav-1 is also shown. n.s., denotes not significant.

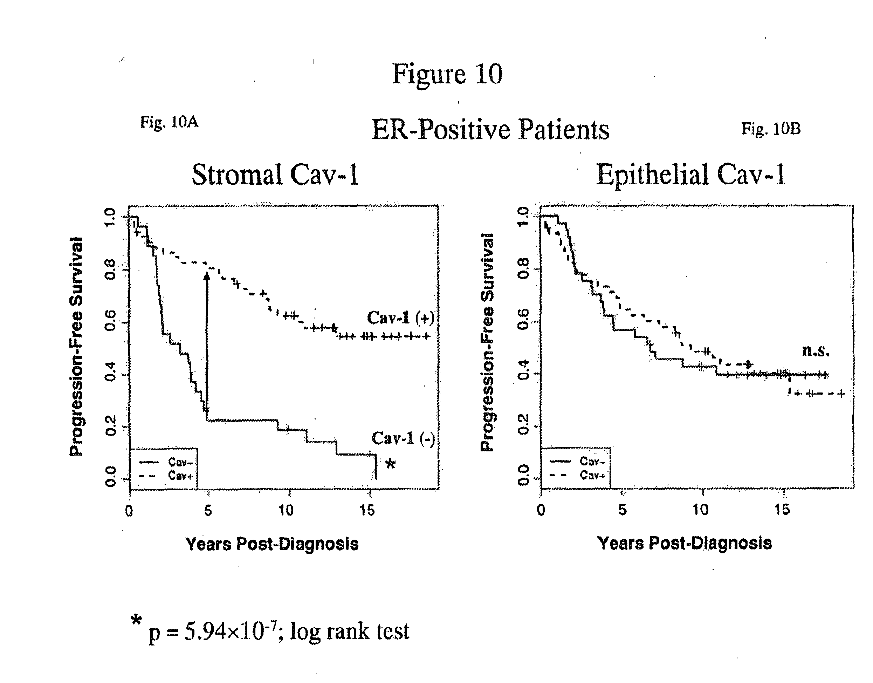

Figure 10. Kaplan-Meier curves of Progression-Free Survival in ER-Positive Patients. (Fig. 10A) Note that an absence of stromal Cav-1 immunostaining also predicts poor clinical outcome in ER-positive patients (p=5.94 x $10^{-7}$, log-rank test), which represents a total of 80 breast cancer patients, 5-year PFS is indicated by an arrow. (Fig. 10B) The status of epithelial Cav-1 is shown for comparison. n.s., denotes not significant.

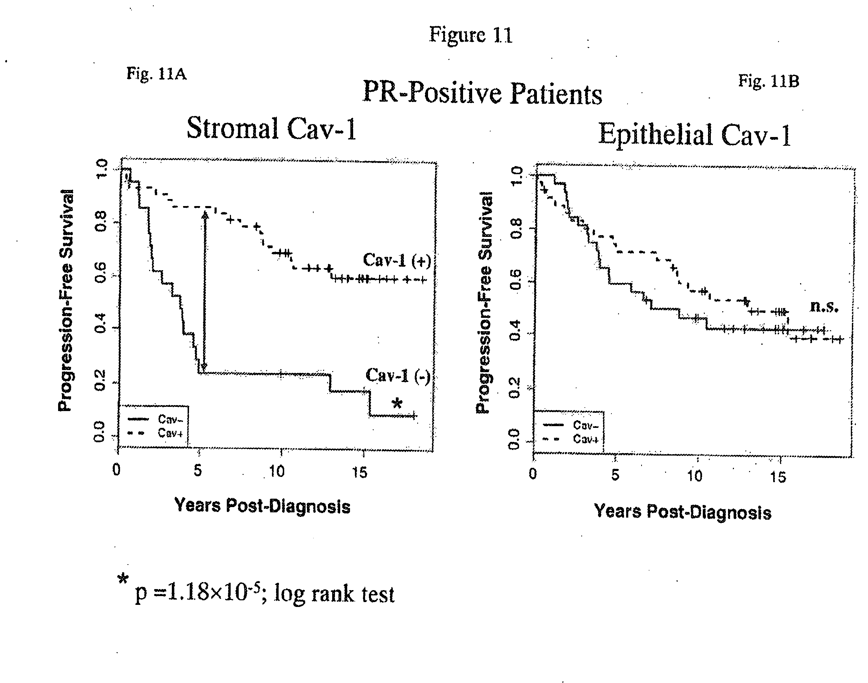

Figure 11. Kaplan-Meier curves of Progression-Free Survival in PR-Positive Patients. (Fig. 11A) Note that an absence of stromal Cav-1 immunostaining also predicts poor clinical outcome in PR-positive patients (p=1.18 x $10^{-5}$, log-rank test), which represents a total of 65 breast cancer patients. 5-year PFS is indicated by an arrow. (Fig. 11B) The status of epithelial Cav-1 is shown for comparison. n.s., denotes not significant.

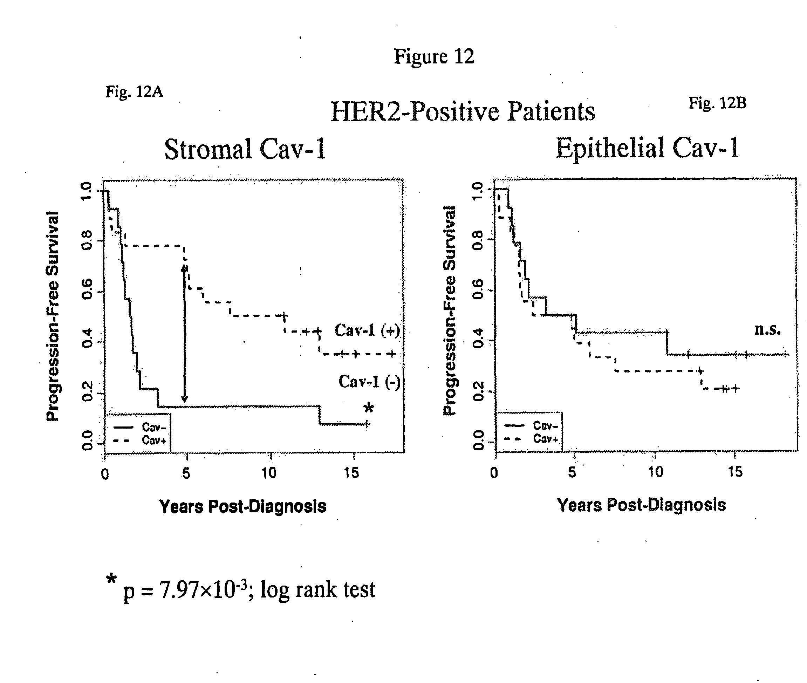

Figure 12. Kaplan-Meier curves of Progression-Free Survival in HER2-Positive Patients. (Fig. 12A) Note that an absence of stromal Cav-1 immunostaining also predicts poor clinical outcome in HER2-positive patients (p=7.97 x $10^{-3}$, log-rank test), which represents a total of 32 breast cancer patients. 5-year PFS is indicated by an arrow. (Fig.

12B) The status of epithelial Cav-1 is shown for comparison. n.s., denotes not significant.

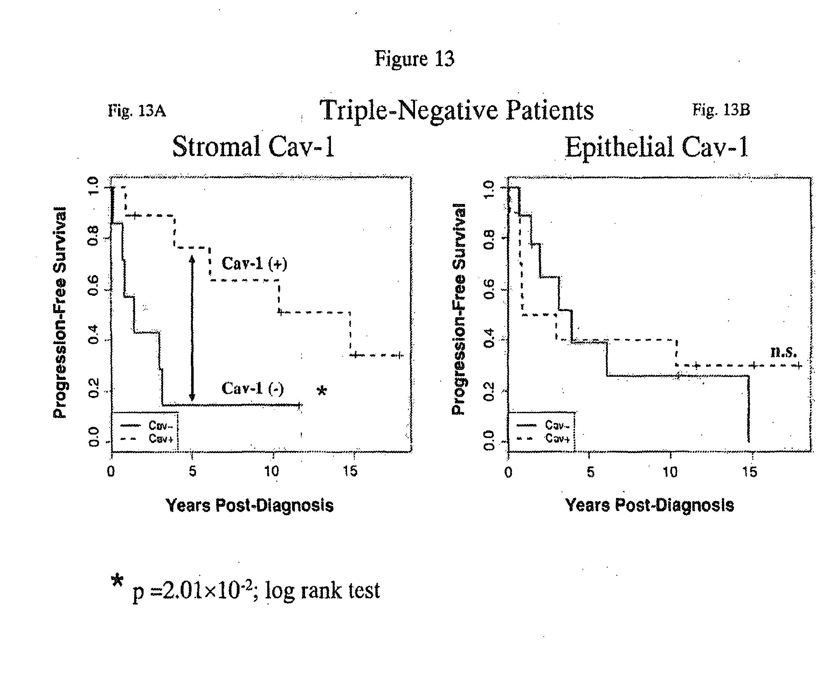

Figure 13. Kaplan-Meier curves of Progression-Free Survival in Triple-Negative Patients. (Fig. 13A) Note that an absence of stromal Cav-1 immunostaining also predicts poor clinical outcome in triple-negative (ER-/PR-/HER2-) patients (p=2.01 x $10^{-2}$, log-rank test), even though this subset of the patient population is small (16 patients). 5-year PFS is indicated by an arrow. (Fig. 13B) The status of epithelial Cav-1 is shown for comparison. n.s., denotes not significant.

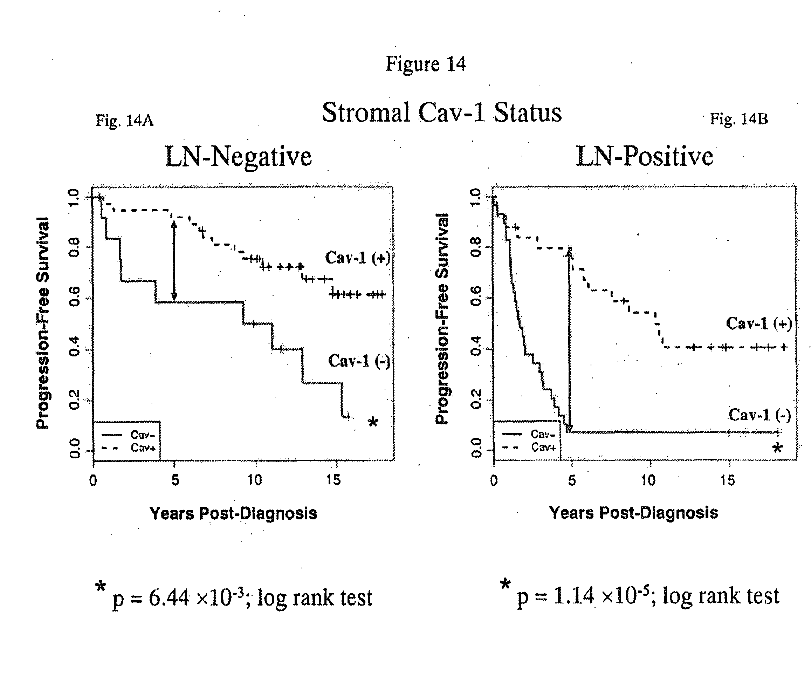

Figure 14. Kaplan-Meier curves of Progression-free Survival in Lymph Node-Negative and Positive Patients. Note that in both LN(-)(Fig. 14A) and LN(+)(Fig. 14B) patients, an absence of stromal Cav-1 still remains a significant predictor of progression-free outcome. However, the results were most dramatic in LN(+) patients, where an absence of stromal Cav-1 is associated with an ~11.5-fold reduction in.5-year progression-free survival. There were 50 patients in the LN(-) group and 54 patients in the LN(+) group. p-values are as shown. 5-year PFS is indicated by an arrow.

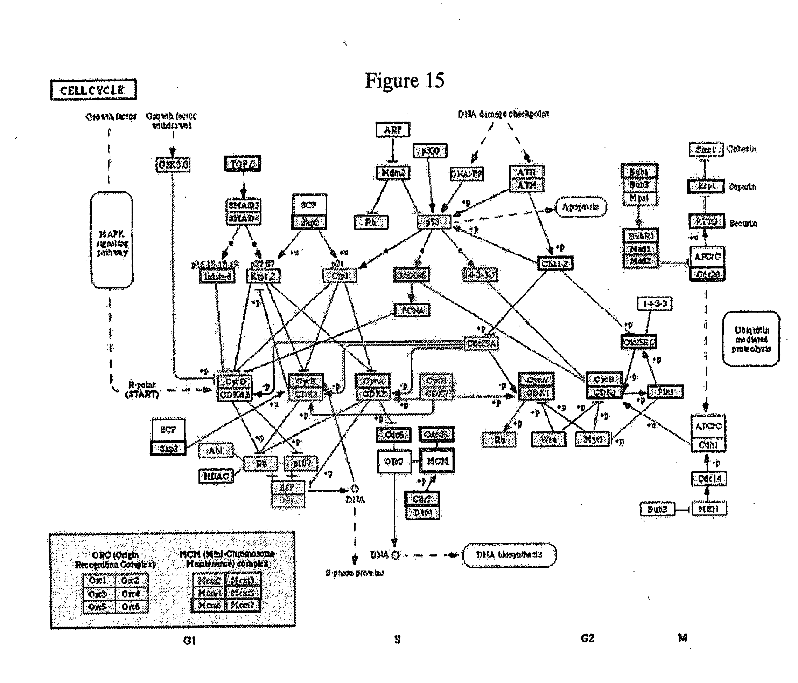

Figure 15. Cav-1 (-/-) MSFs Show the Upregulation of Cell Cycle Associated Genes. The Cell Cyle Pathway from KEGG (Kyoto Encyclopedia of Genes and Genomes) is shown. Cell cycle associated genes that are upregulated in Cav-1 (-/-) MSFs are boxed. KEGG is available online at www.genome.jp/kegg/.

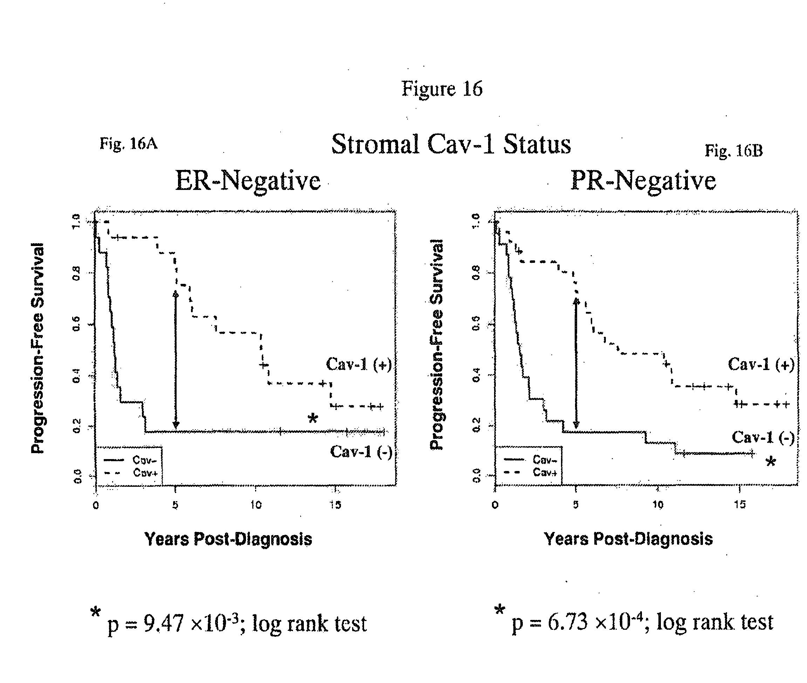

Figure 16. Kaplan-Meier curves of Progression-Free Survival (PFS) in ER-Negative and PR-Negative Patients. Note that in both ER(-)(Fig. 16A) and PR(-)(Fig. 16B) patients, an absence of stromal Cav-1 still remains a significant predictor of progression-free outcome. There were 35 patients in the ER(-) group and 51 patients in the PR(-) group. p-values are as shown. 5-year PFS is indicated by an arrow.

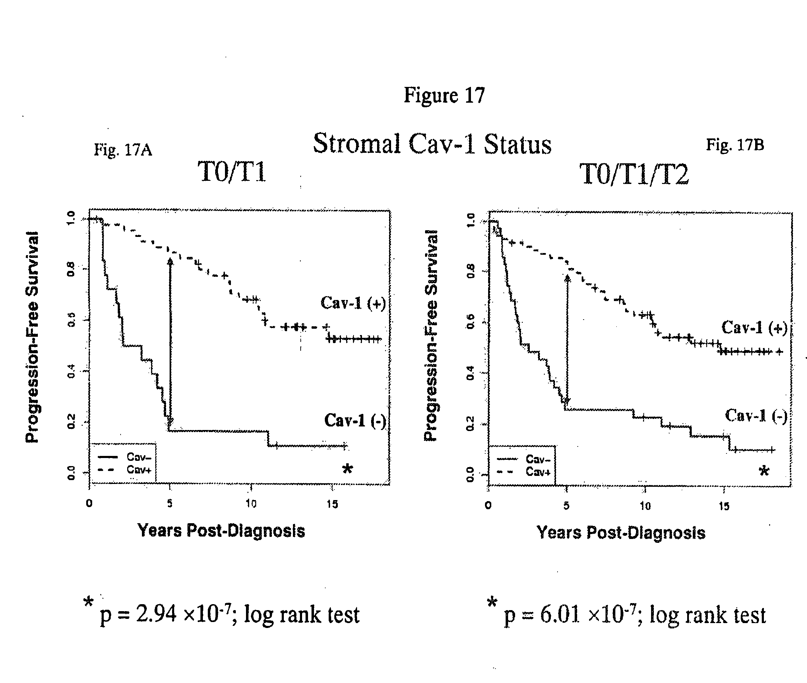

Figure 17. Kaplan-Meier curves of Progression-Free Survival (PFS) in Low Tumor Stage Patients. Note that in low T stage (T0/T1 and T0/T1/T2) patients, an absence of stromal Cav-1 still remains a significant predictor of progression-free outcome. There were 64 patients in the T0/T1 group (Fig. 17A) and 106 patients in the T0/T1/T2 (Fig. 17B) group. p-values are as shown. 5-year PFS is indicated by an arrow.

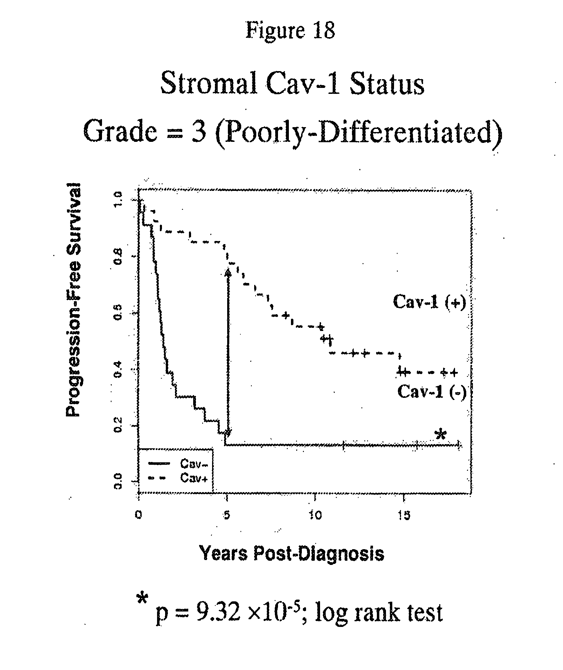

Figure 18. Kaplan-Meier curves of Progression-Free Survival (PFS) in Grade 3 Patients. Note that in grade 3 (poorly-differentiated tumor) patients, an absence of stromal Cav-1 still remains a significant predictor of progression-free outcome. There were 52 patients in the grade 3 group. p-values are as shown. 5-year PFS is indicated by an arrow.

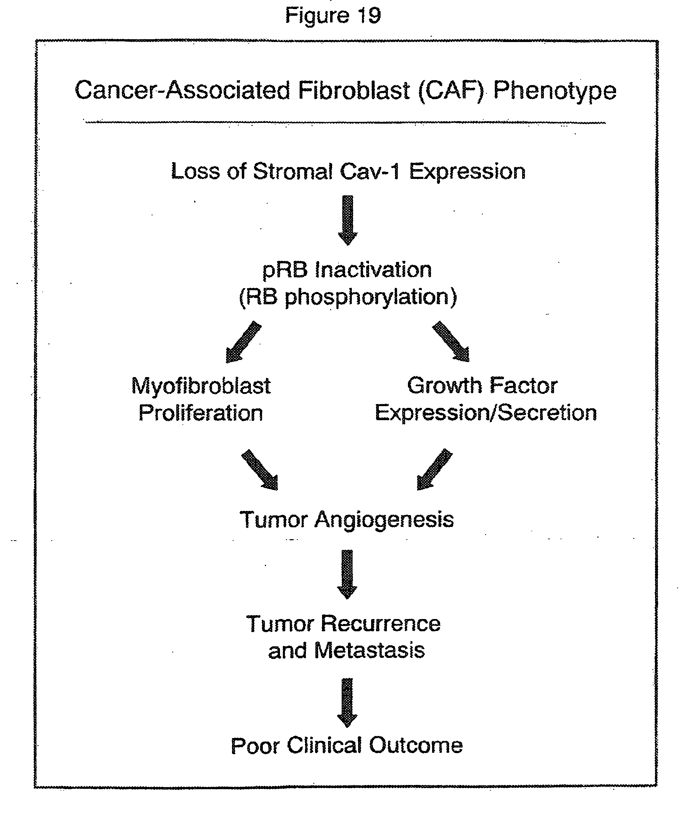

Figure 19. Loss of Stromal Cav-1 Expression Predicts Poor Clinical Outcome in Human Breast Cancer Patients. Mechanistically, loss of stromal Cav-1 expression in the tumor microenvironment leads to RB-inactivation, increased myofibroblast proliferation, and the secretion of angiogenic growth factors. This, in turn, greatly facilitates tumor recurrence and metastasis, leading to poor clinical outcome.

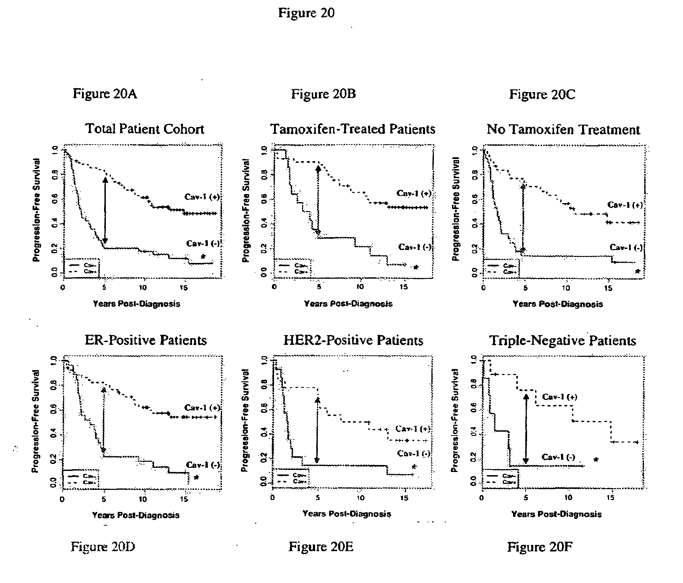

Figure 20. An Absence of Stromal Caveolin-1 Expression Predicts Early Tumor Recurrence and Poor Clinical Outcome in Human Breast Cancers. Total patient cohort is shown as Figure 20A. Note that stromal Cav-1 is a powerful predictive biomarker for estimating a patient's risk of recurrence and survival in all 3 of the most common classes of breast cancer, which are based on ER (Figure 20D), PR (Figure 20F), and HER2 (Figure 20E) expression. Its behavior in tamoxifen-treated (Figure 20B) *versus* non-tamoxifen-treated (Figure 20C) patients is also shown for comparison. An asterisk (*) denotes statistical significance. P values ranged from $10^{-9}$ to $10^{-2}$, depending on the patients selected for analysis. See Witkiewicz et al., 2009.

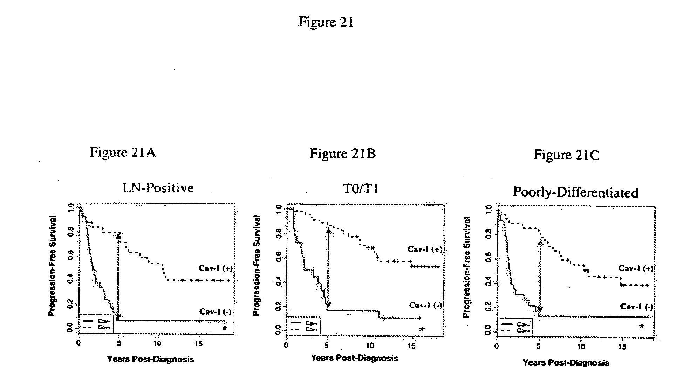

Figure 21. Stromal Cav-1 Expression Correlated to Pathologic Features: Lymph Node-Positivity, Early Tumor Stage, and Advanced Tumor Grade. Stromal Cav-1 is actually very effective in lymph-node positive patients (Figure 21A), showing an 11.5 fold-stratification of 5-year progression free survival (Cav-1 (+), 80% survival versus Cav-1 (-), 7% survival). Stromal Cav-1 is also a valuable predictive marker across all tumor grades and even in early stage (T0/T1) tumor patients (Figure 21B). Its behavior in grade-3 (poorly differentiated) tumors is shown here (Figure 21C). An asterisk (*) denotes statistical significance. LN, lymph node. P values ranged from 10-7 to 10-5, depending on the patients selected for analysis. See Witkiewicz et al., 2009.

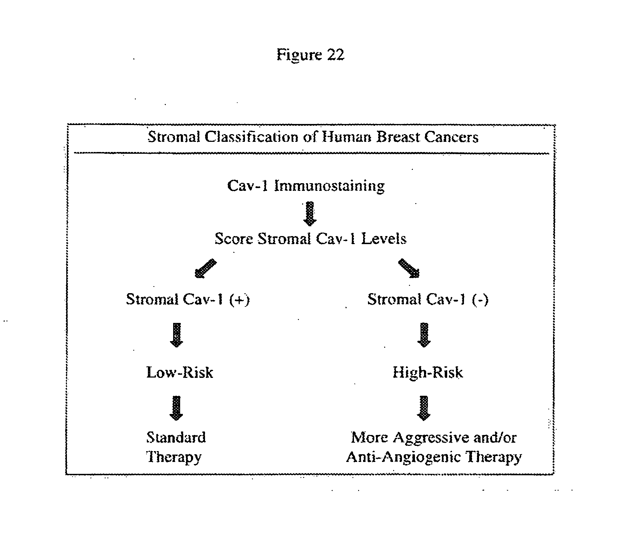

Figure 22. A New "Stromal-Based" Classification System for Human Breast Cancers. In this new "simplified" classification system, patients showing expression of stromal Cav-1 would be considered low-risk and given standard treatments, while patients showing an absence of stromal Cav-1 would be considered high-risk and selected for more aggressive therapies, especially those that target tumor angiogenesis

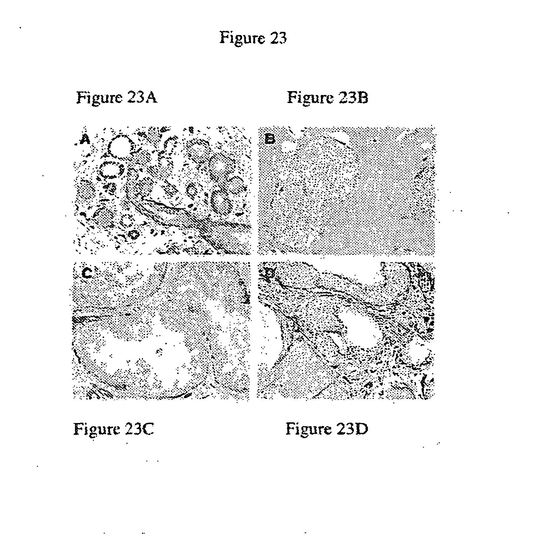

Figure 23. Cav-1 Immunostaining in Normal Breast and Ductal Carcinoma In Situ (DCIS).

Representative examples are shown. Figure23A: Focal weak staining in stromal fiboblasts, in normal terminal duct lobular units. Figure23B: Score = 0 (no stromal Cav-1 staining) in DCIS.

Figure23C: Weak expression of stromal Cav-1 (Score = -1) in DCIS. Figure23D: Strong diffuse staining in stromal fibroblasts in DCIS, representing Score = 2.

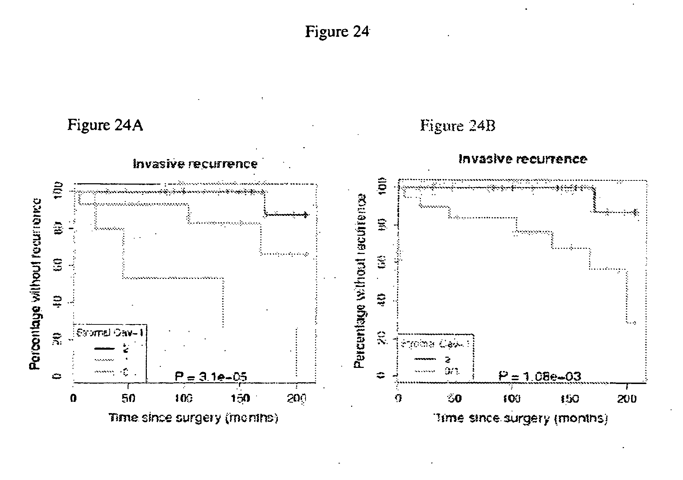

Figure 24. Kaplan-Meier curves for Stromal Cav-1 Status and Time to Invasive Recurrence among ER(+) DCIS patients. Note that an absence or reduction of stromal Cav-1 is strongly associated with an increase in progression to invasive breast cancer. Figure 24A" patients were stratified into 3 different groups. Figure 24B, patients with low or absent Cav-1 were considered as a single group. P values (log rank test) are as shown.

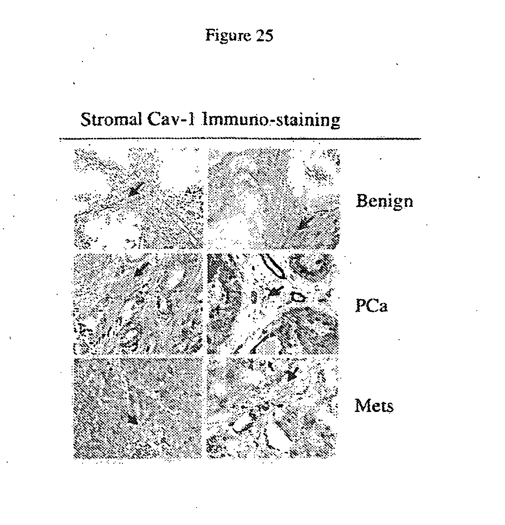

Figure 25. Expression of Stromal Cav-1 in Benign, Primary Prostate Cancers, and Metastatic Tumor Samples. Two images are shown for each diagnostic category. Note that benign prostate samples abundantly express stromal Cav-1. Primary prostate cancers showed differential expression of stromal Cav-1, with dither high (left), moderate (not shown), or absent/low expression (right). Finally, metastatic tumor samples (either from lymph node (at left) or bone (at right)) showed an absence of stromal Cav-1 staining. Note that in ail cases, the vasculature remained Cav-1 positive. Arrowheads point at the tumor stroma in all 6 panels.

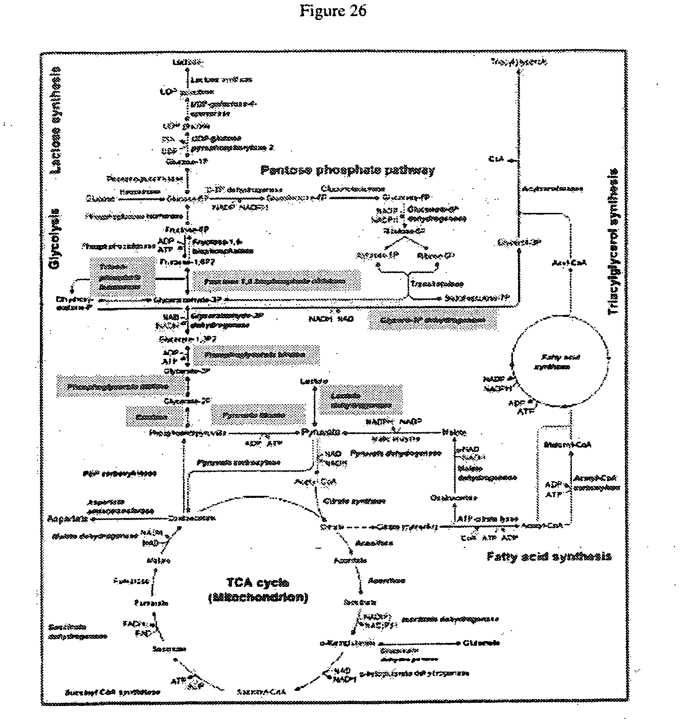

Figure 26. Genetic Ablation of Cav-1 in Stromal Fibroblasts Up-regulates Eight Metabolic Enzymes Associated with the Glycolytic Pathway. Enzymes and metabolites that are part of glycolysis, the pentose phosphate pathway, fatty acid synthesis, triglyceride synthesis, lactose synthesis, and the TCA cycle are shown. Note that the protein spots identified by proteomics analysis/mass spec (listed in Table 11) are 8 enzymes associated with the glycolytic pathway (highlighted in pink). This list includes pyruvate kinase, a key enzyme which is sufficient to mediate the Warburg effect, driving aerobic glycolysis in tumors. This diagram was modified from Beddek et al., 2008, Proteomics, 8; 1502-1515, an article on the proteomics of the lactating mammary gland, and is based on the KEGG pathway database (www.genome.jp/kegg/pathway.html).

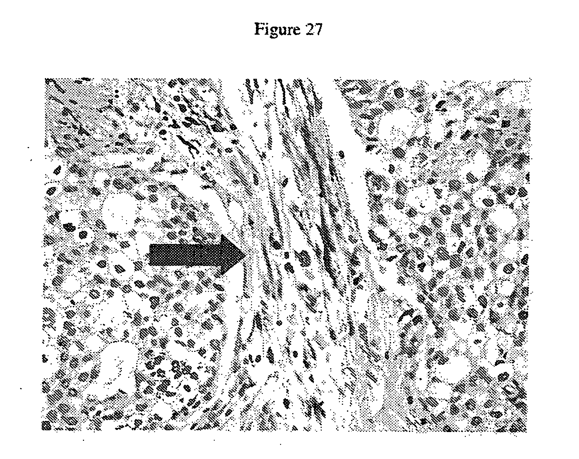

Figure 27. The M2-Isoform of Pyruvate Kinase (M2-PK) is Highly Over-Expressed in the Stroma of Human Breast Cancers that Lack Stromal Cav-1 Expression. A number of human breast cancer cases that lack stromal Cav-1 expression were selected for pre-screening new biomarkers. The image shows a representative example of M2-PK stromal staining (see arrow). Note that sections from the same tumor show a loss of Cav-1 staining in the tumor stromal compartment. These results directly demonstrate the feasibility and success of the proteomics analysis. Thus, for the first time, inventor has now identified that the Warburg effect can originate in the tumor stroma. Note the absence of positive M2-PK staining in the epithelial breast cancer cells. Virtually identical results were obtained with 2 independent M2-PK specific antibodies that do not recognize the M1-isoform.

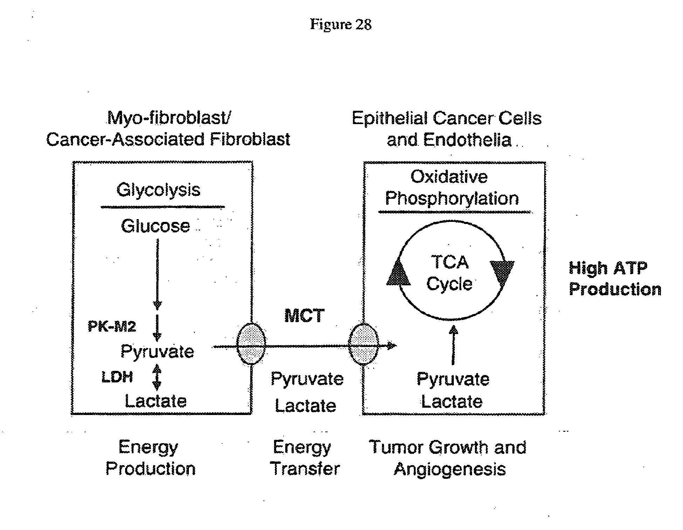

Figure 28. The Reverse Warburg Effect: Aerobic Glycolysis in Cancer Associated Fibroblasts (CAFs) and the Tumor Stroma. Epithelial cancer cells induce the Warburg effect (aerobic glycolysis) in neighboring stromal fibroblasts. These cancer-associated fibroblasts, then undergo myo-fibroblastic differentiation, and secrete lactate and pyruvate (energy metabolites resulting from aerobic glycolysis). Epithelial cancer cells then take up these energy-rich metabolites and use them in the mitochondrial TCA cycle, thereby promoting efficient energy production (ATP generation via oxidative phosphorylation), resulting in a higher proliferative capacity.

Figure 28A and Figure 28B provide complementary views of the model. Transfer of pyruvate/lactate from myo-fibroblasts to epithelial cancer cells and endothelia occur via a monocarboxylate transporter (MCT), such as MCT1/4. Thus, CAFs and the tumor epithelial cells are metabolically coupled.

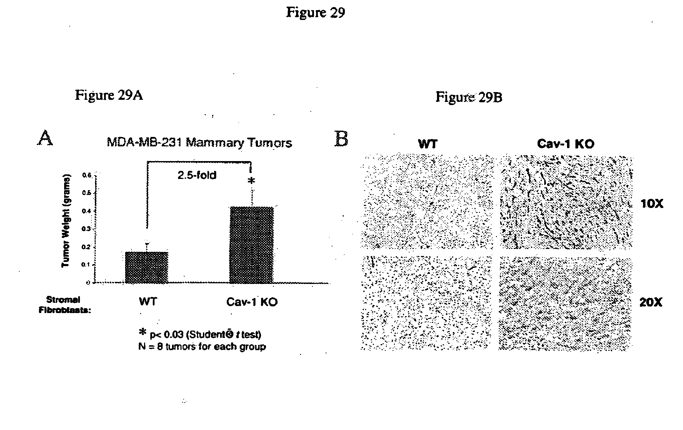

Figure 29. An Absence of Stromal Cav-1 Actively Promotes Mammary Tumor Growth and Angiogenesis in a Xenograft Model Using Human Breast Cancer Cells. Figure 29A, Note that an absence of stromal Cav-1 (KO) increases tumor weight by -2.5-fold (virtually identical results were obtained by measuring tumor volume). Figure 29B, Note also that an absence of stromal Cav-1 (KO) promotes angiogenesis, as seen by CD31 immuno-staining of breast cancer tumor xenograft sections. Original magnifications are as indicated, 10X and 20X. WT, wild-type stromal fibroblasts; KO, Cav-1 (-/-) null stromal fibroblasts.

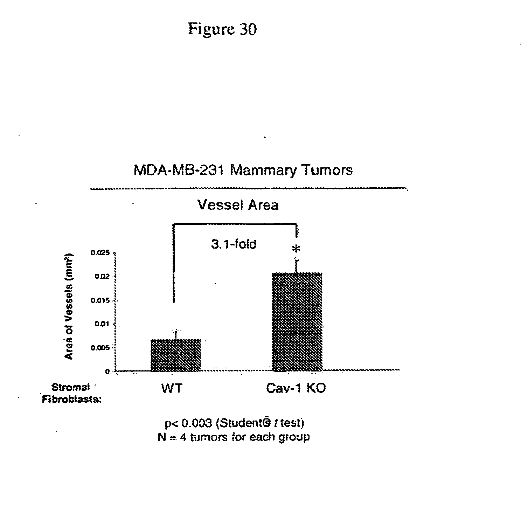

Figure 30. An Absence of Stromal Cav-1 Promotes Mammary Tumor Angiogenesis in a Xenograft Model Using Human Breast Cancer Cells. Note that an absence of stromal Cav-1 (KO) increases tumor vessel area by -3.1-fold (virtually identical results were obtained by measuring tumor vessel number). For quantification of CD31-positive vessels, images were captured of one 20x field from the central region of each tumor section, representing an area of 0.56 mm2 or 560,000 micron$^2$. The total area of each vessel was calculated using Image J and the data is represented graphically. WT, wild-type stromal fibroblasts; KO, Cav-1 (-/-) null stromal fibroblasts.

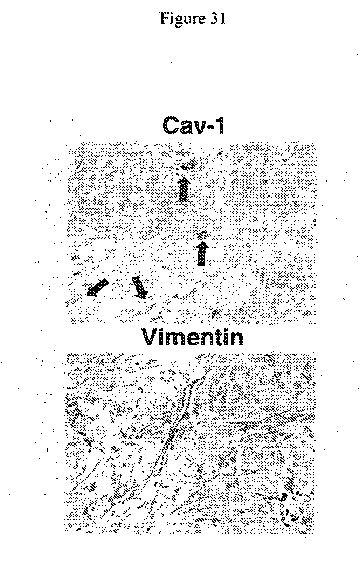

Figure 31. Vimentin is Highly Over-Expressed in the Stroma of Human Breast Cancers that Lack Stromal Cav-1 Expression. A number of human breast cancer cases that lack stromal Cav-1 expression were selected for pre-screening new biomarkers. The upper panel shows a representative example of a loss of Cav-1 stromal staining; however, note that the endothelial vasculature still remains Cav-1 positive (see arrows). Note that sections from the same tumor show the over-expression of vimentin in the tumor stromal compartment. These results directly demonstrate the feasibility and success of the proteomics analysis and co-culture pre-screening approach.

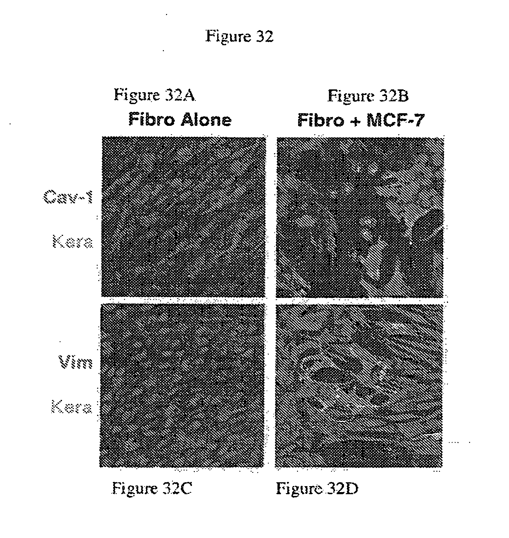

Figure 32. Human Breast Cancer Cells Down-Regulate Cav-1 Expression in Adjacent Stromal Fibroblasts, and Up-Regulate Vimentin. Upper Panel: Loss of Cav-1 in Stromal Fibroblasts. MCF-7 cells were co-cultured for 7 days with stromal fibroblasts (Figure 32B). Note that these fibroblasts show a loss of Cav-1 expression. Their Nuclei are marked with white asterisks. Fibroblasts cultured alone are also shown for comparison (Figure 32A). Both Upper panels were triply-stained for Cav-1 (red), Pan-cytokeratin (green), and Nuclei (blue). 40X magnification. Lower Panel: Increased Vimentin in Stromal Fibroblasts. Figure 32D, Stromal Fibroblasts. MCF-7 cells were co-cultured for 7 days with stromal fibroblasts, triply-stained for Vimentin (red), Pan-cytokeratin (green), and Nuclei (blue). Figure 32C. Fibroblasts cultured alone and triply-stained for Vimentin, Pan-cytokeratin, and Nuclei are also shown for comparison Note that vimentin is increased in stromal fibroblasts co-cultured with MCF-7 cells. 30X magnification. The same exposure settings were used in all panels. It is important to note that MCF-7 cells alone do not express Cav-1 or vimentin (data not shown). Also, fibroblasts alone fail to express epithelial keratins. So, these markers are relatively cell-type specific. Nuclei were counter-stained with the fluorescent-dye, Hoechst.

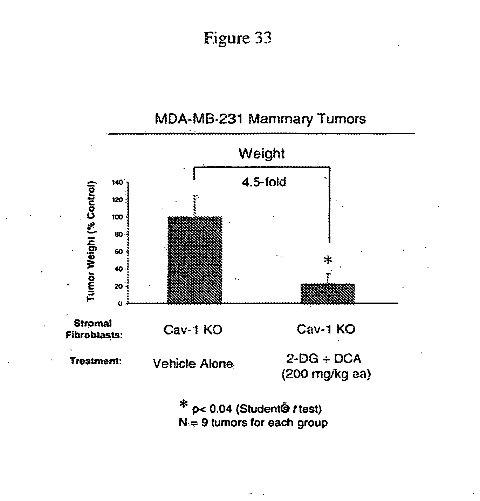

Figure 33. Treatment with Glycolysis Inhibitors Functionally Blocks Cancer-Associated Fibroblast Induced Breast Cancer Tumor Growth. Based on the proteomics studies, the Warburg effect in the myo-fibroblast compartment is a key factor driving tumor growth. Mice co-injected with Cav-1-deficient stromal fibroblasts and MDA-MB-231 breast cancer cells were treated with glycolysis inhibitors: The efficacy of two well-established glycolysis inhibitors, namely 2-DG (2-deoxy-D-glucose) and DCA (dichloro-acetate), individually or in combination were tested. Individually 2-DG or DCA had no effect on tumor growth at a dosage of 200 mg/kg (not shown). However, 2-DG and DCA, used in combination, dramatically reduced tumor growth that was dependent on Cav-1-deficient stromal fibroblasts. Note the observed striking 4.5-fold reduction in tumor mass, as predicted.

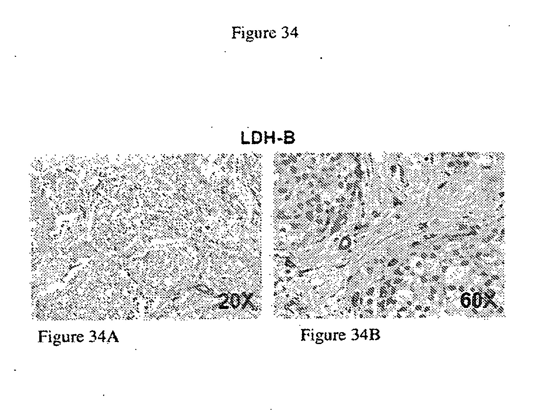

Figure 34. LDH-B is Highly and Selectively Expressed in the Breast Cancer Tumor Stroma. Paraffin-embedded tissue sections from human breast cancer samples were immuno-stained with antibodies directed against LDH-B. Slides were counterstained with hematoxylin. Note that breast cancer tumor sections show the over-expression of LDH-B selectively in the tumor stromal compartment. Tumor cell "nests" surrounded by LDH-B positively-stained stromal fibroblasts were observed. Original magnification, 20X (Figure 34A), and 60X (Figure 34B), as indicated.

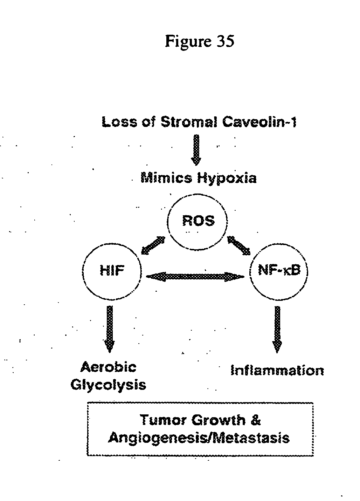

Figure 35. Schematic Diagram Summarizing the Results of the Informatics Analysis of Cav-1 (-/-) Deficient Mesenchymal Stromal Cell Transcriptional Profiles. The that loss of Cav-1 leads to oxidative stress and ROS over-production. This, in turn, leads to activation of NFkB and HIF target genes.

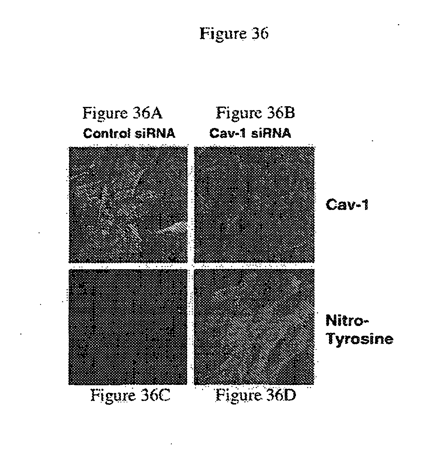

Figure 36. Acute Knock-Down of Cav-1 Leads to Increased Nitro-Tyrosine Production. Fibroblasts were transiently transfected with siRNAs targeting Cav-1 (Figure 36B and Figure 36D) or a scrambled control siRNA (Figure 36A and Figure 36C). Note that a loss of Cav-1 leads to increased nitro-tyrosine staining as visualized with specific antibody probes.

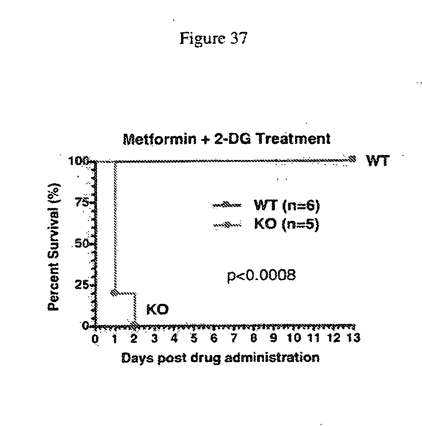

Figure 37. Cav-1 (-/-) Deficient Mice Have Reduced Mitochondrial Reserve Capacity. WT and Cav-1 (-/-) deficient mice (KO) were injected daily with a combination of a mitochondrial complex I inhibitor (Metformin; 200 mg/kg/day per mouse) and a glycolysis inhibitor (2-DG; 500 mg/kg/day per mouse). Note that metabolic restriction with this drug combination was lethal in Cav-1 KO mice. 80% of Cav-1 KO died on the first day after injection, and 100% of Cav-1 KO mice died by the second day after injection. In contrast, WT control mice did not show any negative side effects, even after up to 13 days of daily treatment.

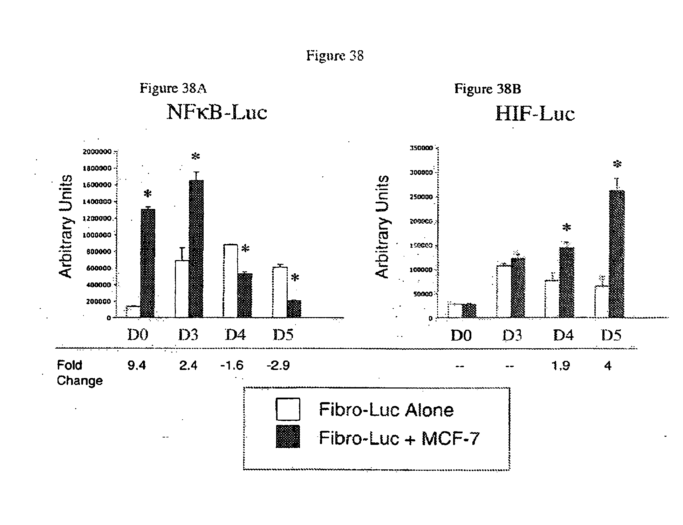

Figure 38. Measuring the Reverse Warburg Effect: A Novel Drug Screening Assay. MCF-7 breast cancer cells were cocultured with fibroblasts engineered to express either NFkB-luciferase or HIF-luciferase transcriptional reporters (Fibro-Luc + MCF-7). For comparison purposes, equal numbers of fibroblasts were also culture alone, in the absence of MCF-7 cells (Fibro-Luc Alone). Note that MCF-7 cells induce a near 10-fold increase in NFkB transcriptional activity in fibroblasts at 8 hours of co-culture (Day 0 (DO)) (Figure 38A), while maximal HIF transcriptional activity in fibroblasts was induced 4-fold on day 5 of coculture (D5) (Figure 38B). An asterisk (*) indicates a significant change in luciferase (Luc) transcriptional activity, $p < 0.05$.

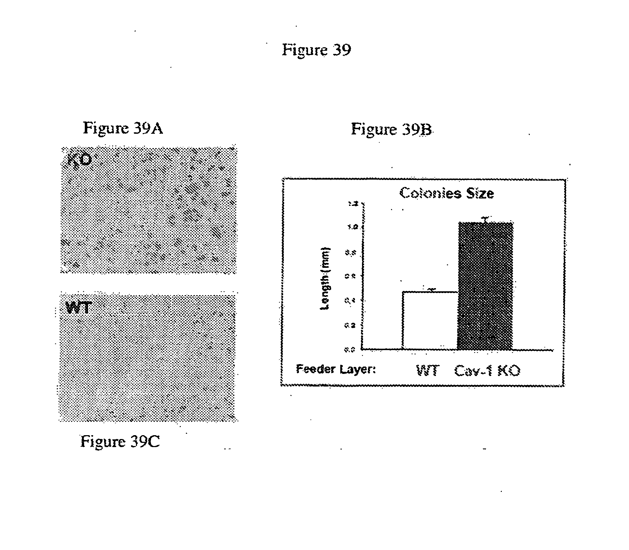

Figure 39. Cav-1 (-/-) Stromal Cells Promote the Growth of Embryonic Stem (ES) Cells in Feeder Layer Cultures. ES cells (the mouse E14 cell line) were cultured on mitomycin C-treated feeder layers consisting of either WT (Figure 39C) or Cav-1 (-/-) deficient fibroblasts (KO) (Figure 39A). Colonies were then visualized with a colorimetric method to identify ES cell colonies via alkaline phosphosphatase staining. Figure 39B, Cav-1 (-/-) deficient fibroblasts (KO) increase ES cell average colony size by greater than 2-fold ($p < 0.05$). Their diameter (a.k.a., length) is shown graphically.

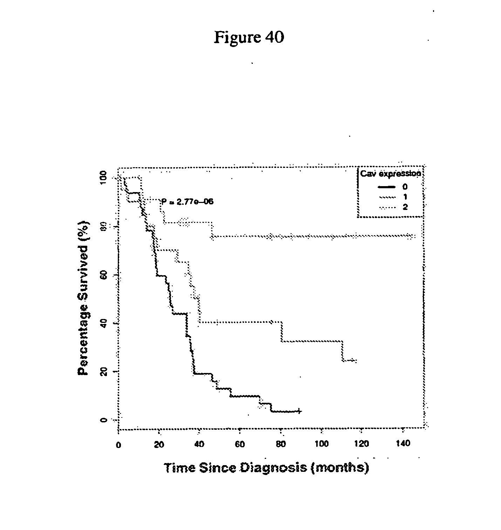

Figure 40. Kaplan-Meier Analysis of Stromal Cav-1 Levels Predicts Overall Survival in Triple Negative Breast Cancer Patients. Of the 88 TN breast cancers examined, 83 could be semi-quantitatively scored for stromal Cav-1 levels. Interestingly, 24 patients showed high levels of Cav-1 stromal staining, while 22 showed a lower, intermediate level of staining, and 37 showed an absence of Cav-1 stromal staining. The results of this analysis were highly statistically significant ($p = 2.8 \times 10^{-6}$). Patients with high-levels of stromal Cav-1 (score = 2), had a good clinical outcome, with >50% of the patients remaining alive during the follow-up period (nearly 12 years). In contrast, the median survival for patients with moderate stromal Cav-1 staining (score = 1) was 33.5 months. Similarly, the median survival for patients with absent stromal Cav-1 staining (score = 0) was 25.7 months.

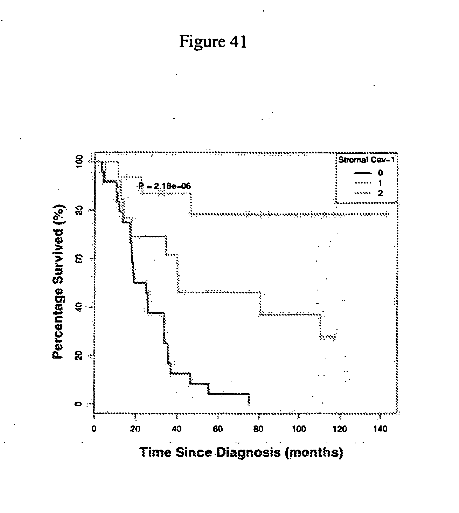

Figure 41. Kaplan-Meier Analysis of Stromal Cav-1 Levels Predicts Overall Survival in Basal-like Triple Negative Breast Cancer Patients. The prognostic value of stromal Cav-1 in basal breast cancer patients was determined, The TN patients who stained positively for either CK5/6 or EGF-R. for inclusion as basal-like breast cancer patients in this analysis. Using this approach, 57 of the TN breast cancer cases were re-classified as basal-like breast cancers. Note that Kaplan-Meier analysis of stromal Cav-1 status in basal-like breast cancer patients was highly statistically significant ($p = 2.2 \times 10^{-6}$). As such, stromal Cav-1 status also has strong prognostic significance in TN patients with the basal-like breast cancer.

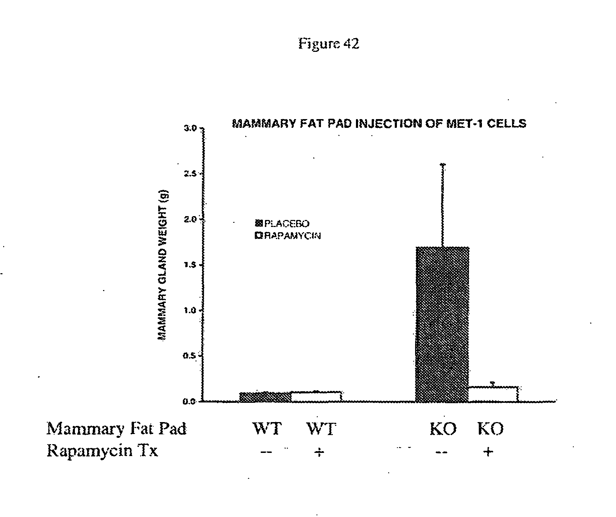

Figure 42. Rapamycin Blocks Tumor Growth that is Induced by the Cav-1 Deficient Tumor Microenvironment. Met-1 cells, an aggressive mouse mammary tumor cell line, were orthotopically implanted into the mammary glands of normal WT FVB mice or Cav-1 (-/-) deficient, FVB mice, and followed over time. At 5 weeks post tumor cell injection, mammary glands were harvested and subjected to a detailed analysis. The results indicate that tumors grown in the Cav-1 (-/-) mammary fat pat microenvironment were greater than 10 times larger, as measured by tumor mass. Tumors grown in the Cav-1 (-/-) mammary fat pat microenvironment showed a striking increase in vascularization due to extensive tumor angiogenesis. Importantly, if mice were treated with a standard dose of rapamycin, this effect was nearly completely abolished. Thus, tumor growth was drastically reduced.

## DETAILED DESCRIPTION OF PREFERRED EMBODIMENTS OF THE INVENTION Human Caveolin

Caveolin-1 is part of a multi-gene family including caveolin-1, caveolin-2 and caveolin-3. Caveolin-1 is a 21-kDa coat/adapter protein of caveolae. Caveolin-1 has a scaffolding domain thought to interact with proteins involved in several signal transduction pathways, e.g. heterotrimeric G proteins, Ha-Ras, c-Src, eNOS, PKC.alpha., MAPK and tyrosine kinase receptors (See e.g., Li et al., J. Biol. Chem. 271:29182-90,1996). Many of these proteins contain a consensus motif for caveolin-1 binding (See Anderson, Annu. Rev. Biochem. 67:199-255,1998). The human caveolin-1 gene is known (See Engelmann et al., FEBS letters 436:403-410,1998). The function of caveolin-1 in human cancers is unclear. Some reports suggest that caveolin-1 functions as a tumor suppressor protein in the NIH-3T3 mouse fibroblast cell line, human breast cancer cell lines and lung carcinoma cell lines (See Koleske et al., Proc. Nad. Acad. Sci. USA 92 (1995), 1381-1385; Lee, S. W. et al., Oncogene 16 (1998),1391-1397; and Racine C. et al., Biochem. Biophys. Res. Commun. 255 (1999). 580-586). U.S. Pat. Nos. 5,783,182 and 6,252,051 disclose that caveolin sequences can be used to identify and target metastatic cells, such as metastatic prostate cancer cells. In addition, CpG islands associated with the caveolin-1 gene are methylated in either primary tumors or tumors-derived cell lines (see Prostate. Feb. 15, 2001;46(3):249-56; FEBS. Lett. Apr. 9, 1999;448(2-3):221-30.). Caveolin-1 and Caveolin-2 are highly homologous proteins that share the same tissue distribution, are co-regulated, and directly interact with each other.

## Caveolin-1 Protein Sequence, NP_001744

```
  1 msggkyvdse ghlytvpire qgniykpnnk amadelsekq vydahtkeid ivnrdpkhln
 61 ddvvkidfed viaepegths fdgiwkasft tftvtkywfy rllsalfgip maliwgiyfa
121 ilsflhiwav vpciksflie iqcisrvysi yvhtvcdplf eavgkifsnv rinlqkei
```

### Caveolin-2 Protein Sequence, AAB88492

```
  1 mgletekadv qlfmdddsys hhsgleyadp ekfadsdqdr dphrlnshlk lgfedviaep
 61 vtthsfdkvw icshalfeis kyvmykfltv flaiplafia gilfatlscl hiwibnpfvk
121 tclmvlpsvq tiwksvtdvi iaplctsvgr cfssvslqls qd
```

## Neoplastic Diseases and Cancer

Cancer is the second leading cause of death in the United States, after heart disease (Boring, C: C. et al., 1993, CA Cancer J. Chin. 43:7), and develops in one in three Americans, and one of every four Americans dies of cancer. Cancer can be viewed as a breakdown in the communication between tumor cells and their environment, including their normal neighboring cells. Signals, both growth-stimulatory and growth-inhibitory, are routinely exchanged between cells within a tissue. Normally, cells do not divide in the absence of stimulatory signals, and likewise, will cease dividing in the presence of inhibitory signals. In a cancerous, or neoplastic state, a cell acquires the ability to "override" these signals and to proliferate under conditions in which normal cells would not grow.

In addition to unhindered cell proliferation, cells must acquire several traits for tumor growth to occur. For example, early on in tumor development, cells must evade the hostimmune system. Further, as tumor mass increases, the tumor must acquire vasculature to supply nourishment and remove metabolic waste. Additionally, cells must acquire an ability to invade adjacent tissue, and ultimately cells often acquire the capacity to metastasize to distant sites.

Cancer of the breast is the most common form of malignant disease occurring among women of the Western World, and it is the most common cause of death among those who are between 40 and 45 years of age.

Cancers that are the subject of the present invention include, but are not limited to, human sarcomas and carcinomas, e.g. carcinomas, e.g., colon carcinoma, pancreatic cancer, breast cancer, ovarian cancer, prostate cancer, fibrosarcoma, myxosarcoma, liposarcoma, chondrosarcoma, osteogenic sarcoma, chondroma, angiosarcoma, endotheliosarcoma, lymphangiosarcoma, lymphangioendotheliosarcoma, synovioma, mesothelioma, Ewing's tumor, leiomyosarcoma, rhabdomyosarcoma, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wiirns' tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, melanoma, neuroblastoma, retinoblastoma, leukemias, e.g., acute lymphocytic leukemia and acute myelocytic leukemia (myeloblastic, promyelocytic, myelomonocytic, monocytic and erythroleukemia); chronic leukemia (chronic myelocytic (granulocytic) leukemia and chronic lymphocytic leukemia); and polycythemia vera, lymphoma (Hodgkin's disease and non-Hodgkin's disease), multiple myeloma, Waldenstrom's macroglobulinemia, and heavy chain disease.

Ductal Carcinoma In Situ (DCIS) - Ductal carcinoma in situ is the most common type of noninvasive breast cancer. In DCIS, the malignant cells have not metastasized through the walls of the ducts into the fatty tissue of the breast. Comedocarcinoma is a type of DCIS that is more likely than other types of DCIS to come back in the same area after lumpectomy, and is more closely linked to eventual development of invasive ductal carcinoma than other forms of DCIS.

Infiltrating (or Invasive) Ductal Carcinoma (IDC) - In IDC, cancerous cells have metastasized through the wall of the duct and invaded the fatty tissue of the breast. At this point, it has the potential to use the lymphatic system and bloodstream for metastasis to more distant parts of the body.

Lobular Carcinoma In situ (LCIS) - While not a true cancer, LCIS (also called lobular neoplasia) is sometimes classified as a type of noninvasive breast cancer. It does not penetrate through the wall of the lobules. Although it does not itself usually become an invasive cancer, women with this condition have a higher risk of developing an invasive breast cancer in the same or opposite breast.

Infiltrating (or Invasive) Lobular Carcinoma (ILC) - ILC is similar to IDC, in that it has the potential to metastasize elsewhere in the body. About 10% to 15% of invasive breast cancers are invasive lobular carcinomas, and can be more difficult to detect by mammogram than IDC.

Inflammatory Breast Cancer - This invasive breast cancer, which accounts for about 1% of all breast cancers, is extremely aggressive. Multiple skin symptoms associated with this cancer are caused by cancer cells blocking lymph vessels or channels in skin over the breast.

**Other Breast Cancer Patient Subgroups**

Virtually identical results were also obtained with ER(-), PR(-), low T stage, and grade 3 patients. In all these additional patient subgroups, an absence of stromal Cav-1 also consistently predicts poor clinical outcome, Data regarding ER (-), PR (-), low T stage and grade 3 patients are shown in Figures 10, 11, and 12. Thus, stromal Cav-1 is a new "universal" or "widely-applicable" breast cancer prognostic marker that is effective in all the well-established breast cancer patient subgroups that we examined.

**Loss of stromal caveolin-1 and/or caveolin-2 expression is a strong predictor of tumor recurrence and dramatically-lower progression-free survival**

Caveolin-1 is expressed in the stroma of invasive breast carcinomas and the Inventors have found that of stromal Cav-1 expression is a strong predictor of tumor recurrence and dramatically lower progression-free survival. Although epithelial Cav-1 expression has been extensively studied in breast carcinomas, there is little or no data on the expression and significance of Cav-1 in the stroma of invasive breast carcinomas. Previous studies demonstrated that epithelial expression of Cav-1 in malignant breast cancer cells correlates with histological grade, loss of ER and PR positivity, and the expression of basal markers including cytokeratins and p63 24. However, in multivariate analysis, epithelial Cav-1 expression was not an independent prognostic factor for patient outcome. Consistent with these published findings, we observed here that epithelial Cav-1 expression was not a prognostic factor for clinical outcome in our patient cohort.

Recently, we showed that CAFs down-regulated Cav-1 protein expression, in conjunction with RB tumor suppressor inactivation 15. Additional studies showed that mammary stromal fibroblasts from Cav-1 (-/-) knock-out mice share a similar gene expression profile with human CAFs 23, and both show the upregulation of RB/E2F responsive genes. Thus, an absence of Cav-1 expression in mammary stromal fibroblasts leads to RB tumor suppressor functional inactivation in vivo, thereby releasing E2F. This, in turn, generates 'activated stromal fibroblasts' that can increase the transcription of a number of cell cycle (S-phase) related genes, including target genes that encode growth promoting factors and cytokines. Loss of caveolin-1 in stromal cells allows for activation of TGF-beta signaling. It has been shown that this activated TGFbeta signaling in CAFs could induce the secretion of growth promoting proteins such as HGF, VEGF, and IL-6.

It remains unknown what causes the down-regulation of Cav-1 in the mammary tumor stroma. However, in experiments with human breast cancer-associated fibroblasts (CAFs), we previously showed that Cav-1 mRNA transcript levels were either increased ~2.3-2.4-fold or not changed, suggesting that the loss of Cav-1 protein expression occurs at a post-transcriptional or post-translational level. Since human breast CAFs show a loss of Cav-1 protein expression, we recently examined the phenotypic behavior of mammary stromal fibroblasts (MSFs) derived from Cav-1 (-/-) null mice. Interestingly, Cav-1 (-/-) MSFs assumed many of the characteristics of CAFs and secreted increased levels of proliferative and pro-angiogenic growth factors, including VEGF 23. In this regard, Cav-1 (-/-) MSFs also underwent endothelial-like trans-differentiation in vitro, with the upregulation of CD31 (Pecam1). In support of the idea that Cav-1 (-/-) MSFs may have increased cellular plasticity, genome-wide transcriptional profiling showed that they also upregulate numerous embryonic stem (ES) cell associated genes. Consistent with these findings, the mammary stromal compartment in Cav-1 (-/-) mice shows dramatically increased vascularization (via CD31 staining) 23 and promotes tumorigenesis in vivo. Thus, based on these mechanistic studies, breast cancer patients lacking stromal Cav-1 will benefit from anti-angiogenic therapy (such as Bevacizumab (a.k.a., Avastin)), in addition to the more standard treatment regimens.

Since ER, PR, and HER2 expression have long served as important epithelial biomarkers for stratifying breast cancer patients into different diagnostic and therapeutic groups, we also assessed the status of stromal Cav-1 in these different patient groups within our cohort. An absence of stromal Cav-1 effectively predicts early tumor recurrence and poor clinical outcome in all 4 groups: ER(+), PR (+), HER2(+), and triple-negative patients (ER-/PR-/HER2). Thus, stromal Cav-1 serves as a new "universal" or "widely-applicable" breast cancer biomarker that can be used to predict early tumor recurrence and clinical outcome across many different "subclasses" of breast cancer. This is a potentially paradigm-shifting notion, and indicates more actively targeting the tumor stroma in therapeutic interventions. Thus, the status of the tumor stroma is a primary determinant of disease recurrence and poor clinical outcome in breast cancer patients.

Loss of Stromal caveolin-1 and/or caveolin-2 expression is closely linked to aggressive biological behaviors, including invasion and metastasis of breast carcinomas. We show the importance of depicting the molecular changes and other phenotypic aspects of stromal-related tumor cells. Uncovering critical molecular events, such as Cav-1 reduction in the mammary tumor stroma, allows unravel the key features of epithelial-stromal cross-talk that are critical for tumor progression and metastasis.

**Loss of Stromal Caveolin-1 and/or Caveolin-2 Expression directly Contributes to the cancer-associated fibroblast phenotype; Other Novel Biomarkers Identified by Gene Profiling of Human Breast Cancer-Associated fibroblasts.**

Interestingly, we recently observed.that human breast cancer-associated fibroblasts show down-regulation of caveolin-1 and/or caveolin-2 protein expression and exhibit facets of RB functional gene inactivation. Thus, loss of caveolin-1 and/or caveolin-2 expression is a critical initiating event leading towards the cancer-associated fibroblast phenotype. Consistent with this, via in vivo transplant studies, we have previously established that the mammary stroma of Cav-1 (-/-) null mice clearly stimulates the growth of both i) normal mammary ductal epithelia and ii) mammary tumor cells.

Loss of stromal Cav-1 expression directly contributes to the cancer-associated fibroblast phenotype. We have found that Cav-1 (-/-) MSFs share many properties with humain CAFs. Like CAFs, Cav-1 (-/-) MSFs show functional, inactivation of RB .via hyper-phosphorylation. Table 2 shows a list of 55 genes that were commonly upregulated in both human breast CAFs and Cav-1 (-/-) MSFs. Table I shows many of these genes are RB/E2F regulated genes, In addition, Cav-1 (-/-) MSFs show the over-expression of 96 RB/E2F target genes. Moreover, we demonstrate that Cav-1 (-/-) MSFs take on the functional characteristics of myofibroblasts, such as: i) contraction/retraction; ii) the upregulation of muscle-related genes (smooth muscle actin, myosin (heavy and light chains), tropomyosin); and iii) the upregulation of TGF-$\beta$ ligands, related factors, and responsive genes (TGF-$\beta$), procollagen genes, interleukin-11, and CTGF). CAFs are thought to mediate their affects through paracrine interactions with mammary epithelial-derived tumor cells. In this regard, we also show that Cav-1 (-/-) MSFs secrete increased amounts of pro-proliferative and pro-angiogenic growth factors, and upregulate the expression of HGF/scatter factor, a key epithelial morphogen. Functionally, conditioned media prepared from Cav-1 (-/-) MSFs is sufficient to induce an epithelial-mesenchymal transition in 3D cultures of Cav-1 (+/+) mammary epithelial cells embedded in Matrigel. Thus, Cav-1 (-/-) MSFs fulfill many of the functional criteria already established for the phenotypic behavior of human CAFs. Here, we also show that Cav-1 (-/-) MSFs demonstrate evidence of activated TGF-$\beta$ signaling and secrete/express increased levels of HGF, VEGF, and IL-6. Similarly, it has been previously shown that TGF-$\beta$ mediated induction of the myofibroblast phenotype induces the enhanced secretion of HGF, VEGF, and IL-6.

Tissue fibroblasts show significant plasticity and are able to differentiate into numerous "terminally differentiated" cell types, including adipocytes and muscle cells, among others. Thus, fibroblasts may also be considered as stem-like progenitor cells. With this in mind, we examined the list of 380 transcripts that were upregulated in Cav-1 (-/-) MSFs and compared them with lists of known ES cell genes and genes controlled by iPS (induced pluripotency)-related transcription factors. Interestingly, Cav-1 (-/-) MSFs show the upregulation of numerous stem/progenitor cell-associated genes (see Table 2). Based on this analysis, it is apparent that Rbm39 (a.k.a., CAPER) is the target of Nanog, Sox2, and Myc, three of the iPS transcription factors. Notably, CAPER is highly upregulated in Cav-1 (-/-) MSFs (~8-fold) and it is known to function as a co-activator of the AP-1 transcription factor and nuclear receptors (such as estrogen and progesterone). Thus, CAPER over-expression is consistent with the idea that Cav-1 (-/-) MSFs may behave more like stem/progenitor cells. In accordance with this idea, we have previously shown that Cav-1 (-/-) mice show an expansion of the epithelial stem/progenitor cell compartment in the skin, mammary gland; and the intestine. This may have important implications for understanding the origin(s) of cancer stem cells within the tumor microenvironment. Interestingly, Mishra et al., 2008 have recently suggested that CAFs have many similarities with and may originate from human bone-marrow mesenchymal stem cells.

In accordance with the hypothesis that fibroblasts can behave as multi-potent progenitor cells, NIH 3T3 fibroblasts treated with "conditioned media" from ES cells undergo endothelial cell trans-differentiation. Conversely, endothelial cells can transdifferentiate into myofibroblasts under the appropriate conditions. Importantly, recent mouse genetic studies have clearly documented that this endothelial-mesenchymal transition (EnMT) frequently occurs in vivo, under pathological conditions including cardiac fibrosis and tumorigenesis. As such, our observation that Cav-1 (-/-) MSFs undergo spontaneous endothelial trans-differentiation is consistent with these findings. Similarly, Cav-1 (-/-) mammary fat pads show dramatically increased vascularization, as compared with WT mice. These findings provide additional support for the idea that Cav-1 (-/-) MSFs can promote mammary stromal angiogenesis and/or undergo endothelial cell trans-differentiation in vivo. Thus, these results have broad implications for understanding the role of cancer-associated fibroblasts in promoting tumor angiogenesis in vivo, via their potential to secrete angiogenic growth factors and to directly undergo endothelial cell trans-differentiation. Pericytes (a.k.a., adventitial cells) are mesenehymal-like vascular mural cells that are associated with connective tissue and are wrapped around the walls of small blood vessels, venules, and capillaries. Interestingly, these undifferentiated pericytes show significant progenitor-like plasticity and can differentiate into several distinct cell types, including fibroblasts, smooth muscle cells, and even macrophages. In addition, pericytes are contractile, express smooth muscle actin, and they function as critical regulators of vascular morphogenesis, angiogenesis, and fibrosis. In this regard, pericytes show striking phenotypic similarities with myofibroblasts and/or cancer-associated fibroblasts. However, pericytes are difficult to define and their origins are still not well understood. Given the abundance of CD31 (+) microvasculature in Cav-1 (-/-) mammary fat pads, it is quite possible that Cav-1 (-/-) MSFs may originate from pericytes. In further support of this idea, pericytes, tumor-associated myofibroblasts, mesenchymal stem cells, and endothelial progenitor cells all express a common tumor-endothelial marker (Tem1), namely endosialin.

**Transcriptional Gene Profiling of Cav-1 (-/-) MSFs Reveals Striking Similarities with Human CAFs, and RB Tumor Suppressor Functional Inactivation.**

Human breast cancer-associated fibroblasts (CAFs) show functional inactivation of the RB tumor suppressor and down-regulation of caveolin-1 (Cav-1) and/or caveolin-2 (Cav-2) protein expression. However, it remains unknown whether loss of Cav-1 is sufficient to confer RB functional inactivation in mammary fibroblasts. Here, to establish a direct cause-effect relationship, we have now employed a genetic approach using Cav-1 (-/-) null mice. Mammary stromal fibroblasts (MSFs) were prepared from wild-type (WT) and Cav-1 (-/-) null mice and subjected to genome-wide transcriptional profiling. The expression of 832 known genes and transcripts was changed in Cav-1 (-/-) MSFs, as compared with Cav-1 (+/+) MSFs; 380 transcripts were up-regulated and 452 transcripts were down-regulated. All of these genes and transcripts changed by> 2-fold and achieved statistical significance ($p < 0.05$). Gene ontology analysis revealed that the 380 upregulated transcripts exhibit a strong enrichment for genes involved in cell cycle control (Table 1). Interestingly, the Cav-1 (-/-) MSF transcriptome significantly overlaps with that of human breast CAFs. We previously reported a CAF gene signature consisting of 118 upregulated known gene transcripts. Table 1 shows a list of 55 genes that were commonly upregulated in both human breast CAFs and Cav-1 (-/-) MSFs. Many of these genes are RB/E2F regulated genes. We also independently scanned the list of 380 transcripts that are upregulated in Cav-1 (-/-) MSFs and identified 96 RB/E2F target genes (See Table 2). Thus, fully one-fourth of the upregulated genes, due to loss of Cav-1, are related to RB functional inactivation.

**Table 1.**

**Common Gene Expression Changes in Human Breast CAFs and Cav-1 (-/-) MSFs.**

| A. Genes Upregulated in both Human Breast CAFs and Cav-1 (-/-) MSFs (55 genes). | | |
|---|---|---|
| Symbol | Gene Name | Fold-Upregulation in Cav-1 KO |
| Anin | anillin, actin binding protein (scraps homolog, Drosophila) | 2.5 |
| Aurka | aurora kinase A | 3.3 |
| Birc5 | baculoviral IAP repeat-containing 5 (survivin) | 3.8 |
| Blm | Bloom syndrome | 2.0 |
| Brca1 | breast cancer 1, early onset | 2.3 |

(continued)

**Common Gene Expression Changes in Human Breast CAFs and Cav-1 (-/-) MSFs.**

**A. Genes Upregulated in both Human Breast CAFs and Cav-1 (-/-) MSFs (55 genes).**

| Symbol | Gene Name | Fold-Upregulation in Cav-1 KO |
|---|---|---|
| Bub1 | BUB1 budding uninhibited by benzimidazoles 1 homolog (yeast) | 3.6 |
| Bub1b | BUB1 budding uninhibited by benzimidazoles 1 homolog beta (yeast) | 2.5 |
| Casc5 | cancer susceptibility candidate 5 | 2.5 |
| Ccnb2 | cyclic B2 | 2.3 |
| Ccnf | cyclin F | 2.1 |
| Cdc20 | CDC20 cell division cycle 20 homolog (S. cerevisiae) | 2.4 |
| Cdc45l | CDC45 cell division cycle 45-like (S. cerevisiae) | 2.0 |
| Cdca31 | cell division cycle associated 1 | 2.4 |
| Cdca3 | cell division cycle associated 3 | 2.7 |
| Cdca5 | cell division cycle associated 5 | 2.1 |
| Cdca8 | cell division cycle associated 8 | 3.0 |
| Cenpa | ceniromere protein A | 3.9 |
| Cenpf | ceniromere protein F, 350/400ka (mitosin) | 2.8 |
| Cenpk | centromere protein K | 2.0 |
| Cep55 | centrosomal protein 55kDa | 3.2 |
| Chap2l | cytoskeleton associated protein 2-like | 2.5 |
| Dlg7 | discs, large homolog 7 (Drosophila) | 2.6 |
| E2f7 | E2F transcription factor 7 | 2.7 |
| Fabp6 | fatty acid binding protein 5 (psoriasis-associated) | 3.9 |
| Foxm1 | forkhead box M1 | 2.4 |
| Hmmr | hyaluronan-mediated mobility receptor (RHAMM) | 3.3 |
| Kif11 | kinesin family member 11 | 2.7 |
| Kif20a | kinesin family member 20A | 2.8 |
| Kif23 | kinesin family member 23 | 2.5 |
| Kif2c | kinesin family member 2C | 3.3 |
| Knic2 | kinetochore associated 2 | 2.6 |
| Mad2l1 | MAD2 mitotic arrest deficient-like 1 (yeast) | 2.3 |
| Mcm10 | MCM10 minichromosome maintenance deficient 10 (S. cerevisiae) | 2.4 |
| Mcm5 | MCM5 minichromosome maintenance deficient 5, cell division cycle 46 (S. cerevisiae) | 3.8 |
| Melk | maternal embryonic leucine zipper kinase | 2.3 |
| Mki67 | antigen identified by monoclonal antibody Ki-67 | 3.1 |
| Nek2 | NIMA (never in mitosis gene a)-related kinase 2 | 2.0 |
| Nusap1 | nuclear and spindle associated protein 1 | 3.1 |
| Oip5 | Opa interacting protein 5 | 2.0 |
| Pbk | PDZ binding kinase | 2.7 |
| Plk1 | polo-like kinase 1 (Drosophila) | 3.2 |
| Prc1 | protein regulator of cytokinesis 1 | 4.0 |
| Prim1 | primase, polypeptide 1,49kDa | 3.7 |
| Pttg1 | pituitary tumor-transforming 1 | 2.4 |
| Racgap1 | Rac GTPase activating protein 1 | 2.1 |
| Rad51ap1 | RAD51 associated protein 1 | 2.7 |
| Shcbp1 | SHC SH2-domain binding protein 1 | 2.9 |
| Spag5 | sperm associated antigen 5 | 2.8 |
| Spbc24 | spindle pole body component 24 homolog (S. cereviaiae) | 2.5 |
| Tk1 | thymidine kinase 1, soluble | 3.3 |

(continued)

**Common Gene Expression Changes in Human Breast CAFs and Cav-1 (-/-) MSFs.**

**A. Genes Upregulated in both Human Breast CAFs and Cav-1 (-/-) MSFs (55 genes).**

| Symbol | Gene Name | Fold-Upregulation in Cav-1 KO |
|---|---|---|
| Top2a | topoisomerase (DNA) II alpha 170kDa | 2.8 |
| Tpx2 | TPX2, microtubule-associated, homolog (Xenopus laevis) | 2.8 |
| Trip13 | thyroid hormone receptor interactor 13 | 2.3 |
| Ttk | TTK protein kinase | 2.5 |
| Tyms | thymidylale synthetase | 3.5 |

**B. Genes Downregulated in both Human Breast CAFs and Cav-1 (-/-) MSFs (8 genes).**

| Symbol | Gene Name | Fold-Downregulation in Cav-1 KO |
|---|---|---|
| Casp1 | caspase 1, apoptosis-related cysteine peptidase (interleukin 1, beta, convertase) | -4.2 |
| Dscril1 | Down syndrome critical region gene 1-like 1 | -3.2 |
| Kcld12 | potassium channel tetramerisation domain containing 12 | -2.1 |
| Mid1 | midline 1 (Opitz/BBB syndrome) | -2.3 |
| Rspo3 | R-spondin 3 homolog (Xenopus laevis) | -2.3 |
| Stmn2 | staihmin-like 2 | -2.2 |
| Tnxb | tenascin XB | -3.2 |
| Zfp38l2 | zinc finger protein 36, C3H type-like 2 | -2.1 |

**Table 2.**

**A Selection of Relevant Gene Changes in Cav-1 (-/-) MSFs.**

| Symbol | Gene Name | Fold-Change in Cav-1 KO |
|---|---|---|
| **Caveolins** | | |
| Cav1 | caveolin, caveolae protein 1 | -116.4 |
| **Muscle-related Genes** | | |
| Acta2 | actin, alpha 2, smooth muscle, aorta | 2.5 |
| Anin | anillin, actin binding protein (scraps homolog, Drosophila) | 2.5 |
| Flnb | filamin, beta | 2.0 |
| Lama2 | laminin, alpha 2 (merosin) | 3.2 |
| Lmod1 | leiomodin 1 (smooth muscle) | 2.1 |
| Myh11 | myosin, heavy polypeptide 11, smooth muscle | 2.0 |
| Myl9 | myosin, light polypeptide 9, regulatory | 2.0 |
| Myo6 | myosin VI | -2.0 |
| Tpm1 | tropomyosin 1, alpha | 2.6 |
| Tpm2 | tropomyosin 2, beta | 2.6 |
| **TGF-beta and Fibrosis Related Genes** | | |
| Bmp6 | bone morphogenetic protein 6 | 2.1 |
| Col1a2 | procollagen, type 1, alpha 2 | 2.6 |
| Col18a1 | procollagen, type XVIII. alpha 1 | -2.8 |
| Smad7 | MAD homolog 7 (Drosaphila) | 2.4 |
| Tgfb2 | transforming growth factor, beta 2 | 2.1 |
| Tgfb3 | transforming growth factor, beta 3 | 3.2 |

(continued)

**Cytokine/Chemokine Signaling**

| | | |
|---|---|---|
| Ccl2 | chemokine (C-C motif) ligand 2 | 2.3 |
| Ccl8 | chemokine (C-C motif) ligand 8 | 2.6 |
| Ccl11 | chemokine (C-C motif) ligand 11 | -12.2 |
| Cxcl7 | chemokine (C-X-C motif) ligand 7 | 2.7 |
| Cxcl16 | chemokine (C-X-C motif) ligand 16 | -3.0 |
| Il11 | interleukin 11 | 2.4 |
| | | |
| Socs2 | suppressor of cytokine signaling 2 | 2.2 |
| Socs3 | suppressor of cytokine signaling 3 | 2.0 |

**Estrogen Receptor Co-activator Genes**

| | | |
|---|---|---|
| Greb1 | gene regulated by estrogen in breast cancer protein | 2.0 |
| Ncoa7 | nuclear receptor coactivator 7 | 4.0 |
| Rbm39 | Rbm39; CAPER ((coactivator of AP-1 and estrogen receptors); EST C79248) | 8.1 |

**Stem Cell Related Genes**

| | | |
|---|---|---|
| Aldh18a1 | aldehyde dehydrogenase 18 family, member A1 | |
| | | 2.0 |
| Cyr61 | cysteine rich protein 61 | 2.5 |
| Hells | helicase, lymphoid specific | 2.7 |
| Mphosph1 | M-phase phosphoprotein 1 | 2.1 |
| | | |
| Rspo2 | R-spondin 2 homolog (Xenopus laevis) | 2.2 |
| Rspo3 | R-spondin 3 homolog (Xenopus laevis) | -2.3 |
| Sprr1a | small proline-rich protein 1A | 2.6 |

**Nucleolar Components/Ribosomal Proteins/RNA-Binding Proteins/RNA Splicing**

| | | |
|---|---|---|
| Hnrpa1 | heterogeneous nuclear ribonucleoprotein A1 | 2.1 |
| Lsm5 | LSM5 homoleg, U6 small nuclear RNA associated (S. cerevisiae) | 2.0 |
| Nol5 | nucleolar protein 5 | 2.2 |
| Npm3 | nucleopiasmin 3 | 2.1 |
| Nusap1 | nucleolar and spindle associated protein 1 | 3.1 |
| Pop4 | processing of precursor 4, ribonuclease P/MRP family, (S. cerevisiae) | 3.0 |
| Raly | hnRNP-associated with lethal yellow | 2.2 |
| Rbm16 | RNA binding motif protein 16 | 2.3 |
| Rbm39 | RNA binding motif protein 39; CAPER: EST C79248 | 8.1 |
| Rpl17 | ribosomal protein L17 | 8.8 |
| Rpl18 | ribosomal protein L18 | 2.0 |
| Rps24 | ribosomal protein S24 | 2.0 |
| Rrm1 | ribonucleotide reductase M1 | 2.8 |
| Snhg3 | small nuclear RNA host gene (non-protein coding) 3 | 2.0 |
| Snord22 | small nucleolar RNA, C/D box 22 | 2.1 |
| Snrpa1 | small nuclear ribonucleoprotein polypoplide A' | 2:1 |
| Snrpd3 | small nuclear ribonucleoprotein 03 | 2.0 |
| Txnl4 | thioredoxin-like | 2.1 |
| U2al1 | U2 small nuclear ribonucleoprotein auxiliary factor (U2AF) 1 | 2.2 |

These results were validated by immunofluorescence and Western blot analysis. Figure 5A,B shows that RB is indeed hyper-phosphorylated in Cav-1 (-/-)MSFs. However, total RB levels remain unchanged in Cav-1 (-/-) MSFs, as seen by immunoblotting. Thus, loss of Cav-1 expression is sufficient to confer functional inactivation of the RB tumor suppressor protein. Consistent with RB hyperphosphorylation and cell cycle progression, Cav-1 (-/-) MSFs showed an ~2.7-fold increase in BrdU incorporation. Genes that were consistently upregulated in Cav-1 (-/-) MSFs were utilized to cluster a breast cancer data set to determine their impact on disease outcome. Specifically, we observed that the Cav-1 (-/-) MSF gene expression signature correlated with an increased risk of recurrence after tamoxifen mono-therapy (Figure 12A,B). Note that this 103- member gene signature consists mainly of RB/E2F-regulated genes (See Table 3). Thus, high expression of the Cav-1 (-/-) MSFs gene signature was associated with a greater than 2-fold decrease in disease-free survival, in breast cancer patients treated with tamoxifen mono-therapy.

**Table 3**

Human Homologues of the Cav-1 (-/-) MSF Gene Signature that are Predictive of Poor Clinical Out come in Tamoxifen-Treated Breast Cancer Patients. 103 **upregulated transcripts are listed.**

| Symbol | Gene Name |
| --- | --- |
| ANLN | anillin, actin binding protein (scraps homolog, Drosophila) |
| ASP1B | ASF1 anti-silencing function 1 homolog B (S. cerevisiae) |
| ASPM | asp (abnormal spindle)-like, microcephaly associated (Drosophila) |
| ATAD2 | ATPase family, AAA domain containing 2 |
| AURKB | aurora kinase B |
| BIRC5 | baculoviral IAP repeat-containing 5 |
| BLM | bleomycin hydrolase |
| BRCA1 | breast cancer 1 |
| BRRN1 | barren homolog (Drosophila) |
| BUB1 | budding uninhibited by benzimidazoles 1 homolog (S. cerevisiae) |
| BUB1B | budding uninhibited by benzimidazoles 1 homolog, beta (S. cerevisiae) |
| C18ORF24 | Pldn; pallidin (mouse) |
| C6ORF173 | 2610036L11Rik; RIKEN cDNA 2610036L11 gene (mouse) |
| CCNA2 | cyclin A2 |
| CCNB1 | cyclin· B1 |
| CCNB2 | cyclin B2 |
| CONE2 | cyclic E2 |
| CCNF | cyclin F |
| CDC2A | cell division cycle 2 homolog A (S. pombe) |
| CDC20 | cell division cycle 20 homolog (S. cerevisiae) |
| CDC25B | cell division cycle 25 homolog B (S. cerevisiae) |
| CDC25C | cell division cycle 25 hamolog C (S. cerevisiae) |
| CDC45L | cell division cycle 45 homolog (S. cerevisiae)-like |
| CDC6 | cell division cycle 6 homolog (S. cerevisiae) |
| CDCA1 | cell division cycle associated 1 |
| DCA3 | cell division cycle associated 3 |
| CDCA5 | cell division cycle associated 5 |
| CDCA8 | cell division cycle associated 8 |
| CDKN3 | cyclin-dependent kinase inhibitor 3 |
| CENPA | centromere protein A |
| CENPE | centromere protein B |
| CENPF | centromere protein F |
| CHEK1 | checkpoint kinase 1 homolog (S. pombe) |
| CIT | citron |
| CKS1B | CDC28 protein kinase 1b |
| CKS2 | CDC28 protein kinase regulatory subunit 2 |

(continued)

Human Homologues of the Cav-1 (-/-) MSF Gene Signature that are Predictive of Poor Clinical Out come in Tamoxifen-Treated Breast Cancer Patients. 103 **upregulated transcripts are listed.**

| Symbol | Gene Name |
| --- | --- |
| DEPDC1B | DEP domain containing 1B |
| DHFR | dihydrofolate reductase |
| DLG7 | discs, large homolog 7 (Drosophila) |
| ECT2 | ect2 oncogene |
| ESPY1 | extra spindle poles-like 1 (S. cerevisiae) |
| EXO1 | exonucloase 1 |
| EZH2 | enhancer of zeste homolog 2 (Drosophila) |
| FAM33A | Ska2; 1110001A07Rik; RIKEN cDNA 1110001A07 gene (mouse) |
| FAM64A | 6720460F02Rik; RIKEN cDNA 6720460F02 gene (mouse) |
| FEN1 | flap structure specific endonuclease 1 |
| FIGNL1 | fidgetin-like 1 |
| FOXM1 | forkhead box M1 |
| GMMN | geminin |
| GSG2 | germ cell-specific gene 2 |
| H2AFX | H2A histone family, member X |
| HELLS | helicase, lymphoid specific |
| HMGB2 | high mobility group box 2 |
| HMMR | hyaluronan mediated mobility receptor (RHAMM) |
| HN1 | hematological and necrological expressed sequences 1 |
| INCENP | inner centromere protein |
| XQGAP3 | IQ motif containing GTPase activating protein 3 |
| KIAA0101 | p15 (FAF); 2810417H13Rik; RIKEN cDNA 2810417H13 gene (mouse) |
| KIAA0841 | LOC666519; 2310022K01Rik; RIKEN oDNA 2310022K01 gene (mouse) |
| KIF11 | kinesin family member 11 |
| KIF20A | kinesin family member 20A |
| KIF23 | kinesin family member 23 |
| KIF2C | kinesin family member 2C |
| HIF4A | kinesin family member 4A |
| KIFC1 | kinesin family member Cl |
| KNTC1 | kinetochore associated 1 |
| KNTC2 | kinetochore associated 2 |
| LIG1 | ligase I, DNA, ATP-dependent |
| LMNB1 | lamin B1 |
| LUZP5 | leucine zipper protein 5 |
| MAD2L1 | MAD2 (mitotic arrest deficient, homolog)-like 1 (yeast) |
| MASTL | microtubule associated serine/threonine kinase-like |
| MCM10 | minichromosome maintenance deficient 10 (S. cerevisiae) |
| MCM3 | minichromosome maintenance deficient 3 (S. cerevisiae) |
| KCM6 | minichromosome maintenance deficient 6 (MISS homolog, S.pombe & S. cerevisiae) |
| MELK | maternal embryonic leucine zipper kinase |
| MXI67 | antigen identified by monoclonal antibody Ki 67 |
| MYBL2 | myeloblastosis oncogene-like 2 |
| NEK2 | NIMA (never in mitosis gene a)-related expressed kinase 2 |
| NUSAP1 | nucleolar and spindle associated protein 1 |

(continued)

Human Homologues of the Cav-1 (-/-) MSF Gene Signature that are Predictive of Poor Clinical Out come in Tamoxifen-Treated Breast Cancer Patients. 103 **upregulated transcripts are listed.**

| Symbol | Gene Name |
|---|---|
| NXT1 | NTF2-related export protein 1 |
| OIP5 | Cpa interacting protein 5 |
| PBK | small nuclear ribonucleoprotein D3 |
| PCNA | proliferating cell nuclear antigen |
| PPIL5 | peptidylprolyl isomerase (cyclophilin) like 5 |
| PRC1 | protein regulator of cytokinesis 1 |
| PRIM1 | DNA primase, p49 subunit |
| RACGAP1 | Rac GTPase-activating protein 1 |
| RAD51 | RAD51 homolog (S. cerevisiae) |
| RAD51AP1 | RAD51 associated protein 1 |
| KFC5 | replication factor C (activator 1) 5 |
| SGOL1 | shugoshin-like 1 (S. pombe) |
| SHCBP1 | Shc SH2-domain binding protein 1 |
| SPAGS | sperm associated antigen 5 |
| TACC3 | transforming, acidic coiled-coil containing protein 3 |
| TERF1 | telomeric repeat binding factor 1 |
| TOP2A | topoisomerase (DNA) II alpha |
| TPX2 | TPX2, microtubule-associated protein homolog (Xenopus laevis) |
| TIP13 | thyroid hormone receptor interactor 13 |
| TTK | Ttk protein kinase |
| TYMS | thymidylate synthase |
| UBE2C | ubiquitin-conjugating enzyme E2C |
| UHRF1 | ubiquitin-like, containing PHD and RING finger domains. 1 |

RB/E2F regulated genes are listed in BOLD.

**Table 4.**

| Proteome Analysis of Cav-1 (-/-) MSF Secreted Proteins | | | |
|---|---|---|---|
| NAME | KO (pg/ml) | WT (pg/ml) | D CHANGE |
| **F-BB** | 2820 $\pm$ 359.5 | ND | $\infty$ |
| **VEGF** | 139.64 $\pm$ 7.72 | 63.5 $\pm$ 2.1 | 2.6** |
| **MIP2** | 47.74 $\pm$ 10.36 | 18.5 $\pm$ 3.26 | 2.6* |
| **MIP1-□lpha** | 3.12 $\pm$ 0.04 | 1.42 $\pm$ 0.24 | 2.2** |
| **GM-CSF** | 13.92 $\pm$ 0.96 | 7.38 $\pm$ 0.34 | 1.9** |
| **TARC** | 234.22 $\pm$ 17.98 | 147.62 $\pm$ 4.48 | 1.6* |
| **L-Selectin** | 100.2 $\pm$ 4.2 | 65.9 $\pm$ 4.48 | 1.5* |
| | | | |
| **IL-6** | 7675.78 $\pm$ 137.46 | 3903.12 $\pm$ 140.72 | 2.0*** |
| **IL-10** | 74.46 $\pm$ 8.44 | 45.86 $\pm$ 0.7 | 1.6* |
| **IL-13** | 170.78 $\pm$ 12.3 | 124.54 $\pm$ 10.74 | 1.4* |
| **NOTE: No Changes (NC)** were observed in the levels of TGFbeta1, Leptin, IL-18, IFN-gamma, MIP1beta, KC, OPN. RANTES, P-Selectin, SDF1beta, CRP, JE, E-Selectin, MMP9. and sVCAM1. $* = p<0.05; ** = p<0.001; *** = p< 0.0001$. ND, not detectable. | | | |

**Cav-1 (-/-) MSFs Behave like Myofibroblasts and Show Evidence of Activated TGF-β Signaling.**

Human breast CAFs possess many of the characteristics of activated myofibroblasts, and this activation process is thought to be controlled by TGF-β signaling. Thus, we examined the expression of muscle-related genes in Cav-1 (-/-) MSFs. Table 2 shows a list of these relevant gene changes observed in Cav-1 (-/-) MSFs. Some of the musclespecific gene transcripts that are upregulated include smooth muscle actin (SMA), anillin, merosin, myosin (heavy and light chains), and tropomyosin. Similarly, genes transcripts related to activated TGF-β signaling and fibrosis were upregulated, including collagen I and interleukin-11, and the TGF-β ligand itself. Transcripts of proteins associated with the nucleolus (site of ribosome biogenesis), ribosomal proteins, and genes associated with RNA splicing, were also upregulated Table 2. Interestingly, there is an emerging relationship between the nucleolus, ribosome biogenesis, and cell transformation.

Several different approaches to functionally validate the potential myofibroblastic phenotype of Cav-1 (-/-) MSFs were used. The cytoskeletal organization of Cav-1 (-/-) MSFs was visualized using FTTC-phalloidin. As predicted, Cav-1 (-/-) MSFs showed more intense FTTC-phaloidin staining, with thicker F-actin based stress fibers consistent with a more myofibroblastic phenotype. The distribution of Cav-1 immuno-staining is shown for comparison. Remarkably, extended culture of confluent Cav-1 (-/-) MSFs (for 4 days) with ascorbic acid consistently resulted in retraction/contraction, indicative a more myo-fibrolastic phenotype. WT mammary fibroblasts did not undergo retraction/contraction. For Cav-1 (-/-) MSFs, 8 out of 8 35 mm dishes plated showed this retraction phenotype. In contrast, for Cav-1 (+/+) MSFs, 0 out of 8 35 mm dishes showed detachment/retraction. An arrow points at the area of retraction/contraction. Activation of TGF-beta/Smad signaling is thought to be one of the major cell signaling mechanisms that confers a myofibroblastic phenotype. Interestingly, we have previously demonstrated that Cav-1 functions as a kinase inhibitor of the TGF-beta type I receptor. Thus, loss of Cav-1 would be predicted to result in constitutive TGF-beta/Smad signaling. Consistent with this hypothesis, our microarray analysis showed upregulation of SMA and IL-11, Table 2, as well as other TGF-beta/Smad responsive genes. To independently validate the increased expression of SMA, we subjected Cav-1 (+/+) and Cav-1 (-/-) MSFs to RT-PCR analysis. Consistent with TGF-beta/Smad activation, RT-PCR analysis of Cav-1 (-/-) MSFs showed quantitative increases in SMA (4-fold), collagen I (1.9-fold), and CTGF (connective tissue growth factor; 2.1-fold)-all TGFbeta/ Smad responsive genes. Interestingly, the co-upregulation of IL-11 and CTGF in human breast cancers has been shown to be associated with a poor prognosis, and an increased risk of metastatic disease. Similarly, the increased expression of collagen I in Cav-1 (-/-) MSFs was independently validated by immunofluorescence analysis and is shown. Overall, these data show that Cav-1 (-/-) MSFs are more myo-fibroblastic, likely due to constitutive TGF-beta/Smad signaling.

**Estrogen Receptor Signaling, HGF/Scatter Factor Expression, and the Epithelial-to- Mesenchymal Transition (EMT).**

Several estrogen-receptor (ER) co-activator genes were upregulated in Cav-1 (-/-) MSFs, showing that ER signaling is activated. These included Rbm39 (a.k.a., CAPER), Ncoa, and Greb1 23 Table 2. Since CAPER showed the highest level of up-regulation, we chose to validate its expression by immunofluorescence analysis. CAPER protein expression is dramatically elevated in Cav-1. (-/-) MSFs. Since CAPER functions as an ER-coactivator gene at the level of the nucleus; the nuclear distribution of CAPER in Cav-1 (-/-) MSFs may reflect its constitutive activation. Consistent with this hypothesis, a number of estrogenregulated genes are appropriately upregulated or downregulated in Cav-1 (-/-) MSFs. Many of these genes are known RB/E2F regulated genes, as estrogen also drives proliferation in certain cell types.

Estrogen/ER signaling normally controls HGF expression and secretion in mammary stromal fibroblasts. HGF expression in Cav-1 (-/-) MSFs was assessed. The levels of HGF expression in Cav-1 (-/-) MSFs are significantly increased by ~10-fold.

The secretion of certain know stromal cell factors (HGF/scatter factor) from mammary fibroblasts is thought to profoundly regulate the phenotypic behavior of mammary epithelial cells: Stromally-derived HGF normally induces an epithelial-mesenchymal transition (EMT) in mammary epithelia, driving their conversion from a mammary epithelial phenotype to a more invasive myo-epithelial/myo-fibroblastic phenotype 24. Thus, we hypothesized that Cav-1 (-/-) MSFs may secrete increased levels of growthpromoting and EMT-promoting factors.

Single-cell suspensions of WT mammary epithelial cells were overlaid onto a 3D Matrigel culture, and stimulated them with "conditioned media" from Cav-1 (+/+) and Cav-1 (-/-) MSFs for a period of 4 days. Then, we subjected these cultures to immunostaining with smooth muscle actin (α-SMA) to visualize the onset of an EMT, and co-staining with propidium iodide (PI) to visualize the distribution of cell nuclei.

Interestingly, our results directly show that "conditioned media" derived from Cav-1 (-/-) MSFs has a profound effect on the phenotypic behavior and morphology of WT mammary epithelial cells. Notably, we observed that Cav-1 (-/-) MSFs "conditioned media" drives the onset of an EMT in normal WT mammary epithelial cells. Under these conditions, WT mammary epithelia fail to form normal 3D acinar structures, but instead appear as clusters of flattened fibroblastic cells that are positive for immuno-staining with SMA, an EMT-marker.

**Proteome Analysis of Cav-1 (-/-) MSF Secreted Factors.**

Conditioned media derived from Cav-1 (+/+) and Cav-1 (-/-) MSFs was subjected to broad-spectrum ELISA analysis to detect potential differences in their patterns of secreted factors. The concentrations of approximately 40 secreted factors (growth factors, cytokines, and chemokines) were quantitatively evaluated by ELISA (Pierce SearchLight Multiplexed Proteome Arrays) and expressed as pg/ml. The results are summarized in Table 3. Interestingly, the secretion of several pro-angiogenic or pro-tumorgenic factors was significantly increased in Cav-1 (-/-) MSFs, such as PDGF, VEGF, MIP2, MIP1 alpha, and GMCSF, among others (TARC, L-Selectin, IL-6, IL-10, and IL-13). Cav-1 (-/-) MSFs have the Capacity to Undergo Endothelial-like Trans-differentiation. As Cav-1 (-/-) MSF conditioned media showed the up-regulation of a number of proangiogenic growth factors (Table 3), we next assessed their potential to undergo endothelial cell differentiation. Since collagen I is important for endothelial cell differentiation and endothelial "tube formation", we optimized the expression and secretion of collagen I by treating Cav-1 (+/+) and Cav-1 (-/-) MSFs with ascorbic acid at confluency. Twenty-four hours later, we assayed these MSFs for the expression of a number of endothelial-specific markers by RT-PCR. Interestingly, our results directly demonstrate that Cav-1 (-/-) MSFs show the clear upregulation of a number of endothelial-specific marker genes and pro-angiogenic factors, as compared with Cav-1 (+/+) MSFs treated identically (Table 4). These endothelial and pro-angiogenic markers included: Pecaml, Tek, Pgf, Plau, II-6, Tbx4, Tgfb3, Col18a1, PDGF-A, Timpl, and Vegf-C. Notably, Pecaml gene expression was increased in Cav-1 (-/-) MSFs by -17.5-fold. It is important to note that most of these markers were not upregulated when subconfluent Cav-1 (-/-) MSFs were examined by gene expression profiling (DNA microarray). Thus, these findings appear to be specific for confluent Cav-1 (-/-) MSFs monolayers treated with ascorbic acid. Furthermore, when Cav-1 (-/-) MSFs confluent monolayers were cultured for extended periods of time (30 days), they underwent spontaneous endothelial "tube formation". Thus, under certain culture conditions that optimize collagen I production. Cav-1 (-/-) MSFs biochemically and functionally undergo endothelial-like transdifferentiation. To determine the in vivo relevance of these findings, we next assessed the vascularization of Cav-1 (-/-) mammary fat pads. For this purpose, we immuno-stained frozen sections derived from age-matched WT and Cav-1 (-/-) virgin female mammary glands with a well-established endothelial cell marker protein, namely CD31 (Pecam1). Cav-1 (-/-) mammary fat pads show dramatically increased vascularization, as compared with WT mice. These findings are consistent with the idea that Cav-1 (-/-) MSFs can promote mammary stromal angiogenesis and/or undergo endothelial cell trans-differentiation *in vivo*.

**Table 5**

Median Progression-Free Survival (PFS; years) According to Stromal Cav-1 Expression. P-values are based on log-rank tests on the stratified Kaplan-Meier curves. * Denotes that less than half the at-risk patients had an event, resulting in no estimate of median PFS.

| | Stromal Cav-1 | | |
| --- | --- | --- | --- |
| | Absent | Present | P-value |
| Low T stage (0,1 or 2) | 2.59 | 14.76 | $6.01\times10^{-7}$ |
| High T stage (3 or 4) | 1.58 | 4.61 | $1.22\times10^{-1}$ |
| No nodes | 10.20 | * | $6.44\times10^{-3}$ |
| Nodes > 0 | 1.73 | 10.38 | $1.14\times10^{-5}$ |
| Grade = 1 | 4.21 | 11.86 | $4.89\times10^{-2}$ |
| Grade = 2 | 3.11 | * | $1.17\times10^{-4}$ |
| Grade = 3 | 1.43 | 10.84 | $9.32\times10^{-5}$ |
| ER Negative | 1.25 | 10.46 | $9.47\times10^{-3}$ |
| ER Positive | 3.23 | * | $5.94\times10^{-7}$ |
| PR Negative | 1.53 | 7.58 | $6.73\times10^{-4}$ |
| PR Positive | 3.73 | * | $1.18\times10^{-5}$ |
| HER2 Negative | 3.16 | * | $1.06\times10^{-6}$ |
| HER2 Positive | 1.58 | 9.21 | $7.97\times10^{-3}$ |
| ER-/PR-/HER2- | 1.43 | 14.76 | $2.01\times10^{-2}$ |
| No Tamoxifen | 1.66 | 10.84 | $7.74\times10^{-5}$ |

(continued)

Median Progression-Free Survival (PFS; years) According to Stromal Cav-1 Expression. P-values are based on log-rank tests on the stratified Kaplan-Meier curves. * Denotes that less than half the at-risk patients had an event, resulting in no estimate of median PFS.

| | Stromal Cav-1 | | |
|---|---|---|---|
| | Absent | Present | P-value |
| Tamoxifen | 3.55 | * | $4.61 \times 10^{-5}$ |
| white | 1.94 | 14.76 | $6.17 \times 10^{-8}$ |
| Other | 2.04 | * | $1.18 \times 10^{-2}$ |
| LVI Negative | 3.86 | * | $4.71 \times 10^{-6}$ |
| LVI Positive | 1.53 | 6.81 | $7.02 \times 10^{-3}$ |
| ER-/PR-/HER2- represents "triple-negative patients". | | | |

**Table 6**

Cox regression of Progression-Free Survival (PFS) on T stage, N stage, Tamoxifen use and Cav-1 score. We find that the Cav-1 score is statistically significant even adjusting for T-stage N-stage and Tamoxifen use. The baseline level has T stage=T0/T1, N stage=N0, no Tamoxifen and Cav-1 present. Model is based on 101 observations due to missing data.

| | N | Coefficient | Hazard ratio | SE(Coef) | Z-score | P value |
|---|---|---|---|---|---|---|
| T stage | | | | | | |
| T0/T1 (ref) | 51 | | | | | |
| T2 | 37 | 0.097 | 1.102 | 0.315 | 0.307 | $7.6 \times 10^{-1}$ |
| T3/T4 | 13 | 0.789 | 2.202 | 0.401 | 1.966 | $4.9 \times 10^{-2}$ |
| N stage | | | | | | |
| N0 (ref) | 48 | | | | | |
| N1 | 31 | 0.458 | 1.581 | 0.34 | 1.345 | $1.8 \times 10^{-1}$ |
| N2/N3 | 22 | 1.439 | 4.215 | 0.372 | 3.872 | $1.1 \times 10^{-4}$ |
| Tamoxifen use | | | | | | |
| No (ref) | 53 | | | | | |
| Yes | 48 | -0.476 | 0.621 | 0.274 | -1.738 | $8.2 \times 10^{-2}$ |
| Stromal Cav-1 | | | | | | |
| Present (ref) | 62 | | | | | |
| Absent | 39 | 1.272 | 3.569 | 0.292 | 4.352 | $1.3 \times 10^{-5}$ |

**Table 7**

Association of Stromal Cav-1 with 5-Year Progression-Free Survival (PFS).

| Patient Groups | Stromal Cav-1 Status | 5-Year Progression-Free Survival (PFS) | | | |
|---|---|---|---|---|---|
| | | Patients Alive & at Risk | Patient Death/Recurrence | Percent of Patients Alive with No Recurrence | P-value |
| 1-Tamoxifen-Treated | Absent | 4 | 10 | 28.6% | $2.42 \times 10^{-5}$ |
| | Present | 37 | 4 | 90.2% | |
| 2-Without Tamoxifen Treatment | Absent | 4 | 24 | 14.3% | $6.21 \times 10^{-6}$ |
| | Present | 22 | 8 | 73.3% | |
| Total Patients (1+2) | Absent | 8 | 34 | 19.1% | $2.10 \times 10^{-11}$ |
| | Present | 59 | 12 | 83.1% | |

Test used: Fisher's exact test

**Table 8:**

Association of Stromal Cav-1 with 5-Year Progression-Free Survival (PFS) in Lymph Node-Positive and -Negative Patients.

| Patient Groups | Stromal Cav-1 Status | Patients Alive & at Risk | 5-Year Progression-Free Survival (PFS) | | P-value |
|---|---|---|---|---|---|
| | | | Patient Death/Recurrence | Percent of Patients Alive with No Recurrence | |
| 1-LN-Positive | ent | 2 | 27 | 6.90% | $6.87 \times 10^{-6}$ |
| | Present | 19 | 5 | 79.17% | |

**Transcriptional Comparison of Cav-1 (-/-) MSFs with CAFs Isolated from Individual Patients.**

Because of the striking phenotypic similarities between Cav-1 (-/-) MSFs and human CAFs, we also analyzed their transcriptional similarity with CAFs obtained from individual patients. This additional analysis was performed as comparison with only the CAF gene signature may underestimate their similarity. Nearly 50% of the genes up-regulated in Cav-1 (-/) MSFs are also up-regulated in CAFs; similarly, nearly 30% of the genes down-regulated in Cav-1 (-/-) MSFs are also down-regulated in CAFs. It is rare to see such concordance between human patient samples and a mouse animal model. For example, comparison of patients 1, 2, and 3 with each other previously yielded a gene signature of 118 up-regulated genes and 66 down-regulated genes 6. The statistical significance for the transcriptome intersection by using hyper-geometric probabilities for any two groups of genes was calculated. By considering the commonality between human and mouse platforms based upon identical transcript identifiers, we generated a p-value for the interesting sets. All these comparisons were statistically significant at p < 0.009.

**An Absence of Stromal Caveolin-1 Expression Predicts Early Tumor Recurrence, Metastasis, Tamoxifen-Resistance, and Poor Clinical Outcome in Human Breast Cancers.**

A loss of Cav-1 in the breast tumor stroma has prognostic significance. A well-annotated breast cancer tissue microarray (TMA) was immuno-stained with antibodies against Cav-1 and scored its stromal expression. This breast cancer TMA consisted of 160 consecutive patients, with 3 random cores from each patient's tumor, and -20 years of follow-up data. The results directly show that loss or an absence of stromal Cav-1 is strongly associated with advanced tumor and nodal staging, early disease recurrence, lymph node metastasis, tamoxifen-resistance, and poor clinical outcome. Using a multivariate Cox regression analysis approach, an absence of stromal Cav-1 was shown to be a powerful independent prognostic marker.

Most importantly, an absence of stromal Cav-1 predicted poor clinical outcome independently of all the epithelial markers tested (see Figure 20). Thus, loss of stromal Cav-1 has predictive value in Ear(+), PR (+), HER2 (+), and the so-called triple-negative patients (ER (-)/PR (-)/HER2 (-)) (2). It was actually the most effective in lymph-node positive patients (see Figure 21), showing an 11.5 fold-stratification of 5-year progression free survival (Cav-1 (+), 80% survival versus Cav-1 (-), 7% survival) (2). Stromal Cav-1 was also a valuable predictive marker across all tumor grades, and even in early stage tumor patients (Figure 21).

**A new "stromal-based" classification System for human breast cancer prognosis and therapy.**

Here, an absence of stromal Cav-1 expression in human breast cancers is a powerful single independent predictor of early disease recurrence, metastasis and poor clinical outcome (Witkiewicz, et al. (2009) Cell Cycle 8, 1654-8.). These findings have been validated in two independent patient populations. Importantly, the predictive value of stromal Cav-1 is independent of epithelial marker status, making stromal Cav-1 a new "universal" or "widely-applicable" breast cancer prognostic marker. Based on the expression of stromal Cav-1, breast cancer patients can be stratified into high-risk and low-risk groups. High-risk patients showing an absence of stromal Cav-1 should be offered more aggressive therapies, such as anti-angiogenic approaches, in addition to the standard therapy regimens (see Figure 22). Mechanistically, loss of stromal Cav-1 is a surrogate biomarker for increased cell cycle progression, growth factor secretion, "sternness", and angiogenic potential in the tumor microenvironment. Since almost all cancers develop within the context of a stromal microenvironment, this new stromal classification system is broadly applicable to other epithelial and non-epithelial cancer subtypes, as well as "pre-malignant" lesions (carcinoma in situ).

**An Absence of Stromal Caveolin-1 Predicts DCIS Progression to Invasive Breast Cancer.**

The association of stromal caveolin-1 (Cav-1) levels with DCIS recurrence and/or progression to invasive breast cancer was determined (Witkiewicz, et al. (2009) Cancer Biol Ther 8, 1167-75.). An initial cohort of 78 DCIS patients with follow-up data was examined (see Figure 23). As ER-positivity was associated with recurrence, the analysis was focused on this subset of 56 patients. In this group; DCIS progressed to invasive breast cancer in -14% of the patient population (8/56), in accordance with an expected progression rate of 12-15%. Nearly ninety percent of DCIS patients (7/8) that underwent recurrence to invasive breast cancer had reduced or absent levels of stromal Cav-1 (4). Remarkably, an absence of stromal Cav-1 (score = 0) was specifically associated with early disease progression to invasive breast cancer, with a nearly 2-fold reduced time to recurrence (170.57 versus 89.3 months) and a higher progression rate (see Figure 24). All DCIS patients with an absence of stromal Cav-1 underwent some form of recurrence (5/5) and the majority (4/5) underwent progression to invasive breast cancer. This represents an overall cumulative incidence rate of 100% for recurrence and 80% for progression. An absence of stromal Cav-1 in DCIS lesions was also specifically associated with the presence of inflammatory cells. Conversely, ninety-seven percent of ER(+) DCIS patients (35/36) with high levels of stromal Cav-1 (score = 2) did not show any invasive recurrence over the duration of follow-up (4-208 months), and 89% of such patients are estimated to remain free of invasive recurrence, even after 15 years. Thus, determination of stromal Cav-1 levels is a useful new biomarker for guiding the treatment of ER(+) DCIS patients

**An Absence of Stromal Caveolin-1 is Associated with Advanced Prostate Cancer and Metastatic Disease Spread.**

The status of stromal Cav-1 expression in patients with benign prostatic hypertrophy (BPH), primary prostate cancers (PCa), and prostate-cancer metastases (Mets) was determined (Di Vizio, et al. (2009) Cell Cycle 8, 2420-4.). Interestingly, an absence of stromal Cav-1 directly correlated with prostate cancer disease progression (**Table 9**) For example, virtually all BPH samples showed abundant stromal Cav-1 immunostaining (see Figure 25). In contrast, in a subset of patients with primary prostate cancer, the stromal levels of Cav-1 were significantly decreased, and this correlated with a high Gleason score, indicative of a worse prognosis and poor clinical outcome (**Table 10**). Remarkably, all metastatic tumors (either from lymph node or bone) were completely negative for stromal Cav-1 staining. Thus, stromal Cav-1 expression is a new biomarker of prostate cancer disease progression and metastasis. As a loss of stromal Cav-1 has predictive value in both breast and prostate cancers, a loss of stromal Cav-1 is a "universal" or "widely-applicable" biomarker for many different types of human cancer

**Table 9.**

**A** Association of Stromal Cav-1 with Tumor Progression

| | Stromal Cav-1 Level | | | |
|---|---|---|---|---|
| | 0 | 1 | 2 | p-value |
| | N=53 | N=31 | N=13 | |
| Tumor Progression | | | | $3.11 \times 10^{-17}$ |
| Benign | 4% (2) | 52% (16) | 92% (12) | |
| PCa | 32% (17) | 48% (15) | 8% (1) | |
| Mets | 64% (34) | 0% (0) | 0% (0) | |

Test used Fisher's exact test

**B** Significant differences in Stromal Cav-1 between different patient groups

| | p-value |
|---|---|
| Benign *vs* PCa | $7.43 \times 10^{-6}$ |
| Benign *vs* Mets | $3.89 \times 10^{-16}$ |
| PCa *vs* Mets | $1.04 \times 10^{-6}$ |

Test used: Fisher's exact test

**Table 10.**

Association of Stromal Cav-1 with Gleason Score (GS)

| | Stromal Cav-1 Status | |
|---|---|---|
| GS | Absent | Present |
| 3+3 - 3+4 | 18% (3) | 60% (11) |
| 4+3 - 5+5 | 82% (14) | 31% (5) |

Test used: Fisher's exact test    p-value = $4.9 \times 10^{-3}$

**Proteomic Analysis of Caveolin-1 (-/-) Null Stromal Fibroblasts: Identification of Novel Biomarkers.**

Since a loss of Cav-1 protein expression in the breast stroma, in both DCIS and breast cancer patients, is predictive of poor clinical outcome other molecules that are up-regulated or down-regulated in the absence of stromal Cav-1 may be novel biomarkers.

To identify new biomarkers, Cav-1 (-/-) stromal cells were subjected to extensive unbiased proteomic analysis. Primary cultures of 1) mammary stromal fibroblasts (MSFs) and 2) bone-marrow derived stromal cells (BMSCs) from WT and Cav-1 (-/-) null mice were analysed. Virtually identical results were obtained with both cell types, consistent with the hypothesis that BMSCs can give rise to MSFs. Two-dimensional separation of WT and Cav-1 (-/-) stromal cell lysates yielded at least 60 protein spots which were differentially expressed. Interestingly, several of the protein spots that were up-regulated were identified as known markers of the myo-fibroblast and/or cancer-associated fibroblast (CAF) phenotype (vimentin, calponin2, tropomyosin, gelsolin, and prolyl 4-hydroxylase alpha) by mass spec analysis (Table 11).

**Table 11**

Proteomic Analysis of Cav-1 (-/-) Null Sromal Cells.

|  | Fold Change (KO/WT) | Accession Number | Protein Spot # |
|---|---|---|---|
| Myo-fibroblast Associated Proteins |  |  |  |
| gelsolin, isoform A | 2.21 | gi\|148676699 | 7 |
| tropomyosin 2 beta | 1.86 | gi\|123227997 | 40 |
| calponin 2 | 1.83 | gi\|6680952 | 44 |
| vimentin | 1.82 | gi\|31982755 | 30 |
| vimentin | 1.75 | gi\|2078001 | 22 |
| prolyt 4-hydroxylase alpha(I)-subunit | 1.7 | gi\|8336898 | 11 |
| Oncogenes |  |  |  |
| Elongation factor 1-delta; EF-1-delta | 1.94 | gi\|13124192 | 41 |
| Tumor Suppressors |  |  |  |
| nucleoside-diphosphate kinase 2; nm23, 2 | -10.3 | gi\|6679078 | 66 |
| Glycolytic and Metabolic Enzymes |  |  |  |
| M2-type pyruvate kinase | 2.78 | gi\|1405933 | 15 |
| phosphoglycerate kinase 1 | 2.41 | gi\|70778976 | 31 |
| lactate dehydrogenase A (Ldha) | 2.11 | gi\|13529599 | 43 |
| fructose-bisphosphate aldolase A | 1.87 | gi\|6671539 | 32 |
| glycerol 3-phosphate dehydrogenase 2, mitochondrial | 1.83 | gi\|224922803 | 12 |
| enolase 1 (Eno1) | 1.77 | gi\|34784434 | 24 |
| triosephosphate isomerase 1 | 1.7 | gi\|6678413 | 57 |
| triosephosphate isomerase 1 | 1.65 | gi\|6678413 | 58 |
| phosphoglycerate mutase 1 | 1.65 | gi\|10179944 | 64 |

Others proteins that were increased were identified as known oncogenes, such as Elongation factor 1-delta (EF-1-delta) (Table 3). Over-expression of EF-1-delta is sufficient to drive cell transformation and tumorigenesis (Lei, et al. (2002) Teratog Carcinog Mutagen 22, 377-83; Joseph, et al. (2002) J Biol Chem 277, 6131-6). Similarly, nm23-isoform2, a known tumor and metastasis suppressor protein (Salerno, et al. (2003) Clin Exp Metastasis 20, 3-10), is dramatically down-regulated > 10-fold in the absence of Cav-1 (Table 10).

Perhaps, most importantly, a loss of Cav-1 resulted in the up-regulation of 8 glycolytic enzymes, including the M2-isoform of pyruvate kinase (Table 11). The M2-isoform of pyruvate kinase is generated by gene splicing and is known to be sufficient to confer the "Warburg effect", i.e., aerobic glycolysis, which is thought to be a characteristic of tumor cells, stem cells, and cancer stem cells (Christofk, et al. (2008) Nature 452, 230-3; Christofk, et al. (2008) Nature 452,181-6). The M1 isoform is the "adult" isoform, while the M2 isoform is the corresponding "embryonic or developmental" isoform, both generated by alternate splicing from the same gene. The M2-isoform of pyruvate kinase can also act a nuclear co-factor to stimulate the transcriptional effects of Oct4, an iPS transcription factor that confers pluripotency in ES cells (Lee, et al. (2008) Int J Biochem Cell Biol 40,1043-54).

Figure 26 shows that all 8 of these enzymes sequentially map to the glycolytic pathway, which should result in the over-production of the metabolites pyruvate and lactate, which could then be secreted into the medium to "feed" adjacent tumor cells. Importantly, Figure 27 shows that the M2-isoform of pyruvate kinase (M2-PK) is highly over-expressed in the stroma of human breast cancers that lack stromal Cav-1 expression, as predicted. Thus, for the first time, the inventor has now identified that the Warburg effect can originate in the tumor stroma.

As such, small molecule inhibitors of the lactate transporter (responsible for pyruvate and lactate release) can now be used to target the Cav-1-negative tumor stroma. This provides a novel mechanism to explain why a loss of stromal Cav-1 in human breast cancers confers early tumor recurrence, metastasis, tamoxifen-resistance, and poor clinical outcome

**The Reverse Warburg Effect: Aerobic Glycolysis in Cancer Associated Fibroblasts and the Tumor Stroma**

Here, the invention provides a new model for understanding the Warburg effect in tumor metabolism (see Figure 28) which is that epithelial cancer cells induce the Warburg effect (aerobic glycolysis) in neighboring stromal fibroblasts-These cancer-associated fibroblasts, then undergo myo-fibroblastic differentiation, and secrete lactate and pyruvate (energy metabolites resulting from aerobic glycolysis). Epithelial cancer cells can then take up these energy-rich metabolites and use them in the mitochondrial TCA cycle, thereby promoting efficient energy production (ATP generation via oxidative phosphorylation), resulting in a higher proliferative capacity. In this alternative model of tumorigenesis, the epithelial cancer cells instruct the normal stroma to transform into a wound-healing stroma, providing the necessary energy-rich micro-environment for facilitating tumor growth and angiogenesis. In essence, the fibroblastic tumor stroma directly feeds the epithelial cancer cells, in a type of host-parasite relationship. This mechanism is termed the "Reverse Warburg Effect." In this scenario, the epithelial tumor cells "corrupt" the normal stromal, turning it into a factory for the production of energy-rich metabolites. This alternative model is still consistent with Warburg's original observation that tumors show a metabolic shift towards aerobic glycolysis. In support of this idea, unbiased proteomic analysis and transcriptional profiling of a new model of cancer-associated fibroblasts (caveolin-1 (Cav-1) deficient stromal cells), shows the upregulation of both (1) myo-fibroblast markers and (2) glycolytic enzymes, under normoxic conditions. The expression of these proteins in the fibroblastic stroma of human breast cancer tissues that lack stromal Cav-1 was validated. Importantly, a loss of stromal Cav-1 in human breast cancers is associated with tumor recurrence, metastasis, and poor clinical outcome. Thus, an absence of stromal Cav-1 is a biomarker for the "Reverse Warburg Effect," explaining its powerful predictive value.

**Caveolin-1 (-/-) Null Stromal Fibroblasts are Sufficient to Promote Breast Tumor Growth and Tumor Angiogenesis In Vivo.**

To provide additional experimental validation for the idea that an absence of stromal Cav-1 promotes breast cancer tumor growth and angiogenesis, a 2-component cell-system using xenografts in immuno-deficient female nude mice was developed. Briefly, the flanks of nude mice were injected with a mixture of 1) MDA-MB-231 cells (a highly aggressive human breast cancer cell line; 1.0 X 10-6 cells) and 2) stromal fibroblasts, derived from either WT or Cav-1 (-/-) null mice (0.3 X 10-6 cells). After 3 weeks, nude mice were sacrificed and breast cancer derived tumors were subjected to analysis for growth (tumor weight and volume measurements) and immuno-histochemistry with markers of angiogenesis, such as CD31 (a.k.a, PECAM1). In accordance with the disclosed mechanism, tumors grown using Cav-1 (-/-) stromal fibroblasts (KO) were ~2.5 fold larger, and showed extensive angiogenesis, as visualized by CD31 staining (Figure 29). Importantly, quantitation revealed an ~3.1-fold increase in tumor vessel area, in tumors grown with Cav-1 (-/-) stromal fibroblasts (see Figure 30).

**Pre-Screening Assays and the Status of Vimentin in Human Breast Cancers Lacking Stromal Cav-1.**

The invention provides new candidate biomarkers whose levels are changed in response to a loss of Cav-1 in stromal cells. Fourteen of these molecules are up-regulated (5 myofibroblast markers (including vimentin), 1 oncogene (EF-1-delta), and 8 glycolytic enzymes (including M2-pyruvate kinase)), and one is down-regulated (1 tumor suppressor (nm23-isoform2)). Two pre-screening assays were implemented. One consists of a co-culture system employing MCF-7 cells and human stromal fibroblasts. Using cell-type specific markers (pan-cyto-keratin for MCF-7 and Cav-1 for fibroblasts), fibroblasts alone express large amounts of Cav-1. In contrast, when both cell types are co-cultured, there is a specific loss of Cav-1 expression in human fibroblasts co-cultured with MCF-7 breast cancer cells (**Figure 38**, Upper panels). In a second pre-screening assay, a number of human breast cancer that show a loss of stromal Cav-1 were selected for immuno-staining with these candidate markers. In support of these two pre-screening approaches, vimentin was evaluated as a candidate marker. As shown in Figure 32 (Lower panels), vimentin is up-regulated in fibroblasts co-cultured with MCF-7 cells. Also, vimentin is highly expressed in human breast cancers that lack stromal Cav-1 expression (see Figure 31). Thus, these results directly demonstrate the feasibility of the two pre-screening/validation approaches. Importantly, it has already been shown that stromal expression of vimentin predicts poor clinical outcome in human colon cancer patients.

**The Reverse Warburg Effect: Glycolysis Inlribitors Prevent the Tumor Promoting Effects of Coveolin-1 Deficient Cancer Associated Fibroblasts**

The invention provides that a loss of stromal caveolin-1 (Cav-1) in cancer-associated fibroblasts (CAFs) is a powerful single independent predictor of breast cancer patient tumor recurrence, metastasis, tamoxifen-resistance, and poor clinical outcome. Loss of stromal Cav-1 mediates these effects clinically. To mechanistically address this issue, the invnetor generated a novel human tumor xenograft model. In this two-component system, nude mice are co-injected with i) human breast cancer cells (MDA-MB-231), and ii) stromal fibroblasts (WT versus Cav-1 (-/-) deficient). This allows a direct evaluation of the effects of a Cav-1 deficiency solely in the tumor stromal compartment. Here, Cav-1-deficient stromal fibroblasts are sufficient to promote both tumor growth and angiogenesis, and recruit Cav-1 (+) micro-vascular cells. Proteomic analysis of Cav-1-deficient stromal fibroblasts indicates that these cells upregulate the expression of glycolytic enzymes, a hallmark of aerobic glycolysis (Warburg effect). Thus, they contribute towards tumor growth and angiogenesis, by providing energy-rich metabolites in a paracrine fashion. This mechanism is termed the "Reverse Warburg Effect". In direct support of this notion, treatment of this xenograft model with glycolysis inhibitors functionally blocks the positive effects of Cav-1-deficient stromal fibroblasts on breast cancer tumor growth (see Figure 33). Thus, metabolic restriction (via treatment with glycolysis inhibitors) is a promising new therapeutic strategy for breast cancer patients that lack stromal Cav-1 expression. The stromal expression of PKM2 and LDH-B are new biomarkers for the "Reverse Warburg Effect" in human breast cancers (see Figure 34).

**A Loss of Stromal Caveolin-1 Leads to Oxidative Stress, Mimics Hypoxis, and Drives Inflammation in the Tumor MicroEnvironment, Conferring the "Reverse Warburg Effect"**

Cav-1 deficient stromal cells are a new genetically-based model for myofibroblasts and cancer-associated fibroblasts. Using an unbiased informatics analysis of the transcriptional profile of Cav-1 (-/-) deficient mesenchymal stromal cells, the inventor has now identified many of the major signaling pathways that are activated by a loss of Cav-1, under conditions of metabolic restriction (with low glucose media). The results show that a loss of Cav-1 induces oxidative stress, which mimics a constitutive pseudo-hypoxic state, leading to 1) aerobic glycolysis and 2) inflammation in the tumor stromal microenvironment (see Figure 35). This occurs via the activation of 2 major transcription factors, namely HIF (aerobic glycolysis) and NF-kappa-B (inflammation). Experimentally, it is shown that Cav-1 deficient cells may possess defective mitochondria, due to the over-production of nitric oxide (NO), resulting in the nitration of the mitochondrial respiratory chain components (such as complex I). Elevated levels of nitro-tyrosine were observed both in Cav-1 (-/-) stromal cells, and via acute knockdown with siRNA targeting Cav-1 (see Figure 36). Finally, metabolic restriction with mitochondrial (complex I) and glycolysis inhibitors was synthetically lethal with a Cav-1 (-/-) deficiency in mice (see Figure 37). As such, Cav-1 deficient mice show a dramatically reduced mitochondrial reserve capacity. Thus, a mitochondrial defect in Cav-1 deficient stromal cells could drive oxidative stress, leading to aerobic glycolysis, and inflammation, in the tumor microenvironment. These stromal alterations may underly the molecular basis of the "Reverse Warburg Effect", and could provide the key to targeted anti-cancer therapies using metabolic inhibitors.

**Mammary Cancer Cells Induce the Warburg Effect in Adjacent Stromal Fibroblasts Via Oxidative Stress, Leading to NFkB- and HIF- Transcriptional Activation.**

In order to understand how caveolin-1 (Cav-1) is down-regulated in tumor stromal fiboblasts, the inventor devised a co-culture system employing i) human breast cancer cells (MCF-7) and ii) normal human fibroblasts. Using this system, it was observed that within 5 days of co-culture MCF-7 cells down-regulate the expression of Cav-1 in adjacent stromal fibroblasts. This occurs via the degradation of Cav-1 in lysosomal structures, and can be inhibited by chloroquine, an anti-lysomal agent. Most importantly, MCF-7 cells induce oxidative stress in the adjacent fibroblasts via the over-production of reactive oxygen species (ROS). ROS, in turn, is known to stabilize and activate certain transcription factors, such as NFkB and HIF. NFkB- and HIF- are the master regulators of "inflammation" and "hypoxia/aerobic glycolysis" in the tumor microenvironment. Thus, in order to detect the activation of NFkB and HIF only in the fibroblast cell population, two different fibroblast cell lines engineered to express either a NFkB-luciferase or HIF-luciferase transcriptional reporter were used. The results directly show that MCF-7 cells transcriptionally activate both NFkB and HIF in the adjacent fibroblast population, with different kinetics (see Figure 38). As such, NFkB is activated first, while HIF is activated on day 4 and day 5 of coculture, coicident with Cav-1 down-regulation. Thus, cancer cells induce the Warburg effect in adjacent stromal fibroblasts via the activation of NFkB and HIF-target genes, leading to inflammation and aerobic glycolysis. These findings provide a mechanistic basis for the "Reverse Warburg Effect", and a new high-throughput drug-screening assay for the identification of novel compounds that block the "Reverse Warburg Effect".

**Modeling the Tumor-Stromal Micro-Environment Using ES Cell Cultures and Cav-1 (-/-) Deficient Stromal Fibroblast Feeder Layers.**

The cancer stem cell theory states that "stem-like" tumor initiating cells are required to generate the bulk of the epithelial cancer cells of a given tumor type, including human breast cancers. Here, mouse embryonic stem cells (ES) were used as a model for cancer stem cells, to understand how the tumor stromal microenvironment may positively influence the growth of cancer stem cells. For this purpose, ES cells were grown on WT fibroblasts feeder layers or Cav-1 (-/-) deficient fibroblast feeder layers. Importantly, Cav-1 (-/-) deficient fibroblasts are a new genetically well-defined model for human cancer-associated fibroblasts. Interestingly, we show that Cav-1 (-/-) deficient fibroblast feeder layers dramatically stimulate the growth of two independent mouse ES cell lines in culture. Results are shown in Figure 39. As these same fibroblasts also stimulate mammary tumor growth and tumor angiogenesis in vivo, this may be mechanistically via the expansion of the cancer stem population within the tumor. This may explain why human breast cancer patients with a loss of stromal Cav-1 show a very significant increase in tumor recurrence, metastasis, and tamoxifen-resistance, as well as overall poor clinical outcome. Finally, the effect of Cav-1 (-/-) deficient fibroblasts can be mimicked by using another fibroblast cell line specifically engineered to lack functional mitochondria. As such, these fibroblasts are completely glycolytic and cannot undergo oxidative metabolism. These results directly support the "Reverse Warburg Hypothesis", in which stromal cells feed tumor cells in a paracrine fashion using energy-rich metabolites derived from aerobic glycolysis.

**Loss of Stromal Caveolin-1 Expression Predicts Poor Clinical Outcome in Triple Negative and Basal-Like Breast Cancer.**

The predictive value of stromal caveolin-1 (Cav-1) as a biomarker for clinical outcome in triple negative (TN) breast cancer patients was determined. A cohort of 88 TN breast cancer patients was available, with the necessary annotation and nearly 12 years of follow-up data. The primary outcome of interest in this study was overall survival. Interestingly, TN patients with high-levels of stromal Cav-1, had a good clinical outcome, with >50% of the patients remaining alive during the follow-up period (Figure 40). In contrast, the median survival for TN patients with moderate stromal Cav-1 staining was 33.5 months. Similarly, the median survival for TN patients with absent stromal Cav-1 staining was 25.7 months. A comparison of 5-year survival rates yields a similar pattern. TN patients with high stromal Cav-1 had a good 5-year survival rate, with 75.5% of the patients remaining alive. In contrast, TN patients with moderate or absent stromal Cav-1 levels had progressively worse 5-year survival rates, with 40% and 9.4% of the patients remaining alive, In contrast, in a parallel analysis, the levels of tumor epithelial Cav-1 had no prognostic significance. As such, the prognostic value of Cav-1 immunostaining in TN breast cancer patients is compartment-specific, and selective for an absence of Cav-1 staining in the stromal fibroblast compartment. A recursive-partitioning algorithm was used to assess which factors are most predictive of overall survival in TN breast cancer patients. In this analysis, we included tumor size, histologic grade, whether the patient received surgery, radiotherapy or chemotherapy, CK5/6, EGFR, P53 and Ki67 status, as well as the stromal Cav-1 score. This analysis indicated that stromal Cav-1 expression was the most important prognostic factor for overall survival in TN breast cancer. Virtually identical results were obtained with CK5/6 (+) and/or EGF-R (+) TN breast cancer cases, indicating that a loss of stromal Cav-1 is also a strong prognostic factor for basal-like breast cancers (Figure 41). These findings have important implications for the close monitoring and treatment stratification of TN breast cancer patients.

**Rapamycin Can Therapeutically Target the "Reverse Warburg Effect" in the Caveolin-1 Deficient Breast Cancer Tumor Microenvironment,**

The invention provides a new system to investigate the use of therapies targeted agaist the Cav-1 deficient tumor microenvironment. Met-1 cells, an aggressive mouse mammary tumor cell line, were orthotopically implanted into the mammary glands of normal WT FVB mice or Cav-1 (-/-) deficient FVB mice, and followed over time.
At 5 weeks post tumor cell injection, mammary glands were harvested and subjected to a detailed analysis. The results indicate that tumors grown in the Cav-1 (-/-) mammary fat pat microenvironment were greater than 15 times larger, as measured by tumor mass (Figure 25). Tumors grown in the Cav-1 (-/-) mammary fat pat microenvironment showed a striking increase in vascularization due to extensive tumor angiogenesis.
Importantly, if mice were treated with a standard therapeutic dose of rapamycin, this effect was nearly completely abolished (Figure 42). Thus, tumor growth was drastically reduced. As such, rapamycin or its derivatives may be used to therapeutically target the Cav-1 deficient breast cancer tumor microenvironment.

**Therapeutic Compounds**

The markers and marker sets of the present invention assess the likelihood of short or long term survival in cancer patients, e.g., patients having breast cancer. Using this prediction, cancer therapies can be evaluated to design a therapy regimen best suited for patients.

Known angiogenesis inhibitors that may used in methods of the invention include, but are not limited to, both direct and indirect angiogenesis inhibitors such as Angiostatin, bevacizumab (Avastin), Arresten, Canstatin, Combretastatin, Endostatin, N-3, Thrombospondin, Tumstatin, 2-methoxyestradiol, and Vitaxin, ZD1839 (Iressa; getfitinib), ZD6474, OSI774 (tarceva), CI1033, PKI1666, IMC225 (Erbitux), PTK787, SU6668, SU11248, Herceptin, Marimastat, COL-3, Neovastat, 2-ME, SU6668, anti-VEGF antibody, Medi-522 (Vitaxin II), tumstatin, arrestin, recombinant EPO, troponin I, EMD121974, and IFN-$\alpha$, CELEBREX® (celecoxib), and THALOMID® (thalidomide), have also been recognized as angiogenesis inhibitors (Kerbel et al., Nature Reviews, Vol. 2, October 2002, pp. 727). A further example of an anti-angiogenic compound includes, but is not limited to PD 0332991 (see Fry, D.W. et al. Specific inhibition of cyclin-dependent kinase 4/6 by PD 0332991 and associated antitumor activity in human tumor xenografts. Mol Cancer Ther. 2004;3:1427-1438). Suitable antiangiogenic compositions include, but are not limited to Galardin (GM6001, Glycomed, Inc., Alameda, Calif.), endothelial response inhibitors (e.g., agents such was interferon alpha, TNP-470, and vascular endothelial growth factor inhibitors), agents that prompt the breakdown of the cellular matrix (e.g., Vitaxin (human LM-609 antibody. Ixsys Co., San Diego, Calif.; Metastat, CollaGenex, Newtown, Pa.; and Marimastat BB2516, British Biotech), and agents that act directly on vessel growth (e.g., CM-101, which is derived from exotoxin of Group A Streptococcus antigen and binds to new blood vessels inducing an intense host inflammatory response; and Thalidomide). Preferred anti-angiogenic inhibitors include, for example, bevacizumab, getfitinib thalidomide, tarceva, celecoxib, erbitux, arrestin, recombinant EPO, troponin I, herceptin. Dosages and routes of administration for these Food and Drug Administration (FDA) approved therapeutic-compound are known to those of ordinary skill in the art as a matter of the public record.

Several kinds of steroids have also been noted to exert antiangiogenic activity. In particular, several reports have indicated that medroxyprogesterone acetate (MPA), a synthetic progesterone, potently inhibited neovascularization in the rabbit corneal assay (Oikawa (1988) Cancer Lett. 43: 85). A pro-drug of 5FU, 5'-deoxy-5-fluorouridine (5'DFUR), might be also characterized as an antiangiogenic compound, because 5'DFUR is converted to 5-FU by the thymidine phosphorylase activity of PD-ECGF/TP. 5'DFUR might be selectively active for PD-ECGF/TP positive tumor cells with high angiogenesis potential. Recent clinical investigations in showed that 5'DFUR is likely to be effective for PD-ECGF/TP-positive tumors. It was showed that a dramatic enhancement of antitumor effect of 5'DFUR appeared in PD-ECGF/TP transfected cells compared with untransfected wild-type cells (Haraguchi (1993) Cancer Res. 53: 5680 5682). In addition, combined 5'DFUR+MPA compounds are also effective antiangiogenics (Yayoi (1994) Int J Oncol. 5: 27 32). The combination of the 5'DFUR+MPA might be categorized as a combination of two angiogenesis inhibitors with different spectrums, an endothelial growth factor inhibitor and a protease inhibitor. Furthermore, in in-vivo experiments using DMBA-induced rat mammary carcinomas, 5'DFUR exhibited a combination effect with AGM-1470 (Yamamoto (1995) Oncol Reports 2:793 796).

Another group of antiangiogenic compounds for use in this invention include polysaccharides capable of interfering with the function of heparin-binding growth factors that promote angiogenesis (e.g., pentosan polysulfate).

Other modulators of angiogenesis include platelet factor IV, and AGM 1470. Still others are derived from natural sources collagenase inhibitor, vitamin D3-analogues, fumigallin, herbimycin A, and isoflavones.

Therapeutic agents for use in the methods of the invention include, for example, a class of therapeutic agents known as proteosome inhibitors. As used herein, the term "proteasome inhibitor" refers to any substance which directly inhibits enzymatic activity of the 20S or 26S proteasome in vitro or in vivo. In some embodiments, the proteasome inhibitor is a peptidyl boronic acid. Examples of peptidyl boronic acid proteasome inhibitors suitable for use in the methods of the invention are disclosed in Adams et al., U.S. Pat. Nos. 5,780,454 (1998), 6,066,730 (2000), 6,083,903 (2000); 6,297,217 (2001), 6,465,433 (2002), 6,548,668 (2003), 6,617,317 (2003), and 6,747,150 (2004), each of which is hereby incorporated by reference in its entirety, including all compounds and formulae disclosed therein. Preferably, the peptidyl boronic acid proteasome inhibitor is selected from the group consisting of: N(4 morpholine)carbonyl-.beta.-(1-naphthyl)-L-alanine-L-leucine boronic acid; N(8 quinoline)sulfonyl-.beta.-(1-naphthyl)-L-alanine-L-alanine-L-leucine boronic acid; N(pyrazine)carbonyl-L-phenylalanine-L-leucine boronic acid, and N(4 morpholine)-carbonyl-[O-(2-pyridylmethylyl-L-tyrosine-L-leucine boronic acid. In a particular embodiment, the proteasome inhibitor is N (pyrazine)carbonyl-L-phenylalanine-L-leucine boronic acid (bortezomib; VELCADE®; formerly known as MLN341 or PS-341).

Additional peptidyl boronic acid proteasome inhibitors are disclosed in Siman et al., international patent publication WO 99/30707; Bemareggi et al., international patent publication WO 05/021558; Chatterjee et al., international patent publication WO 05/016859; Furet et al., U.S. patent publication 2004/0167337; Furet et al., international patent publication 02/096933; Attwood et al., U.S. Pat. No. 6,018,020 (2000); Magde et al., international patent publication WO 04/022070; and Purandare and Laing, international patent publication WO 04/064755.

Additionally, proteasome inhibitors include peptide aldehyde proteasome inhibitors, such as those disclosed in Stein et al., U.S. Pat. No. 5,693,617 (1997); Siman et al., international patent publication WO 91/13904; Iqbal et al., J. Med. Chem. 38:2276-2277 (1995); and Iinuma et al., international patent publication WO 05/105826, each of which is hereby incorporated by reference in its entirety.

Additionally, proteasome inhibitors include peptidyl epoxy ketone proteasome inhibitors, examples of which are disclosed in Crews et al., U.S. Pat. No. 6,831,099; Smyth et al., international patent publication WO 05/111008; Bennett et al., international patent publication WO 06/045066; Spaltenstein et al. Tetrahedron Lett. 37:1343 (1996); Meng, Proc. Natl. Acad. Sci. 96: 10403 (1999); and Meng, Cancer Res. 59: 2798 (1999), each of which is hereby incorporated by reference in its entirety.

Additionally, proteasome inhibitors include alpha-ketoamideproteasome inhibitors, examples of which are disclosed in Chatterjee and Mallamo, U.S. Pat. Nos. 6,310,057 (2001) and 6,096,778 (2000); and Wang et al., U.S. Pat. Nos. 6,075,150 (2000) and 6,781,000 (2004), each of which is hereby incorporated by reference in its entirety.

Additional proteasome inhibitors include peptidyl vinyl ester proteasome inhibitors, such as those disclosed in Marastoni et al., J. Med. Chem. 48:5038 (2005), and peptidyl vinyl sulfone and 2-keto-1,3,4-oxadiazole proteasome inhibitors, such as those disclosed in Rydzewski et al., J. Med. Chem. 49:2953 (2006); and Bogyo et al., Proc. Natl. Acad. Sci. 94:6629 (1997), each of which is hereby incorporated by reference in its entirety.

Additional proteasome inhibitors include azapeptoids and hydrazinopeptoids, such as those disclosed in Bouget et al., Bioorg, Med. Chem. 11:4881 (2003); Baudy-Floc'h et al., international patent publication WO 05/030707; and Bonnemains et al., international patent publication WO 03/018557, each of which is hereby incorporated by reference in its entirety.

Furthermore, proteasome inhibitors include peptide derivatives, such as those disclosed in Furet et al., U.S. patent publication 2003/0166572, and efrapeptin oligopeptides, such as those disclosed in Papathanassiu, international patent publication WO 05/115431, each of which is hereby incorporated by reference in its entirety.

Further, proteasome inhibitors include lactacystin and salinosporamide and analogs thereof, which have been disclosed in Tenteany et al., U.S. Pat. Nos. 5,756,764 (1998), 6,147,223 (2000), 6,335,358 (2002), and 6,645,999 (2003); Fenteany et al., Proc. Natl. Acad. Sci. USA (1994) 91:3358; Fenical et al., international patent publication WO 05/003137; Palladino et al., international patent publication WO 05/002572; Stadler et al., international patent publication WO 04/071382; Xiao and Patel, U.S. patent publication 2005/023162; and Corey, international patent publication WO 05/099687, each of which is hereby incorporated by reference in its entirety.

Further, proteasome inhibitors include naturally occurring compounds shown to have proteasome inhibition activity can be used in the present methods. For example, TMC-95A, a cyclic peptide, and gliotoxin, a fungal metabolite, have been identified as proteasome inhibitors. See, e.g., Koguchi, Antibiot. (Tokyo) 53:105 (2000); Kroll M, Chem. Biol. 6:689 (1999); and Nam S, J. Biol. Chem. 276: 13322 (2001), each of which is hereby incorporated by reference in its entirety. Additional proteasome inhibitors include polyphenol proteasome inhibitors, such as those disclosed in Nam et al., J. Biol. Chem. 276:13322 (2001); and Dou et al., U.S. patent publication 2004/0186167, each of which is hereby incorporated by reference in its entirety.

Preferred proteasome inhibitors include, for example, bortezomib. Dosages and routes of administration for Food and Drug Administration (FDA) approved therapeutic compounds are known to those of ordinary skill in the art as a matter of the public record.

Preferred angiogenesis inhibitors and other anti-cancer compounds, for use in the methods of the invention include, for example, 17-AAG, Apatinib, Ascomycin, Axitinib, Bexarotene, Bortezomib. Bosutinib, Bryostatin 1, Bryostatin 2, Canertinib, Carboplatin, Cediranib, Cisplatin, Cyclopamine, Dasatinib, 17-DMAG, Docetaxel, Doramapimod, Dovitinib, Erlotinib, Everolimus, Gefitinib, Geldanamycin, Gemcitabine, Imatinib, Imiquimod, Ingenol 3-Angelate, Ingenol 3-Angelate 20-Acetate, Irinotecan, Lapatinib, Lestaurtinib, Nedaplatin, Masitinib, Mubritinib, Nilotinib, NVP-BEZ235, OSU-03012, Oxaliplatin, Paclitaxel, Pazopanib, Picoplatin, Pimecrolimus, PKC412, Rapamycin, Satraplatin, Sorafenib, Sunitinib, Tandutinib, Tivozanib, Thalidomide, Temsirolimus, Tozasertib, Vandetanib, Vargatef, Vatalanib, Zotarolimus, ZSTK474, Bevacizumab (Avasti), Cetuximab, Herceptin, Rituximab, Trastuzumab.

Preferred protein kinase inhibitors for use in the methods of the invention include, for example, Apatinib, Axitinib, Bisindolylmaleimide I, Bisindolylmaleimide I, Bosutinib, Canertinib, Cediranib, Chelerythrine, CP690550, Dasatinib, Dovitinib, Erlotinib, Fasudil, Gefitinib, Genistein, Gö 6976, H-89, HA-1077, Imatinib, K252a, K252c, Lapatinib, Di-p-Toluenesulfonate, Lestaurtinib, LY 294002, Masitinib, Mubritinib, Nilotinib. OSU-03012. Pazopanib, PD 98059, PKC412, Roscovitine, SB 202190, SB 203580, Sorafenib, SP600125, Staurosporine, Sunitinib, Tandutinib, Tivozanib, Tozasertib, Tyrphostin AG 490, Tyrphostin AG 1478, U0126, Vandetanib, Vargatef, Vatalanib, Wortmannin, ZSTK474. Preferred Hedgehog and Smoothened (Smo) Inhibitors for use in the methods of the invention include, for example, Cyclopamine.

Platinum-based Anti-Cancer Compounds for use in the methods of the invention include, for example, Carboplatin, Cisplatin, Eptaplatin, Nedaplatin, Oxaliplatin, Picoplatin, Satraplatin. Proteasome Inhibitors for use in the methods of the invention include, for example, Bortezomib (Velcade). Anti-Diabetes Drugs for use in the methods of the invention include, for example, Metformin.

Fibrosis Inhibitors for use in the methods of the invention include, for example, Halofuginone. Metformin, N-acetyl-cysteine (NAC). NfkB Inhibitors for use in the methods of the invention include, for example. RTA 402 (Bardoxolone methyl), Auranofin, BMS-345541. IMD-0354, PS-1145, TPCA-1, Wedclolactone. HIF Inhibitors for use in the methods of the invention include, for example, Echinomycin. Glycolysis Inhibitors for use in the methods of the invention include, for example, 2-deoxy-D-glucose (2-DG), 2-bromo-D-glucose, 2-fluoro-D-glucose, and 2-iodo-D-glucose, dichloro-acetate (DCA), 3-chloro-pyruvate, 3-Bromo-pyruvate (3-BrPA), 3-Bromo-2-oxopropionate, Oxamate.

PI-3 Kinase, Akt, and mTOR inhibitors for use in the methods of the invention include, for example, LY 294002, NVP-BEZ235, Rapamycin, Wortmannin. Isoflavones for use in the methods of the invention include, for example, Quercetin, and Resveratrol. Anti-Oxidants for use in the methods of the invention include, for example, N-acetyl-cysteine (NAC), N-acetyl-cysteine amide (NACA).

Immunosuppressants for use in the methods of the invention include, for example, Ascomycin, CP690550, Cyclosporin A, Everolimus, Fingolimod, FK-506, Mycophenolic Acid, Pimecrolimus, Rapamycin, Temsirolimus, Zotarolimus. Cyclin dependent kinase inhibitors (CDK) inhibitors for use in the methods of the invention include, for example, Roscovitine, and PD 0332991 (CDK4/6 inhibitor). Lysosomal acidification inhibitors for use in the methods of the invention include, for example, Chloroquine. PARP Inhibitors for use in the methods of the invention include, for example, BSI-201, Olaparib, DR 2313, NU 1025.

Compounds described herein can be administered to a human patient per se, or in pharmaceutical compositions mixed with suitable carriers or excipient(s). Techniques for formulation and administration of the compounds of the instant application may be found in "Remington's Pharmaceutical Science," Mack Publishing Co., Easton, Pa., latest edition. Suitable routes of administration may, for example, include oral, rectal, transmucosal, or intestinal administration; parenteral delivery, including intramuscular, subcutaneous, intramedullary injections, as well as intrathecal, direct intraventricular, intravenous, intraperitoneal, intranasal, or intraocular injections. Pharmaceutical compositions suitable for use in the present invention include compositions wherein the active ingredients are contained in an amount effective to achieve its intended purpose. More specifically, a therapeutically effective amount means an amount of compound effective to prevent, alleviate or ameliorate symptoms of disease or prolong the survival of the subject being treated. Determination of a therapeutically effective amount is well within the capability of those skilled in the art, especially in light of the detailed disclosure provided herein.

**Antibodies**

The invention provides antibodies to caveolin-1 and/or caveolin-2 proteins, or fragments of caveolin-1 and/or caveolin-2 proteins. The term "antibody" as used herein refers top immunoglobulin molecules and immunologically active portions of immunoglobulin (Ig) molecules, i.e., molecules that contain an antigen binding site that specifically binds (immunoreacts with) an antigen. Such antibodies include, but are not limited to, polyclonal, monoclonal, chimeric, single chain, Fab, Fab and F(ab)$_2$ fragments, and an Fab expression library, in general, an antibody molecule obtained from humans relates to any of the classes IgG, IgM, IgA, JgE and IgD, which differ from one another by the nature of the heavy chain present in the molecule. Certain classes have subclasses as well, such as IgG$_1$, IgG$_2$, and others. Furthermore, in humans, the light chain may be a kappa chain or a lambda chain. Reference herein to antibodies includes a reference to all such classes, subclasses and types of human antibody species.

**Predictive Medicine**

The invention also pertains to the field of predictive medicine in which diagnostic assays, prognostic assays, pharmacogenomics, and monitoring clinical trials are used for prognostic (predictive) purposes to thereby treat an individual prophylactically. Accordingly, one aspect of the invention relates to diagnostic assays for determining stromal caveolin-1 protein expression as well as stromal caveolin-1 and/or caveolin-2 activity, in the context of a biological sample (e.g., blood, serum, cells, tissue) to thereby determine whether an individual is afflicted with a disease or disorder, or is at risk of developing a disorder, associated with aberrant caveolin-1 expression or activity. The disorders include cell proliferative disorders such as cancer. The invention also provides for prognostic (or predictive) assays for determining whether an individual is at risk of developing, a disorder associated with caveolin-1 protein expression or activity. Such assays may be used for prognostic or predictive purpose to thereby prophylactically treat an individual prior to the onset of a disorder characterized by or associated with caveolin-1 protein, nucleic acid expression, or biological activity.

Another aspect of the invention provides methods for determining caveolin-1 protein expression or activity in an individual to thereby select appropriate therapeutic or prophylactic agents for that individual (referred to herein as "pharmacogenomics"). Pharmacogenomics allows for the selection of agents (e.g., drugs) for therapeutic or prophylactic treatment of an individual based on the genotype of the individual (e.g., the genotype of the individual examined to determine the ability of the individual to respond to a particular agent.)

Yet another aspect of the invention pertains to monitoring the influence of agents (e.g. drugs, compounds) on the expression or activity of stromal caveolin-1 in clinical trials.

**Diagnostic Assays**

An exemplary method for detecting the presence or absence of caveolin-1 in a biological sample involves obtaining a biological sample from a test subject and contacting the biological sample with a compound or an agent capable of detecting caveolin-1 protein such that the presence of caveolin-1 is detected in the biological sample, wherein the biological sample includes, for example, stromal cells.

An agent for detecting caveolin-1 and/or caveolin-2 protein is an antibody capable of binding to caveolin-1 protein, preferably an antibody with a detectable label. Antibodies can be polyclonal, or more preferably, monoclonal. An intact antibody, or a fragment thereof (e.g., Fab or $F(ab')_2$) can be used. The term "labeled", faith regard to the probe or antibody, is intended to encompass direct labeling of the probe or antibody by coupling (i.e., physically linking) a detectable substance to the probe or antibody, as well as indirect labeling of the probe or antibody by reactivity with another reagent that is directly labeled. Examples of indirect labeling include detection of a primary antibody using a fluorescently-labeled secondary antibody and end-labeling of a DNA probe with biotin such that it can be detected with fluorescently-labeled streptavidin. The term "biological sample" is intended to include tissues, cells and biological fluids isolated from a subject, as well as tissues, cells and fluids present within a subject That is, the detection method of the invention can be used to detect caveolin-1 protein in a biological sample in vitro as well as in vivo. For example, in vitro techniques for detection of caveolin-1 protein include enzyme linked immunosorbent as (ELISA), Western blot, immunoprecipitation, and immunofluorescence. Furthermore, in vitro techniques for detection of caveolin-1 protein include introducing into a subject a labeled anti-caveolin-1 antibody. For example the antibody can be labeled with a radioactive marker whose presence and location in a subject can be detected by standard imaging techniques.

In another embodiment, the methods further involve obtaining a control biological sample from a control subject, contacting the control sample with a compound or agent capable of detecting caveolin-1 protein, such that the presence of caveolin-1 and/or caveolin-2 protein, is detected in the biological sample, and comparing the presence of caveolin-1 protein, or lack thereof in cells, for example stromal cells, compared to the control sample with the presence of caveolin-1 protein, in the test sample.

The invention also encompasses kits for detecting the presence of caveolin-1 and/or caveolin-2 in a biological sample. For example, the kit can comprise: a labeled compound or agent capable of detecting caveolin-1 protein in a biological sample, for example stromal.cells; means for determining the amount of caveolin-1 in the sample; and means for comparing the amount or caveolin-1 in the sample with a standard. The compound or agent can be packaged in a suitable container. The kit can further comprise instructions for using the kit to detect caveolin-1 protein in, for example, stromal cells.

**Prognostic Assays**

The diagnostic methods described herein can furthermore be utilized to identify subjects having or at risk of developing a disease or disorder associated with aberrant stromal caveolin-1 expression or activity. For example, the assays described herein. Such as the preceding diagnostic assays or the following assays, can be utilized to identify a subject having or at risk of developing a disorder associated with caveolin-1 protein, nucleic acid expression or activity. Alternatively, the prognostic assays can be utilized to identify a subject having or at risk for developing, a disease or disorder. Thus the invention provides method for identifying a disease or disorder associated with aberrant caveolin-1 and/or caveolin-2.expression or activity in which a test sample is obtained from a subject and caveolin-1 and/or caveolin-2 protein is detected, wherein the presence or absence of caveolin-1 protein in stromal cells is diagnostic for a subject having or at risk of developing a disease or disorder associated with aberrant caveolin-1 expression or activity. As used herein, a "test sample" refers to a biological sample obtained from a subject of interest. For example, a test sample can be a biological fluid (e.g. serum), cell sample, and/or tissue, including but not limited to stromal cells.

Furthermore, the prognostic assays described herein can be used to determine whether a subject can be administered an agent (e.g., an agonist, antagonist, peptidomimetic, protein, peptide, nucleic acid, small molecule, or other drug candidate) to treat a disease or disorder associated with aberrant caveolin-1 expression or activity. For example, such methods can be used to determine whether as subject can be effectively treated with an agent for a disorder. Thus, the invention provides methods for determining whether a subject can be effectively treated with an agent for a disorder associated with aberrant caveolin-1 and/or caveolin-2 expression or activity in which a test sample is obtained and caveolin-1 protein expression or activity is detected (e.g., herein the presence of caveolin-1 and/or caveolin-2 protein is diagnostic for a subject that can be administered the agent to treat a disorder associated with aberrant caveolin-1 expression or activity).

The methods described herein may be performed, for example, by utilizing prepackaged diagnostic kits comprising at least one antibody reagent described herein, which may be conveniently used, e.g., in clinical settings to diagnose patients exhibiting symptoms or family history of a disease or illness involving a caveolin-1 and/or caveolin-2 gene.

Furthermore, any cell type or tissue, preferably peripheral blood leukocytes, in which caveolin-1 is expressed may be utilized in the prognostic assays described herein. However, any biological sample containing nucleated cells may be used, including, for example, buccal mucosal cells.

The term "control" refers, for example, to a cell or group of cells that is exhibiting common characteristics for the particular cell type from which the cell or group of cells was isolated. A normal cell sample does not exhibit tumorigenic potential, metastatic potential, or aberrant growth in vivo or in vitro. A normal control cell sample can be isolated from tissues in a subject that is not suffering from cancer. It may not be necessary to isolate a normal control cell sample each time a cell sample is tested for cancer as long as the normal control cell sample allows for probing during the testing procedure. In some embodiments, the levels of expression of the protein markers in the stromal cell sample are compared to the levels of expression of the protein markers in a normal control cell sample of the same tissue type as the cell sample.

A "control" refers, for example, to a sample of biological material representative of healthy, cancer-free animals, and/or cells or tissues. The level of caveolin-1 and/or caveolin-2 in a control sample is desirably typical of the general population of normal, cancer-free animals or off particular individual at a particular time (e.g. before, during or after a treatment regimen), or in a particular tissue. This sample can be removed from an animal expressly for use in the methods described in this invention, or can be any biological material representative of normal, cancer-free animals, including cancer-free biological material taken from an animal with cancer elsewhere in its body. A control sample can also refer to an established level of caveolin-1 and/or caveolin-2, representative of the cancer-free population, that has been previously established based on measurements from normal, cancer-free animals. In one embodiment, the control may be adjacent normal tissue. In one embodiment, the control may be any commonly used positive or negative controls. In one embodiment, the control is a non-invasive, non-metastatic control sample. Kits may also comprise, for example, positive and negative control samples for quality control purposes.

**Monitoring of Effects During Clinical Trials**

Monitoring the influence of agents (e.g., drugs, compounds) on the expression or activity of caveolin-1 and/or caveolin-2 (e.g., the ability to modulate aberrant cell proliferation and/or differentiation) can be applied not only in basic drug screening, but also in clinical trials. For example, the effectiveness of an agent determined by a screening assay as described herein to increase caveolin-1 gene expression, protein levels, or upregulate caveolin-1 activity, can be monitored in clinical trails of subjects exhibiting decreased caveolin-1 expression, protein levels, or downregulated caveolin-1 activity or expression, for example in stromal cells. Alternatively, the effectiveness of an agent determined by a screening assay to decrease caveolin-1 expression, protein levels, or downregulate caveolin-1 and/or caveolin-2 activity or expression, can be monitored in clinical trails of subjects exhibiting increased caveolin-1 expression, protein levels, or upregulated caveolin-1 and/or caveolin-2 activity. In such clinical trials, the expression or activity of caveolin-1 and/or caveolin-2 and, preferably, other genes that have been implicated in, for example, a cellular proliferation or immune disorder can be used as a "read out" or markers of the immune responsiveness of a particular cell.

In one embodiment, the invention provides a method for monitoring the effectiveness of treatment of a subject with an agent (e.g., an agonist, antagonist, protein, peptide, peptidomimetic, nucleic acid, small molecule, or other drug candidate) comprising the steps of (i) obtaining a pre-administration sample from a subject prior to administration of the agent: (ii) detecting the level of expression of a caveolin-1 and/or caveolin-2 protein, in the preadministration sample; (iii) obtaining one or more post-administration samples from the subject; (ill) detecting the level of expression or activity of the caveolin-1 and/or caveolin-2 protein, in the post-administration samples; (v) comparing the level of expression or activity of the caveolin-1 and/or caveolin-2 protein, in the pre-administration sample with the caveolin-1 protein, in the post administration sample or samples; and (vi) altering the administration of the agent to the subject accordingly. For example, increased administration of the agent may be desirable to increase the expression or activity of caveolin-1 to higher levels than detected, i.e., to increase the effectiveness of the agent. Alternatively, decreased administration of the agent may be desirable to decrease expression or activity of caveolin-1 and/or caveolin-2 to lower levels than detected, i.e., to decrease the effectiveness of the agent.

**Methods of Treatment**

The invention provides for both prophylactic and therapeutic methods of treating a subject at risk of (or susceptible to) a disorder or having a disorder associated with aberrant caveolin-1 expression or activity. The disorders include, but are not limited to cell proliferative disorder such as cancer.

**Prophylactic Methods**

In one aspect, the invention provides a method for preventing, in a subject, a disease or condition associated with an aberrant caveolin-1 expression or activity, by administering to the subject an agent that modulates caveolin-1 and/or caveolin-2 expression or at least one caveolin-1 activity, in for example stromal cells. Subjects at risk for a disease that is caused or contributed to by aberrant caveolin-1 and/or caveolin-2 expression or activity can be identified by, for example, any or a combination of diagnostic or prognostic assays as described herein. Administration of a prophylactic agent can occur prior to the manifestation of symptoms characteristic of the caveolin-1 aberrancy, such that a disease or disorder is prevented or, alternatively, delayed in its progression. Depending upon the type of caveolin-1 and/or caveolin-2 aberrancy, for example, a caveolin-1 and/or caveolin-2 agonist or caveolin-1 and/or caveolin-2 antagonist agent can be used for treating the subject. The appropriate agent can be determined based on screening assays described herein.

**Therapeutic Methods**

Another aspect of the invention pertains to methods of modulating caveolin-1 expression or activity in, for example stromal cells, for therapeutic purposes. The modulatory method of the invention involves contacting a cell with an agent that modulates one or more of the activities of caveolin-1 protein activity associated with the cell. An agent that modulates caveolin-1 protein activity can be an agent as described herein, such as a nucleic acid or a protein, a naturally-occurring cognate ligand of a caveolin-1 and/or caveolin-2 protein, a peptide, a caveolin-1 peptidomimetic, or other small molecule. In one embodiment, the agent stimulates one or more caveolin-1 and/or caveolin-2 protein activity. Examples of such stimulatory agents include active caveolin-1 protein and a nucleic acid molecule encoding caveolin-1 that has been introduced into the cell. In another embodiment, the agent inhibits one or more caveolin-1 protein activity. Examples of such inhibitory agents include antisense caveolin-1 nucleic acid molecules and anti-caveolin-1 antibodies. These modulatory methods can be performed in vitro (e.g. by culturing the cell with the agent) or, alternatively, in vivo (e.g., by administering the agent to a subject). As such, the invention provides methods of treating an individual afflicted with a disease or disorder characterized by aberrant expression or activity of a caveolin-1 protein molecule. In one embodiment, the method involves administering an agent (e.g., an agent identified by a screening assays described herein), or combination of agents that modulates (e.g., up-regulates or down-regulates) caveolin-1 and/or caveolin-2 expression or activity.

Stimulation of caveolin-1 and/or caveolin-2 activity is desirable in situations in which caveolin-1 is abnormally downregulated and/or in which increased caveolin-1 activity is likely to have a beneficial effect One example of such a situation is where a subject has a disorder characterized by aberrant cell proliferation and/or differentiation (e.g., cancer or immune associated disorders). Another example of such a situation is where the subject has a gestational disease (e.g., preclampsia).

**Determination of the Biological Effect of the Therapeutic**

In various embodiments of the invention, suitable in vitro or in vivo assays are performed to determine the effect of a specific therapeutic and whether its administration is indicated for treatment of the affected tissue.

In various specific embodiments, in vitro assays may be performed with representative cells of the type(s) involved in the patient's disorder, to determine if a given Therapeutic exerts the desired effect upon the cell type(s). Compounds for use in therapy may be tested in suitable animal model systems including, but not limited to rats, mice, chicken, cows, monkeys, rabbits, and the like, prior to testing in human subjects. Similarly, for in vivo testing, any of the animal model system known in the art may be used prior to administration to human subjects.

**Kits**

As used herein, the term "label" encompasses chemical or biological molecules that are used in detecting the presence in a sample of a target molecule which is capable of binding to or otherwise interact with the label so as to indicate its presence in the sample, and the amounts of the target molecule in the sample. Examples of such labels include, but not limited to, a nucleic acid probe such as a DNA probe, or RNA probe, an antibody, a radioisotope, a fluorescent dye, and the like.

As used herein, the term "usage instruction" includes instructions in the kit for carrying out the procedure for detecting the presence of a target molecular such as caveolin-1 in the sample to be tested. In the context of kit being used in the United States, the usage instruction comprising the statement of intended use required by the U.S. Food and Drug Administration (FDA) in labeling in vitro diagnostic products. It would be apparent to one with ordinary skill in the art of medical diagnostic devices as to the format and content of these usage instructions as required by the FDA.

As used in the present invention, an appropriate binding assay for selecting Specific caveolin-1-related angiogenesis inhibitor includes HPLC, immunoprecipitation, fluorescent-binding assay, capillary electrophoresis, and so forth.

As used herein, an "anti-angiogenesis assay" is an experiment where a pool of candidate molecules are screened in order to discover the effectiveness of the candidate molecules in inhibiting angiogenesis. In order to discover whether a molecule has anti-angiogenesis property, various methods can be applied to carry out the present invention. For example, proteins and peptides derived from these and other sources, including manual or automated protein synthesis, may be quickly and easily tested for endothelial proliferation inhibiting activity using a biological activity assay such as the bovine capillary endothelial cell proliferation assay. Other bioassays for inhibiting activity include the chick embryonic chorioallantoic membrane (CAM) assay, the mouse corneal assay, and the effect of administering isolated or synthesized proteins on implanted tumors. The chick CAM assay is described by O'Reilly, et al. in "Angiogenic Regulation of Metastatic Growth", Cell, vol. 79 (2). Oct. 21, 1994, pp. 315-328, which is hereby incorporated by reference in its entirety. Additional anti-angiogenesis assays for screening for angiogenesis inhibitors can be found in Yu, et al., PNAS, Vol. 101. No. 21, pp 8005-8010 (2004), which is hereby incorporated by reference in its entirety.

In some embodiments of the invention, methods such as flow cytometry as well as Enzyme-linked Immunosorbent Assay (ELISA) techniques are used for quantification of the caveolin-1 peptide..

Detection of the protein molecule of caveolin-1 can be performed using techniques known in the art (e.g., radioimmunoassay, ELISA (enzyme-linked immunosorbant assay), "sandwich" immunoassays, immunoradiometric assays, gel diffusion precipitation reactions, immunodiffusion assays, in situ immunoassays (e.g., using colloidal gold, enzyme or radioisotope labels, for example), Western blots, precipitation reactions, agglutination assays (e.g., gel agglutination assays, hemagglutination assays, etc.), complement fixation assays, immunofluorescence assays, protein A assays, and immunoelectrophoresis assays, etc.

For example, antibody binding is detected by detecting a label on the primary antibody. In another embodiment, the primary antibody is detected by detecting binding of a secondary antibody or reagent to the primary antibody. In a further embodiment, the secondary antibody is labeled. Many methods are known in the art for detecting binding in an immunoassay and are within the scope of the present invention.

In certain cases, an automated detection assay is utilized. Methods for the automation of immunoassays include those described in U.S. Pat. Nos. 5,885,530, 4,981,785, 6,159,750, and 5,358,691, each of which is herein incorporated by reference. In some embodiments, the analysis and presentation of results is also automated. For example, in some embodiments, software that generates a prognosis based on the presence or absence of a series of proteins corresponding to cancer markers is utilized.

Antibodies specific for caveolin-1 and/or caveolin-2 and caveolin-1 analogs and/or Caveolin-2 analogs are made according to techniques and protocols well known in the art. The antibodies may be either polyclonal or monoclonal. The antibodies are utilized in well-known immunoassay formats, such as competitive and non-competitive immunoassays, including ELISA, sandwich immunoassays and radioimmunoassays (RIAs), to determine the presence or absence of the endothelial proliferation inhibitors of the present invention in body fluids. Examples of body fluids include but are not limited to blood, serum, peritoneal fluid, pleural fluid, cerebrospinal fluid, uterine fluid; saliva, and mucus.

The present invention provides isolated antibodies that can be used in the diagnostic kits in the detection of caveolin-1 and/or caveolin-2. In preferred embodiments, the present invention provides monoclonal antibodies that specifically bind to cavcolin-1 and/or caveolin-2.

An antibody against caveolin-1 in the present invention may be any monoclonal or polyclonal antibody, as long as it'can recognize the protein. Antibodies can be produced by using caveolin-1 and/or caveolin-2 or its analogue as the antigen using conventional antibody or antiserum preparation processes.

The present invention contemplates the use of both monoclonal and polyclonal antibodies. Any suitable method may be used to generate the antibodies used in the methods and compositions of the present invention, including but not limited to, those disclosed herein. For example, for preparation of a monoclonal antibody, protein, as such, or together with a suitable carrier or diluent is administered to an animal (e.g., a mammal) under conditions that permit the production of antibodies. For enhancing the antibody production capability, complete or incomplete Freund's adjuvant may be administered. Normally, the protein is administered once every 2 weeks to 6 weeks, in total, about 2 times to about 10 times. Animals suitable for use in such methods include, but are not limited to, primates, rabbits, dogs, guinea pigs, mice, rats, sheet, goats, etc.

For preparing monoclonal antibody-producing cells, an individual animal whose antibody titer has been confirmed (e.g., a mouse) is selected, and 2 days to 5 days after the final immunization, its spleen or lymph node is harvested and antibody-producing cells contained therein are fused with myeloma cells to prepare the desired monoclonal antibody producer hybridoma. Measurement of the antibody titer in antiserum can be carried out, for example, by reacting the labeled protein, as described hereinafter with the antiserum and then measuring the activity of the labeling agent bound to the antibody. The cell fusion can be carried out according to known methods, for example, the method described by Koehler and Milstein (Nature 256:495 [1975]). As a fusion promoter, for example, Sendai virus (HVJ) or, preferably, polyethylene glycol (PEG), is used.

Polyclonal antibodies .may be prepared by any known method or modifications of these methods including obtaining antibodies from patients. For example, a complex of an immunogen (an antigen against the protein) and a carrier protein is prepared and an animal is immunizes by the complex according to the same manner as that described with respect to the above monoclonal antibody preparation. A material containing the antibody against is recovered from the immunized animal and the antibody is separated and purified.

The present invention provides for a method of inhibiting the proliferation and/or migration of endothelial cells by producing a combination antibody containing an anti-caveolin-1 antibody linked to a cytotoxic agent such as a chemokine, i.e. a Tumor Necrosis Factor-alpha, etc. When such a combination antibody is administered to a cell sample including an endothelial cells, the anti-caveolin-1 antibody will direct the toxic agent to the endothelial cells, and thus bring the toxic agent such as Tumor Necrosis Factor-alpha to act upon the endothelial cells, and filling the cell growth.

Methods of linking an antibody to a second agent such as a cytotoxic agent in order to form a combination antibody, also know as an immunotoxic, is well known in the art. Two major advances in the immunotoxin field have been the use of the recombinant DNA technique to produce recombinant toxins with better clinical properties and the production of single-chain immunotoxins by fusing the DNA elements encoding combining regions of antibodies, growth factors, or cytokines to a toxin gene.

First-generation immunotoxins were constructed by coupling toxins to MAb or antibody fragments using a heterobifunctional cross-linking agent. It was also discovered that genetic engineering could be used to replace the cell-binding domains of bacterial toxins with the Fv portions of antibodies or with growth factors.

The present invention provides kits for the detection and characterization of caveolin-1 in cancer diagnostics. In some embodiments, the kits contain antibodies specific for caveolin-1, in addition to detection reagents and buffers. In other embodiments, the kits contain reagents specific for the detection of caveolin-1. In preferred embodiments, the kits contain all -of the components necessary to perform a detection assay, including all controls, directions for performing assays, and any necessary software for analysis and presentation of results.

Kits containing labels such as antibodies against caveolin-1 for measurement of caveolin-1 are also contemplated as part of the present invention. Antibody solution is prepared such that it can detect the presence of caveolin-1 peptides in extracts of plasma, urine, tissues, and in cell culture media are further examined to establish easy to use kits for rapid, reliable, sensitive, and specific measurement and localization of caveolin-1. These assay kits include but are not limited to the following techniques; competitive and non-competitive assays, radioimmunoassay; bioluminescence and chemiluminescence assays, fluorometric assays, sandwich assays, immunoradiometric assays, dot blots, enzyme linked assays including ELISA, microtiter plates, antibody coated strips or dipsticks for rapid monitoring of urine or blood, and immunocytochemistry. For each kit the range, sensitivity, precision, reliability, specificity and reproducibility of the assay are established according to industry practices that are commonly known to and used by one with ordinary skill in the art.

This caveolin-1 immunohistochemistry kit provides instructions, caveolin-1 molecules, preferably labeled and linked to a fluorescent molecule such as fluorescein isothiocyanate, or to some other reagent used to visualize the primary antiserum. Immunohistochemistry techniques are well known to those skilled in the art. This caveolin-1 immunohistochemistry kit permits localization of caveolin-1 in tissue sections and cultured cells using both light and electron microscopy. It is used for both research and clinical purposes. For example, tumors are biopsied or collected and tissue sections cut with a microtome to examine sites of caveolin-1 production. Such information is useful for diagnostic and possibly therapeutic purposes in the detection and treatment of cancer.

**Diagnostic Applications**

The subject antibody and/or polypeptide compositions may be used in a variety of diagnostic applications. Exemplary embodiments of such diagnostic applications are described below.

**Diagnosis and Prognosis of Cancer by Detection of caveolin-1 and/or caveolin-2**

**Expression and/or Expression Levels**

As noted above, the present invention is based on the discovery that caveolin-1 and/or caveolin-2 expression in stromal cells is decreased in cells of high metastatic potential relative to cells of low metastatic potential, cells of non-metastatic potential, and to normal cells. In general, the terms "high metastatic potential" and "low metastatic potential" are used to describe the relative ability of a cell to give rise to metastases in an animal model, with "high metastatic potential" cells giving rise to a larger number of metastases and/or larger metastases than "low metastatic potential" cells. Thus, a cell of high metastatic potential poses a greater risk of metastases to the subject than a cell of low metastatic potential. "Non-metastatic cells" are those cells that are cancerous, but that do not develop detectable metastases following injection in an animal model.

The invention thus features methods and compositions for diagnosis and prognosis, as well as grading and staging of cancers, by detection of caveolin-1 and/or caveolin-2 expression in a biological test sample, e.g. cell sample or tissue sample. The methods of the invention can also be used to monitor patients having a predisposition to develop a particular cancer, e.g., through inheritance of an allele associated with susceptibility to a cancer (e.g., BRCA1; BRCA2, TP53, ATM, or APC for breast cancer). Detection and monitoring of caveolin-1 expression levels can be used to detect potentially malignant events at a molecular level before they are detectable at a gross morphological level.

In general, diagnosis, prognosis, and grading and/or staging of cancers may be performed by a number of methods to determine the relative level of expression of the differentially expressed caveolin-1 and/or caveolin-2 gene at the transcriptional level, and/or the absence or presence or altered amounts of a normal or abnormal caveolin-1 polypeptide in patient cells. As used herein, "differentially expressed gene" is intended to refer to a gene having an expression level (e.g., which in turn is associated with a level of caveolin-1 polypeptide production and/or caveolin-1 transcription) that is associated with a decrease in expression level of at least about 25%, usually at least about 50% to 75%, more usually at least about 90% or more: In general, such a decrease in differentially expressed caveolin-1 is indicative of the onset or development of the metastatic phenotype

"Diagnosis" as used herein generally includes determination of a subject's susceptibility to a disease or disorder, determination as to whether a subject is unaffected, susceptible to, or presently affected by a disease or disorder, and/or to identify a tumor as benign, non-cancerous, or cancerous (e.g., non-metastatic or metastatic, e.g., high metastatic potential or low metastatic potential). "Prognosis" is used herein to generally mean a determination of the severity of disease (e.g., identification or pre-metastatic or metastatic cancerous states, stages of cancer, etc..), which in turn can be correlated with the potential outcome, response to therapy, etc. A complete diagnosis thus can include diagnosis as discussed above, as well as determination of prognosis, cancer staging, and tumor grading. The present invention particularly encompasses diagnosis and prognosis of subjects in the context of cancers of various origins, particularly breast cancer (e.g., carcinoma in situ (e.g., ductal carcinoma in situ), estrogen receptor (ER)-positive breast cancer, ER-negative breast cancer, or other forms and/or stages of breast cancer) and prostate cancer.

"Sample" or "biological sample" as used throughout here are generally meant to refer to samples of biological fluids or tissues, particularly samples obtained from tissues, especially from cells of the type associated with the disease for which the diagnostic application is designed (e.g., stromal cells, and/or ductal adenocarcinoma), and the like. "Samples" is also meant to encompass derivatives and fractions of such samples (e.g., cell lysates). Where the sample is solid tissue, the cells of the tissue can be dissociated or tissue sections can be analyzed.

Methods of the subject invention useful in diagnosis or prognosis typically involve , comparison of the amount of caveolin-1 and/or caveolin-2 gene product in a sample of interest with that of a control to detect relative differences in the expression of the gene product, where the difference can be measured qualitatively and/or quantitatively. Quantitation can be accomplished, for example, by comparing the level of expression product detected in the sample with the amounts of product present in a standard curve. A comparison can be made visually using ELISA to detect relative amounts of caveolin-1 and/or caveolin-2 polypeptides in test and control samples; by using a technique such as densitometry, with or without computerized assistance, to detect relative amounts of detectably labeled caveolin-1 and/or caveolin-2 polypeptides; or by using an array to detect relative levels of anti-caveolin-1 polypeptide antibody binding, and comparing the pattern of antibody binding to that of a control.

In some embodiments of the methods of the invention it may be particularly desirable to detect expression of a caveolin-1 and/or caveolin-2 gene products as well as at least one gene product other caveolin-1. Caveolin-1 and/or caveolin-2 expression decreases upon development of metastasis, and may be undetectable in metastatic cells, while caveolin-1 is expressed in non-metastatic and in normal cells. It may also be desirable to detect expression of other gene products in addition to caveolin-1 and/or caveolin-2.

Other gene products that can serve as controls or increase the sensitivity of classification of the metastatic phenotype of a cell, as well as gene products that can serve as controls for identification of normal cells (e.g., gene products that are expressed in normal cells but not in cancerous cells, or expressed in normal cells, but not in metastatic cells, etc.) are known in the art. In addition, the cells can be classified as normal or cancerous based on conventional methodologies such as general morphology as determined by light microscopy. For example, conventional techniques for classifying a cell as cancerous based on morphology can be performed prior to or simultaneously with detection of caveolin-1 expression. Thus, a cell that exhibits abnormal morphology associated with the cancer phenotype, and that expresses a low level of caveolin-1 relative to a normal cells or in which caveolin-1 expression is not detectable is identified as a cell of high metastatic potential.

Methods for qualitative and quantitative detection of caveolin-1 polypeptides in a sample, as well as methods for comparing such to control samples are well known in the art. The patient from whom the sample is obtained can be apparently healthy, susceptible to disease (e.g., as determined by family history or exposure to certain environmental factors), or can already be identified as having a condition in which altered expression of a gene product of the invention is implicated.

In the assays of the invention, the diagnosis can be determined based on detected caveolin-1 gene product expression levels, and may also include detection of additional diagnostic markers and/or reference sequences. Where the diagnostic method is designed to detect the presence or susceptibility of a patient to metastatic cancer, the assay preferably involves detection of a caveolin-1 and/or caveolin-2 gene product and comparing the detected gene product levels to a level associated with a normal sample, to levels associated with a low metastatic potential sample, and/or to level associated with a high metastatic potential sample. For example, detection of a lower level of caveolin-1 and/or caveolin-2 expression relative to a normal level is indicative of the presence in the sample of a cell having high metastatic potential. Given the disclosure provided herein, variations on the diagnostic and prognostic assays described herein will be readily apparent to the ordinarily skilled artisan.

Any of a variety of detectable labels can be used in connection with the various methods of the invention. Suitable detectable levels include fluorochromes, radioactive labels, and the like. Suitable labels include, but are not necessarily limited to, fluorochromes, e.g. fluorescein isothiocyanate (FITC), rhodamine, Texas Red, phycoerythrin, allophycocyanin, 6-carboxyfluorescein (6-FAM), 2',7-dimethoxy-4',5'-dichloro-6-carboxyfluorescein (JOE), 6-carboxy-X-rhodamine (ROX), 6-carboxy-2',4',7',4,7-hexachlorofluorescein (HEX). 5-carboxyfluorescein (5-FAM) or N,N,N',N'-tetramethyl-6-carboxyrhodamine (TAMRA), radioactive labels, e.g. 32P, 35S, 3H; etc. The detectable label can involve a two stage system (e.g., biotin-avidin, hapten-anti-hapten antibody, etc.).

Reagents specific for the polynucleotides and polypeptides of the invention, such as detectably labeled antibodies or detectably labeled nucleotide probes, can be supplied in a kit for detecting the presence of an expression product in a biological sample. The kit can also contain buffers or labeling components, as well as instructions for using the reagents to detect and quantify expression products in the biological sample. Exemplary embodiments of the diagnostic methods of the invention are described below in more detail.

## Polypeptide Detection in Diagnosis, Prognosis, Cancer Grading and Cancer Staging

In one embodiment, the test sample is assayed for the level of a caveolin-1 polypeptide. Diagnosis can be accomplished using any of a number of methods to determine the absence or presence or altered amounts of the differentially expressed polypeptide in the test sample. For example, detection can utilize staining of cells or histological sections (e.g., from a biopsy sample) with labeled antibodies, performed in accordance with conventional methods. Cells can be permeabilized to stain cytoplasmic molecules. In general, antibodies that specifically bind a differentially expressed polypeptide of the invention are added to a sample, and incubated for a period of time sufficient to allow binding to the epitope, usually at least about 10 minutes. The antibody can be detectably labeled for direct detection (e.g., using radioisotopes, enzymes, fluorescers, chemiluminescers, and the like), or can be used in conjunction with a second stage antibody or reagent to detect binding (e.g., biotin with horseradish peroxidase-conjugated avidin, a secondary antibody conjugated to a fluorescent compounds, e.g. fluorescein, rhodamine, Texas red, etc.). The absence or presence of antibody binding can be determined by various methods, including flow symmetry of dissociated cells, microscopy, radiography, scintillation counting, etc. Any suitable alternative methods can of qualitative or quantitative detection of levels or amounts of differentially expressed polypeptide can be used, for example ELISA, western blot, immunoprecipitation, radioimmunoassay, etc.

In general, the detected level of caveolin-1 polypeptide in the test sample is compared to a level of the differentially expressed gene product in a reference or control sample, e.g., in a normal cell or in a cell having a known disease state (e.g., cell of high metastatic potential).

## Immunological Methods

In the context of the present invention, "immunological methods" are understood as meaning analytical methods based on immunochemistry, in particular on an antigen-antibody reaction. Examples of immunological methods include immunoassays such as radioimmunoassay (RIA), enzyme immunoassay (EIA, combined with solid-phase technique: ELISA) or else immunofluorescence assays. The immunoassay is carried out by exposing the sample to be investigated to an SP-C-binding antibody and detecting and quantifying the amount of antibody which binds to SP-C In these assays, detection and quantification is carried out directly or indirectly in a known manner. Thus, detection and quantification of the antigen-antibody complexes is made possible by using suitable labels which may be carried by the antibody directed against SP-C and/or by a secondary antibody directed against the primary antibody. Depending on the type of the abovementioned immunoassays, the labels are, for example, radioactive labels, fluorescent dyes or else enzymes, such as phosphatase or peroxidase, which can be detected and quantified with the aid of a suitable substrate.

In one embodiment of the invention, the immunological method is carried out with the aid of a suitable solid phase. Suitable solid phases which may be mentioned include the customary commercial microtiter plates made of polystyrene or membranes (for example made of polyvinylidene difluoride, PVDF) which are customarily used for the ELISA technique. Surprisingly, it has been found that even chromatography plates are suitable for use as solid phase in the process according to the invention. The implementation of the process according to the invention using chromatography plates is hereinbelow also referred to as immuno-TLC.

**Screening for Caveolin-1 and/or Caveolin-2 Targeted Drugs**

In one embodiment, any of the caveolin-1 and/or caveolin-2 sequences as described herein are used in drug screening assays. The caveolin-1 and/or caveolin-2 proteins, antibodies, nucleic acids, modified proteins and cells containing caveolin-1 and/or caveolin-2 sequences are used in drug screening assays or by evaluating the effect of drug candidates on a "gene expression profile" or expression profile of polypeptides. In one embodiment, the expression profiles are used, preferably in conjunction with high throughput screening techniques to allow monitoring for expression profile genes after treatment with a candidate agent, Zlokarnik, et al., Science 279, 84-8 (1998), Heid, et al., Genome Res., 6:986-994 (1996).

In another-embodiment, the caveolin-1 and/or caveolin-2 proteins, antibodies, nucleic acids, modified proteins and cells containing the native or modified caveolin-1 and/or caveolin-2 proteins are used in screening assays. That is, the present invention provides novel methods for screening for compositions that modulate the cancer phenotype. This can be done by screening for modulators of gene expression or for modulators of proteins activity. Similarly, this may be done on an individual gene or protein level or by evaluating the effect of drug candidates on a "gene expression profile". In a preferred embodiment, the expression profiles are used, preferably in conjunction with high throughput screening techniques to allow monitoring for expression profile genes after treatment with a candidate agent, see Zlokarnik, supra.

Having identified the caveolin-1 and/or caveolin-2 genes herein, a variety of assays to evaluate the effects of agents on gene expression may be executed. In a preferred embodiment, assays may be run on an individual gene or protein level. That is, having identified a particular gene as aberrantly regulated in cancer, candidate bioactive agents may be screened to modulate the gene's regulation. "Modulation" thus includes both an increase and a decrease in gene expression or activity. The preferred amount of modulation will depend on the original change of the gene expression in normal versus tumor tissue, with changes of at least 10%, preferably 50%, more preferably 100-300%, and in some embodiments 300-1000% or greater. Thus, if a gene exhibits a 4 fold increase in tumor compared to normal tissue, a decrease of about four fold is desired; a 10 fold decrease in tumor compared to normal tissue gives a 10 fold increase in expression for a candidate agent is desired, etc. Alternatively, where the caveolin-1 and/or caveolin-2 sequence has been altered but shows the same expression profile or an altered expression profile, the protein will be detected as outlined herein.

As will be appreciated by those in the art, this may be done by evaluation at either the gene or the protein level; that is, the amount of gene expression may be monitored using nucleic acid probes and the quantification of gene expression levels, or, alternatively, the level of the gene product itself can be monitored, for example through the use of antibodies to the caveolin-1 and/or caveolin-2 protein and standard immunoassays. Alternatively, binding and bioactivity assays with the protein may be done as outlined below.

In a preferred embodiment, gene expression monitoring is done and a number of genes, i.e. an expression profile, is monitored simultaneously, although multiple protein expression monitoring can be done as well.

In this embodiment, the caveolin-1 and/or caveolin-2 nucleic acid probes are attached to biochips as outlined herein for the detection and quantification of caveolin-1 and/or caveolin-2 sequences in a particular cell. The assays are further described below.

Generally, in a preferred embodiment, a candidate bioactive agent is added to the cells prior to analysis. Moreover, screens are provided to identify a candidate bioactive agent that modulates a particular type of cancer, modulates caveolin-1 and/or caveolin-2 proteins, binds to a caveolin-1 and/or caveolin-2 protein, or interferes between the binding of a caveolin-1 and/or caveolin-2 protein and an antibody.

The term "potential therapeutic agent" "candidate bioactive agent" or "drug candidates" or grammatical equivalents as used herein describes any molecule, e.g., protein, oligopeptide, small organic or inorganic molecule, polysaccharide, polynucleotide, etc., to be tested for bioactive agents that are capable of directly or indirectly altering either the cancer phenotype, binding to and/or modulating the bioactivity of a caveolin-1 and/or caveolin-2 protein, or the expression of a caveolin-1 and/or caveolin-2 sequence, including both nucleic acid sequences and protein sequences. In a particularly preferred embodiment, the candidate agent increases a caveolin-1 and/or caveolin-2 phenotype, for example to a normal tissue fingerprint. Generally a plurality of assay mixtures are run in parallel with different agent concentrations to obtain a differential response two the various concentrations. Typically, one of these concentrations serves as a negative control, i.e., at zero concentration or below the level of detection.

In one aspect, a candidate agent will neutralize the effect of a caveolin-1 and/or caveolin-2 protein. By "neutralize" is meant that activity of a protein is either inhibited or counter acted against so as to have substantially no effect on a cell.

Potential therapeutic agents encompass numerous chemical classes, though typically they are organic or inorganic molecules, preferably small organic compounds having a molecular weight of more than 100 and less than about 2,500 Daltons. Preferred small molecules are less than 2000, or less than 1500 or less than 1000 or less than 500 D. Candidate agents comprise functional groups necessary for structural interaction with proteins, particularly hydrogen bonding, and typically include at least an amine, carbonyl, hydroxyl or carboxyl group, preferably at least two of the functional chemical groups. The candidate agents often comprise cyclical carbon or heterocyclic structures and/or aromatic or polyaromatic structures substituted with one or more of the above functional groups. Candidate agents are also found among biomolecules including peptides, saccharides, fatty acids, steroids, purines, pyrimidines, derivatives, structural analogs or combinations thereof. Particularly preferred are peptides.

Candidate agents are obtained from a wide variety of sources including libraries of synthetic or natural compounds. For example, numerous means are available for random and directed synthesis of a wide variety of organic compounds and biomolecules, including expression of randomized oligonucleotides. Alternatively, libraries of natural compounds in the form of bacterial, fungal, plant and animal extracts are available or readily produced. Additionally, natural or synthetically produced libraries and compounds are readily modified through conventional chemical, physical and biochemical means. Known pharmacological agents may be subjected to directed or random chemical modifications, such as acylation, alkylation, esterification, or amidification to produce structural analogs.

In one embodiment, the candidate bioactive agents are proteins. By "protein" herein is meant at least two covalently attached amino acids, which includes proteins, polypeptides, oligopeptides and peptides. The protein may be made up of naturally occurring amino acids and peptide bonds, or synthetic peptidomimetic structures. Thus "amino acid", or "peptide residue", as used herein means both naturally occurring and synthetic amino acids. For example, homo-phenylalanine, citrulline and norleucine are considered amino acids for the purposes of the invention. "Amino acid" also includes imino acid residues such as proline and hydroxyproline. The side chains may be in either the (R) or the (S) configuration. In the preferred embodiment, the amino acids are in the (S) or L-configuration. If non-naturally occurring side chains are used, non-amino acid substituents may be used, for example to prevent or retard in vivo degradations.

In a preferred embodiment, the candidate bioactive agents are naturally occurring proteins or fragments of naturally occurring proteins. Thus, for example, cellular extracts containing proteins, or random or directed digests of proteinaceous cellular extracts, may be used. In this way libraries of prokaryotic and eukaryotic proteins may be made for screening in the methods of the invention. Particularly preferred in this embodiment are libraries of bacterial, fungal, viral, and mammalian proteins, with the latter being preferred, and human proteins being especially preferred.

In another preferred embodiment, the candidate bioactive agents are peptides of from about 5 to about 30 amino acids, with from about 5 to about 20 amino acids being preferred, and from about 7 to about 15 being particularly preferred. The peptides may be digests of naturally occurring proteins as is outlined above, random peptides, or "biased" random peptides. By "randomized" or grammatical equivalents herein is meant that each nucleic acid and peptide consists of essentially random nucleotides and amino acids, respectively. Since generally these random peptides (or nucleic acids, discussed below) are chemically synthesized, they may incorporated any nucleotide or amino acid at any position. The synthetic process can be designed to generate randomized proteins or nucleic acids, to allow the formation of all or most of the possible combinations over the length of the sequence, thus forming a library of randomized candidate bioactive proteinaceous agents.

In one embodiment, the library is fully randomized, with no sequence preferences or constants at any position. In a referred embodiments, the library is biased. That is, some positions within the sequences are either held constant, or are selected from a limited number of possibilities. For example, in a preferred embodiment, the nucleotides or amino acid residues are randomized within a defined class, for example, of hydrophobic amino acids, hydrophilic residues, sterically biased (either small or large) residues, towards the creation of nucleic acid binding domains, the creation of cysteines, for cross-linking, prolines for SH-3 domains, serines, threonines, tyrosines or histidines for phosphorylation sites, etc., or to purines, etc.

In one embodiment, the candidate bioactive agents are nucleic acids. As described generally for proteins, nucleic acid candidate bioactive agents may be naturally occurring nucleic acids, random nucleic acids, or "biased" random nucleic acids. In another embodiment, the candidate bioactive agents are organic chemical moieties, a wide variety, of which are available in the literature.

In assays for testing alteration of the expression profile of one or more caveolin-1 and/or caveolin-2 genes, after the candidate agent has been added and the cells allowed to incubate for some period of time, a nucleic acid sample, containing the target sequences to be analyzed is prepared. The target sequence is prepared using known techniques (e.g., converted from RNA to labeled cDNA, as described above) and added to a suitable microarray. For example, an in vitro reverse transcription with labels covalently attached to the nucleosides is performed. Generally, the nucleic acids are labeled with a label as defined herein, especially with biotin-FITC or PE, Cy3 and Cy5.

As will be appreciated by those in the art, these assays can be direct hybridization assays or can comprise "sandwich assays", which include the use of multiple probes, as is generally outlined in U.S. Pat. Nos. 5,681,702, 5,597,909, 5,545,730, 5,594,117, 5,591,584, 5,571,670, 5,580,731, 5,571,670, 5,591,584, 5,624,802, 5,635,352, 5,594,118, 5,359,100, 5,124,246 and 5,681,697, all of which are hereby incorporated by reference. In this embodiment, in general, the target nucleic acid is prepared as outlined above, and then added to the biochip comprising a plurality of nucleic acid probes, under conditions that allow the formation of a hybridization complex.

A variety of hybridization conditions may be used in the present invention, including high, moderate and low stringency conditions as outlined above. The assays are generally run under stringency conditions that allow formation of the label probe hybridization complex only in the presence of target. Stringency can be controlled by altering a step parameter that is a thermodynamic variable, including, but not limited to, temperature, formamide concentration, salt concentration, chaotropic salt concentration, pH, organic solvent concentration, etc. These parameters may also be used to control non-specific binding, as is generally outlined in U.S. Pat. No. 5,681,697. Thus it may be desirable to perform certain steps at higher stringency conditions to reduce non-specific binding.

The reactions outlined herein may be accomplished in a variety of ways, as will be appreciated by those in the art. Components of the reaction may be added simultaneously, or sequentially, in any order, with preferred embodiments outlined below. In addition, the reaction may include a variety of other reagents in the assays. These include reagents like salts, buffers, neutral proteins, e.g. albumin, detergents, etc which may be used to facilitate optimal hybridization and detection, and/or reduce non-specific or background interactions. Also reagents that otherwise improve the efficiency of the assay, such as protease inhibitors, nuclease inhibitors, anti-microbial agents, etc., may be used, depending on the sample preparation methods and purity of the target. In addition, either solid phase or solution based (i.e.. kinetic PCR) assays may be used.

Once the assay is run, the data are analyzed to determine the expression levels, and changes in expression levels as between states, of individual genes, forming a gene expression profile.

In a preferred embodiment, as for the diagnosis and prognosis applications, having identified the differentially expressed gene(s) or mutated gene(s) important in any one state, screens can be run to test for alteration of the expression of the caveolin-1 and/or caveolin-2 genes individually. That is, screening for modulation of regulation of expression of a single gene can be done. Thus, for example, in the case of target genes whose presence or absence is unique between two states, screening is done for modulators of the target gene expression.

In addition, screens can be done for novel genes that are induced in response to a candidate agent. After identifying a candidate agent based upon its ability to modulate a caveolin-1 and/or caveolin-2 expression pattern leading to a normal expression pattern, or modulate a single caveolin-1 and/or caveolin-2 gene expression profile so as to mimic the expression of the gene from normal tissue, a screen as described above can be performed to identify genes that are specifically modulated in response to the agent. Comparing expression profiles between normal tissue and agent treated tissue reveals genes that are not expressed in normal tissue, but are expressed in agent treated tissue. These agent specific sequences can be identifed and used by any of the methods described herein for caveolin-1 and/or caveolin-2 genes or proteins. In particular these sequences and the proteins they encode find use in marking or identifying agent-treated cells.

Thus, in one embodiment, a candidate agent is administered to a population of cells, that thus has an associated expression profile. By "administration" or "contacting" herein is meant that the candidate agent is added to the cells in such a manner as to allow the agent to act upon the cells, whether by uptake and intracellular action, or by action at the cell surface. In some embodiments, nucleic acid encoding a proteinaceous candidate agent (i.e. a peptide) may be put into a viral construct such as a retroviral construct and added to the cell, such that expression of the peptide agent is accomplished; see PCT US97/01019, hereby expressly incorporated by reference.

Once the candidate agent has been administered to the cells, the cells can be washed if desired and are allowed to incubate under preferably physiological conditions for some period of time. The cells are then harvested and a new gene expression profile is generated, as outlined herein..

In a preferred embodiment, screening is done to alter the biological function of the expression product of the caveolin-1 and/or caveolin-2 gene. Again, having identified the importance of a gene in a particular state, screening for agents that bind and/or modulate the biological activity of the gene product can be run as is more fully outlined below.

In a preferred embodiments, screens are designed to first find candidate agents that can bind to caveolin-1 and/or caveolin-2 proteins, and then these agents may be used in assays that evaluate the ability of the candidate agent to modulate the caveolin-1 and/or caveolin-2 activity and the cancer phenotype. Thus, as will be appreciated by those in the art, there are a number of different assays that may be run; binding assays and activity assays.

In a preferred embodiment, binding assays are done. In general, purified or isolated gene product is used; that is, the gene products of one or more caveolin-1 and/or caveolin-2 nucleic acids are made. In general, this is done as is known in the art. For example, antibodies are generated to the protein gene products, and standard immunoassays are run to determine the amount of protein present Alternatively, cells comprising the caveolin-1 and/or caveolin-2 proteins can be used in the assays.

Thus, in a preferred embodiment, the methods comprise combining a caveolin-1 and/or caveolin-2 protein and a candidate bioactive agent, and determining the binding of the candidate agent to the caveolin-1 and/or caveolin-2 protein. Preferred embodiments utilize the human or mouse caveolin-1 and/or caveolin-2 protein, although other mammalian proteins may also be used, for example for the development of animal models of human disease. In some embodiments, as outlined herein, valiant or derivative caveolin-1 and/or caveolin-2 proteins may be used.

Generally, in a preferred embodiment of the methods herein, the caveolin-1 and/or caveolin-2 protein or the candidate agent is non-diffusably bound to an insoluble support having isolated sample receiving areas (e.g. a microtiter plate, an array, etc.). The insoluble support may be made of any composition to which the compositions can be bound, is readily separated from soluble material, and is otherwise compatible with the overall method of screening. The surface of such supports may be solid or porous and of any convenient shape. Examples of suitable insoluble supports include microtiter plates, arrays, membranes and beads. These are typically made of glass, plastic (e.g., polystyrene), polysaccharides, nylon or nitrocellulose, Teflon.RTM., etc. Microtiter plates and arrays are especially convenient because a large number of assays can be carried out simultaneously, using small amounts of reagents and samples.

The particular manner of binding of the composition is not crucial so long as it is compatible with the reagents and overall methods of the invention, maintains the activity of the composition and is nondiffusable. Preferred methods of binding include the use of antibodies (which do not sterically block either the ligand binding site or activation sequence when the protein is bound to the support), direct binding to "sticky" or ionic supports, chemical crosslinking, the synthesis of the protein or agent on the surface, etc. Following binding of the protein or agent, excess unbound material is removed by washing. The sample receiving areas may then he blocked through incubation with bovine serum albumin (BSA), casein or other innocuous protein or other moiety.

In a preferred embodiment, the caveolin-1 and/or caveolin-2 protein is bound to the support, and a candidate bioactive agent is added to the assay. Alternatively, the candidate agent is bound to the support and the caveolin-1 and/or caveolin-2 protein is added. Novel binding agents include specific antibodies, non-natural binding agents identified in screens of chemical libraries, peptide analogs, etc. Of particular interest are screening assays for agents that have a low toxicity for human cells. A wide variety of assays may be used for this purpose, including labeled in vitro protein-protein binding assays, electrophoretic mobility shift assays, immunoassays for protein binding, functional assays (phosphorylation assays, etc.) and the like.

The determination of the binding of the candidate bioactive agent to the caveolin-1 and/or caveolin-2 protein may be done in a number of ways. In a preferred embodiment, the candidate bioactive agent is labeled, and binding determined directly. For example, this may be done by attaching all or a portion of the caveolin-1 and/or caveolin-2 protein to a solid support, adding a labeled candidate agent (for example a fluorescent label), washing off excess reagent, and determining whether the label is present on the solid support. Various blocking and washing steps may be utilized as is known in the art.

By "labeled" herein is meant that the compound is either directly or indirectly labeled with a label which provides a detectable signal, e.g. radioisotope, fluorescers, enzyme, antibodies, particles such as magnetic particles, chemiluminescers, or specific binding molecules, etc. Specific binding molecules include pairs, such as biotin and streptavidin, digoxin and antidigoxin etc. For the specific binding members, the complementary member would normally be labeled with a molecule which provides for detection, in accordance with known procedures, as outlined above. The label can directly or indirectly provide a detectable signal.

In some embodiments, only one of the components is labeled. For example, the proteins (or proteinaceous candidate agents) may be labeled at tyrosine positions using sup.125I, or with fluordphores. Alternatively, more than one component may be labeled with different labels; using .sup.125I for the proteins, for example, and a fluorophore for the candidate agents.

In a preferred embodiment, the binding of the candidate bioactive agent is determined through the use of competitive binding assays. In this embodiment, the competitor is a binding moiety known to bind to the target molecule (i.e. caveolin-1 and/or caveolin-2 protein), such as an antibody, peptide, binding partner, ligand, etc. Under certain circumstances, there may be competitive binding as between the bioactive agent and the binding moiety, with the binding moiety displacing the bioactive agent.

In one embodiment, the candidate bioactive agent is labeled. Either the candidate bioactive agent, or the competitor, or both, is added first to the protein for a time sufficient to allow binding, if present. Incubations may be performed at any temperature which facilitates optimal activity, typically between 4 and 40.degree. C. Incubation periods are selected for optimum activity, but may also be optimized to facilitate rapid high throughput screening. Typically between 0.1 and 1 hour will be sufficient. Excess reagent is generally removed or washed away. The second component is then added, and the presence or absence of the labeled component is followed, to indicate binding.

In a preferred embodiment, the competitor is added first, followed by the candidate bioactive agent. Displacement of the competitor is an indication that the candidate bioactive agent is binding to the caveolin-1 and/or caveolin-2 protein and thus is capable of binding to, and potentially modulating, the activity of the caveolin-1 and/or caveolin-2 protein. In this embodiment, either component can be labeled. Thus, for example, if the competitor is labeled, the presence of label in the wash solution indicates displacement by the agent. Alternatively, if the candidate bioactive agent is labeled, the presence of the label on the support indicates displacement.

In an alternative embodiment, the candidate bioactive agent is added first, with incubation and washing, followed by the competitor. The absence of binding by the competitor may indicate that the bioactive agent is bound to the caveolin-1 and/or caveolin-2 protein with a higher affinity. Thus, if the candidate bioactive agent is labeled, the presence of the label on the support, coupled with a lack of competitor binding, may indicate that the candidate agent is capable of binding to the caveolin-1 and/or caveolin-2 protein.

In a preferred embodiment, the methods comprise differential screening to identity bioactive agents that are capable of modulating the activity of the caveolin-1 and/or caveolin-2 proteins. In this embodiment, the methods comprise combining a caveolin-1 and/or caveolin-2 protein and a competitor in a first sample. A second sample comprises a candidate bioactive agent, a caveolin-1 and/or caveolin-2 protein and a competitor. The binding of the competitor is determined for both sampled, and a change, or difference in binding between the two samples indicates the presence of an agent capable of binding to the caveolin-1 and/or caveolin-2 protein and potentially modulating its activity. That is, if the binding of the competitor is different in the second sample relative to the first sample, the agent is capable of binding to the caveolin-1 and/or caveolin-2 protein.

Positive controls and negative controls may be used in the assays. Preferably all control and test samples are performed in at least triplicate to obtain statistically significant results. Incubation of all samples is for a time sufficient for the binding of the agent to the protein. Following incubation, all samples are washed free of non-specifically bound material and the amount of bound, generally labeled agent determined. For example, where a radiolabel is employed, the samples may be counted in a scintillation counter to determine the amount of bound compound.

A variety of other reagents may be included in the screening assays. These include reagents like salts, neutral proteins, e.g. albumin, detergents, etc which may be used to facilitate optimal protein-protein binding and/or reduce non-specific or background interactions. Also reagents that otherwise improve the efficiency of the assay, such as protease inhibitors, nuclease inhibitors, anti-microbial agents, etc., may be used. The mixture of components may be added in any order that provides for the requisite binding.

Screening for agents that modulate the activity of caveolin-1 and/or caveolin-2 proteins may also be done. In a preferred embodiment, methods for screening for a bioactive agent capable of modulating the activity of caveolin-1 and/or caveolin-2 proteins comprise the steps of adding a candidate bioactive agent to a sample of caveolin-1 and/or caveolin-2 proteins, as above, and determining an alteration in the biological activity of caveolin-1 and/or caveolin-2 proteins. "Modulating the activity of a caveolin-1 and/or caveolin-2 protein" includes an increase in activity, a decrease in activity, or a change in the type or kind of activity present. Thus, in this embodiment, the candidate agent-should both bind to caveolin-1 and/or caveolin-2 proteins (although this may not be necessary), and alter its biological or biochemical activity as defined herein. The methods include both in vitro screening methods, as are generally outlined above, and in vivo screening of cells for alterations in the presence, distribution, activity or amount of caveolin-1 and/or caveolin-2 proteins.

Thus, in this embodiment, the methods comprise combining a caveolin-1 and/or caveolin-2 sample and a candidate bioactive agent, and evaluating the effect on caveolin-1 and/or caveolin-2 activity. By "caveolin-1 and/or caveolin-2 activity" or grammatical equivalents herein is meant one of the caveolin-1 and/or caveolin-2 protein's biological activities, including, but not limited to, its role in tumorigenesis, including cell division, preferably in lymphatic tissue, cell proliferation, tumor growth and transformation of cells.

In a preferred embodiment, the activity of the caveolin-1 and/or caveolin-2 protein is increased; in another preferred embodiment, the activity of the caveolin-1 and/or caveolin-2 protein is decreased. Thus, bioactive agents that are antagonists are preferred in some embodiments, and bioactive agents that are agonists may be preferred in other embodiments.

In a preferred embodiment, the invention provides methods for screening for bioactive agents capable of modulating the activity of a caveolin-1 and/or caveolin-2 protein. The methods comprise adding a candidate bioactive agent, as defined above, to a cell comprising caveolin-1 and/or caveolin-2 proteins. Preferred cell types include almost any cell. The cells contain a recombinant nucleic acid that encodes a caveolih-1 and/or caveolin-2 protein. In a preferred embodiment, a library of candidate agents is tested on a plurality of cells.

In one aspect, the assays are evaluated in the presence or absence or previous or subsequent exposure of physiological signals, for example hormones, antibodies, peptides, antigens, cytokines, growth factors, action potentials, pharmacological agents including chemotherapeutics, radiation, carcinogenic, or other cells (i.e. cell-cell contacts). In another example, the determinations are determined at different stages of the cell cycle process.

In this way, bioactive agents are identified. Compounds with pharmacological activity are able to enhance or interfere with the activity of the caveolin-1 and/or caveolin-2 proteins.

## Animal Models and Transgenics

In another preferred embodiment caveolin-1 and/or caveolin-2 genes find use in generating animal models of cancers. As is appreciated by one of ordinary skill in the art, gene therapy technology wherein antisense RNA directed to the caveolin-1 and/or caveolin-2 gene will diminish or repress expression of the gene. An animal generated as such serves as an animal model of caveolin-1 and/or caveolin-2 that finds use in screening bioactive drug candidates. Similarly, gene knockout technology, for example as a result of homologous recombination with an appropriate gene targeting vector, will result in the absence of the caveolin-1 and/or caveolin-2 protein. When desired, tissue-specific expression or knockout of the caveolin-1 and/or caveolin-2 protein may be necessary.

It is also possible that the caveolin-1 and/or caveolin-2 protein is overexpressed in cancer. As such, transgenic animals can be generated that overexpress the caveolin-1 and/or caveolin-2 protein. Depending on the desired expression level, promoters of various strengths can be employed to express the transgene. Also, the number of copies of the integrated transgene can be determined and compared for a determination of the expression level of the transgene. Animals generated by such methods find use as animal models of caveolin-1 and/or caveolin-2 and are additionally useful in screening for bioactive molecules to treat cancer.

The invention will be illustrated in more detail with reference to the following Examples, but it should be understood that the present invention is not deemed to be limited thereto.

## EXAMPLES

**Case Selection and Tissue Microarray (TMA) Construction.** Breast tissues for tissue microarray construction were obtained from the Surgical Pathology files at the University of Michigan with Institutional Review Board (IRB) approval. The TMA contained tissues derived from 154 largely consecutive patients with invasive carcinomas of the breast, with follow-up information treated at the University of Michigan from 1987-1991. Clinical and pathological variables were determined following well-established criteria. All invasive carcinomas were graded according to the method described by Elston and Ellis 17; Lymphovascular Invasion (LVI) was classified as either present or absent. The tissue microarrays were constructed, as previously described, using a tissue arrayer (Beecher Instruments. Silver Spring, MD). Three tissue cores (0.6 mm diameter) were sampled from each block to account for tumor and tissue heterogeneity and transferred to the recipient block. Only cases with two or three cores containing tumor stromal cells were considered for Statistical analysis in order to address possible heterogeneity of the staining in various tumor portions. Clinical and treatment information was extracted by chart review.

**Patients.** Our study population consists of 154 women diagnosed with breast cancer, with a median age of 59.5 years (range 28-96 years). 85% of the women were white. The median follow-up time for all survivors was 8.4 years (>30 days - 18.5 years). 45% of the subjects underwent Tamoxifen treatment after diagnosis, and 31% had a recurrence of breast cancer during follow-up. The median time to recurrence or death from any cause was 7.1 years.

**Immuno-histochemistry.** Cav-1 expression in the tumor stroma was assessed by employing a standard immuno-peroxidase method (DakoCytomation LSAB2 System- HRP, Carpinteria, CA), using rabbit polyclonal anti-Cav-1 IgG (N-20; directed against Nterminal residues, 2-21 of human Cav-1; Santa Cruz Biotechnology. Santa Cruz, CA; dilution 1:500). The staining was scored semi-quantitatively as negative (0; no staining), weak (1; either diffuse weak staining or strong staining in less than 30% of stromal cells per core) and strong (2; defined as strong staining of 30% or more of the stromal cells). These were given numerical raw scores of 0, 1 and 2, respectively, and the median score of 2-3 cores was taken as the final score of the sample.

**Statistical Analysis.** For each patient, the date of breast cancer diagnosis, date of last follow-up, vital status at last follow-up, causes of death (breast cancer or other), and breast cancer recurrence, were recorded. Stromal caveolin was scored for each tissue sample based on 3 cores taken from the sample and given a numeric score of 0,1 or 2, depending on the degree of stromal Cav-1 staining. The median of the three numeric scores was taken to be the stromal Cav-1 score for the sample. In the event that only two of the cores were scorable, and the median score was fractional, it was rounded upward to reflect the presence of stromal Cav-1. A median score of 0 was interpreted as an absence of stromal Cav-1, and scores of 1 and 2 were interpreted as the presence of stromal Cav-1. For an absence of stromal Cav-1 (final median score = 0), >70% of the patients had a raw score of 0 for all three sample cores (000) and >90% had a raw score of either 000 or 001, indicating strong consistency of this phenotype between all three patient tumor core samples.

Our primary outcome of interest in this study is progression-free survival (PFS) from time of diagnosis to the presence of metastasis, death, or last visit. PFS is evaluated using Kaplan-Meier estimation, and comparison of stratified survival curves was done using Jog-rank tests. Cox proportional hazard regression was used to evaluate the association of stromal Cay-1 with PFS, in the presence of various potential prognostic factors for PFS: Associations between the presence of stromal Cav-1 and other factors, including age, race, tumor grade, tumor size, lymph node status, histological subtype, estrogen receptor (ER), progesterone receptor (PR) and HER2, and presence of recurrent disease, were evaluated using Fisher's exact and Kruskal-Wallis tests, depending on the discrete or continuous nature of the other factors.

The default settings of the recursive partitioning function in R (rpart version 3.1-41; http://mayoresearch.mayo.edu/mayo/research/biostat/splusfunctions.cfm) was used to fit a survival tree model to the data and evaluate prognostic factors for PFS 20,21. All pvalues are two-sided, and p < 0.05 was considered significant. Statistical analysis was performed, and graphs constructed, using the R statistical analysis software version 2.7.2 22.

**Example 1 -** Clinicopathologic Features of the Specimens.

Of the 160 invasive carcinomas used to construct the TMA, 154 had at least 2 cores available for evaluation. Therefore, our study population consists of 154 women with a median age of 59.5 years (range 28-96 years). 85% of the women were white. The median follow-up time for all survivors was 8.4 years and the median time to metastasis, death, or last visit was 7.1 years. 45% of the subjects underwent Tamoxifen treatment after diagnosis, and 31% had a recurrence of breast cancer during follow-up.

**Example 2 -** ER, PCR, HER2, and Stromal Cav-1 Expression Analysis of the Specimens. One hundred forty patients were evaluated for ER. PR and HER2, of whom 66% were ER+ and 15% were triple-negative. One hundred and twenty five patients had samples that could be scored for stromal Cav-1. We established a Cav-1 grading scale (0, 1, and 2), with 0 representing an absence of stromal Cav-1 and 2 representing high levels of stromal Cav-1.37% of the samples showed a loss/or absence of stromal Cav-1 (score=0). A median score of 0 was interpreted as an absence of stromal Cav-1, and scores of 1 and 2 were interpreted as the presence of stromal Cav-1. Normal human breast tissue (TDLUs; terminal ductal lobular units) is shown for comparison purposes. Note that the intralobular Mammary stroma, the vasculature, and myo-epithelial cells are normally Cav-1 positive. Tables 2 and 3 show the relation of stromal Cav-1 expression to various clinico-pathological variables.

**Example 3 -** Stromal Cav-1 Expression Correlated to Pathologic Features. We find that an absence of stromal Cav-1 is strongly associated with tumor stage and nodal stage, as well as with recurrence rate and number of lymph node metastases. Loss of stromal Cav-1 is also significantly associated with lymphovascular invasion (LVI) (Table 4). In all cases, the absence

of Cav-1 is associated with markers of more aggressive disease (higher T-stage, higher N-stage, higher recurrence rate, more positive lymph nodes, and the presence of LVI) (Tables 2 and 4). For example, patients with stromal Cav-1 expression showed an ~3.6-fold reduction in disease recurrence and a -2- fold reduction in lymph node metastasis. Interestingly, patients with high stromal Cav-1 (score = 2) showed an ~5-fold reduction in disease recurrence and a ~2.6-fold reduction in lymph node metastasis (See Table 1). However, there was no association between stromal Cav-1 expression and tumor grade. Stromal Cav-1 was also not associated with ER, PR, HER2, or triple negative (ER-/PR-/HER2-) status, or with demographic parameters (Table 3).

**Example 4 -** Stromal Carr-1 Expression as a Clinically Relevant Biomarker. Lack of stromal Cav-1 was also seen to be an important prognostic factor for progression free survival (PFS). Figure 3 gives the median PFS for subjects with and without stromal Cav-1, in the presence of a.number of other potential prognostic factors. We find that an absence of stromal Cav-1 results in significantly lower PFS, even in the presence of other prognostic factors, with median survival reduced by several years in many cases even when adjusted for the same tumor grade. For example, the median PFS was 1.43 years versus 10.84 years in poorly-differentiated breast cancers, depending on the status of stromal Cav-1 (Table 5).

To highlight this, Figure 3 shows the Kaplan-Meier survival curves for patients who did and did not receive Tamoxifen therapy. Note that when only patients who underwent tamoxifen-treatment were selected for analysis (Figure 3 (Left panel)), an absence of Cav-1 in the mammary stroma was a strong predictor of poor clinical outcome, suggestive of an association with tamoxifen-resistance. In direct support of these IHCbased observations, virtually identical results were obtained when a "gene-expression signature", generated using Cav-1 (-/-) null mammary stromal fibroblasts, was used to cluster an independent cohort of ER(+) breast cancer patients who underwent tamoxifen mono-therapy.

Cox regression/multivariate analysis using T stage, N stage, Tamoxifen use, and the presence of stromal Cav-1 showed that an absence of stromal Cav-1 conferred significantly reduced PFS, with the adjusted hazard ratio being -3.6 (p < 0.0001). We used a survival tree approach to assess the relative importance of the presence of stromal Cav-1 in predicting PFS, using default settings in the R package rpart. Age, Race, T stage, N stage, ER, PR, HER2, Tamoxifen use and LVI were also included in this model. We find that an absence of stromal Cav-1 is the strongest factor in predicting PFS, even in the presence of other well-known predictor. Our analyses show that loss or absence of Cav-1 in stromal cells is an important independent predictor of progression-free survival in breast cancer, not associated with ER, PR or HER2 status.

Absence of stromal Cav-1 expression was also associated with dramatic reductions in 5- year Progression-Free Survival (PFS) (See Figure 1 for 5-year survival rates). Very similar results were also obtained using overall survival (See Supplemental Figure S1 at ajp.amjpathol.org). However, PFS is considered more of a cancer-specific measure of clinical outcome.

**Example 5 -** Stromal versus Epithelial Cav-1 as Predictive Breast Cancer Biomarkers. In order to assess the predictive value of epithelial Cav-1 expression, the same patient population was also scored for the expression of Cav-1 in the epithelial tumor cells, using the same scoring scheme as for stromal Cav-1 (0= absent; 1 or 2 = present). However, as presented in Figure 9, epithelial Cav-1 did not show any .correlation with patient clinical outcome. This is an important internal control for ourcunrent studies, and reinforces the idea that stromal expression of Cav-1 is a primary determinant of clinical outcome in breast cancer patients.

**Example 6 -** Status of Stromal Cav-1 in ER(+), PR(+), and HER2(+) Breast Cancer Patients. Historically, ER, PR, and HER2 expression have all served as important prognostic and predictive epithelial biomarkers for stratifying breast cancer patients into prognosis and therapy-relevant groups. Thus, we wondered whether stromal Cav-1 would function as a strong predictive biomarker in all three of these patient groups. Figures 10,11,12 show that regardless of epithelial marker status for ER, PR, or HER2, stromal Cav-1 serves as an important predictor of progression-free outcome. Thus, the status of stromal Cav-1 expression appears to be a critical predictor of clinical outcome that is clearly independent of epithelial marker status. The predictive value of epithelial Cav-1 is shown for comparison; it does not behave as a predictive biomarker in any of these patient groups.

**Example 6 -** Status of Stromal Cav-1 in Triple-Negative Breast Cancer Patients. Triple-negative breast cancers lack expression of the 3 most commonly used epithelial makers (ER-/PR-/HER2-), are generally poorly-differentiated, and are associated with poor clinical outcome. Thus, we examined the predictive value of stromal Cav-1 in triple-negative patients, within our patient population. Interestingly, stromal Cav-1 was also a strong predictor of progression-free outcome in triple negative breast cancer patients (Table 5). For example, the median PFS was 1.43 years versus 14.76 years in triple-negative patients, depending on the status of stromal Cav-1 (Table 5). However, epithelial Cav-1 did not show any predictive value in triple-negative patients (Figure 9). Thus, stromal Cav-1 is a powerful predictive biomarker for estimating a patient's risk of recurrence and survival in all of the 4 most common classes of breast cancer, which are based on ER, PR, and HER2 expression.

Additional data on ER(-), PR(-), low T stage, and grade 3 patients are provided as Supplemental Figure S2, S3, and S4 at ajp.amjpathol.org. In all these additional patient subgroups, an absence of stromal Cav-1 also consistently predicts poor clinical outcome.

**Example 7 -** Status of Stromal Cav-1 in Lymph Node Negative and Positive-Patients. Lymph node (LN) status is often used as a critical predictor of disease recurrence, metastasis, and survival in breast cancer patients. As an absence of stromal Cav-1 behaves as a predictor of disease recurrence and poor clinical outcome, we also assessed the predictive role of stromal Cav-1 in.LN(-) and LN (+) patients. Our results are shown in Figure 14. Note that in both LN(-) and LN(+) patients, an absence of stromal Cav-1 still remains a significant predictor of progression-free outcome. However, the results were most dramatic in LN(+) patients, where an absence of stromal Cav-1 is associated with an ~11.5-fold reduction in 5-year survival (Table 8).

Thus, the used of stromal Cav-1 as a predictive biomarker, especially in LN(+) patients, may allow for early interventions with more aggressive therapies.

**Example 8 -** Materials. Antibodies and their sources were as follow: phospho-Rb (pS807/811) from Cell Signaling; Rb (M-153), Cav-1 (N-20) and HGF beta from Santa Cruz Biotechnology; $\alpha$-smooth muscle actin and $\alpha$-actin from Sigma; Collagen type I from Novus Biologicals, CO; CAPER from BioVision. Other reagents were as follows: DAPI, Propidium Iodide, Prolong Gold Antifade Mounting Reagent. Slow-Fade Antifade Reagent (from Molecular Probes); Phalloidin-FITC, Hydrocortisone, Cholera Toxin, Insulin, and Gentamicin (from Sigma); Collagenase Type I (from Gibco); Reduced Growth Factor Matrigel (from Trevigen); and Lab-TekII 8-well chamber slides (from Nalgene Nunc).

**Example 9 -** Isolation and Primary Culture of Mammary Stromal Fibroblasts. Primary mammary fibroblasts were isolated from the mammary glands of 8-week old virgin mice. Briefly, the 4th and 5th mammary glands from WT and Cav-1 (-/-) mice were removed aseptically, minced with surgical blades, incubated in a shaker (for 2-3 hours at 37oC) in 30-35 ml of Digestion Media (DMEM/F12, 5% Horse Serum, 20 ng/ml EGF, 0.5 $\mu$g/ml Hydrocortisone, 100 ng/ml Cholera Toxin, 10 $\mu$g/ml Insulin, Pen/Strep) containing 2 mg/ml collagenase type 1, and 50 $\mu$g/ml gentamicin. Then, the cell suspensions were spun 10 min at 1,000 rpms to eliminate floating fat cells. Cell pellets were washed twice in 10 ml of MSF Growth Media (DMEM, 10% FBS, Pen/Strep). Then, cell pellets were disaggregated by pipetting up and down 10-15 times with a sterile 1-ml-blue-pipette-tip. Mammary fibroblasts were then cultured in Growth Media and passaged three times. At this point, greater than > 95% of the cells were mammary fibroblasts. At least 3 independent isolates of primary MSFs for each genotype were utilized for our experiments. Each of these cultures were derived from separate mice. These MSF cultures appeared very homogeneous and failed to express adipocyte (adiponectin), epithelial (keratin 8/18), and endothelial (CD31/Pecaml) cell markers. Under our culture conditions, which dramatically favor fibroblasts, other possible contaminating cell types (such as skeletal muscle and macrophages) fail to proliferate. Finally, >90% of the cells in these MSF cultures highly express fibroblastspecific markers, such as vimentin and collagen type L

**Example 10 -** Gene Expression Profiling. These studies were carried out essentially as we have previously described for other cell types. RNA was prepared from 3 WT and 3 Cav-1 (-/-) MSF isolates; each of these biological replicates were derived from separate mice. Total RNA (5 $\mu$g) was reverse transcribed using Superscript III First-Strand Synthesis System (Invitrogen) using a HPLC purified T7-dT24 primer (Sigma Genosys) which contains the T7 polymerase promoter sequence. The single stranded cDNA was converted to double stranded cDNA using DNA polymerase I (promega) and purified by cDNA spin column purification using GeneChip Sample Cleanup Module (Affymetrix). The double stranded cDNA was used as a template to generate biotinylated cRNA using Bioarray HighYield RNA Transcription Labeling Kit (Enzo) and the labeled cRNA purified by GeneChip Sample Cleanup Module (Affymetrix). 15 $\mu$ g of cRNA was fractionated to produce fragments of between 35-200 bp using 5x Fragmentation buffer provided in the Cleanup Module. The sample was hybridized to mouse 430 2.0 microarray (Affymetrix) representing over 39,000 transcripts. The hybridization and washing steps were carried out in accordance with Affymetrix protocols for eukaryotic arrays. The arrays were scanned at 570 nm with a confocal scanner from Affymetrix. Analysis of the arrays was performed using the R statistics package and the limma library of the Bioconductor software package 8, 9. Arrays were normalized using robust multiarray analysis (RMA), and P-value of 0.05 was applied as criteria for statistically differentially expressed genes.

**Example 11 -** Gene Array Data Analysis. Gene ontology analyses was performed using the DAVID 2007 bioinformatics resource. Microarray data (series GSE1378 and GSE1379) from X.J. Ma et al. 10 were obtained from the National Center for Biotechnology Information Gene Expression Omni bus website (www.ncbi.nlm.nih.gov/ geo/query/acc.cgi?acc=GPL1223) and manipulated using GeneSpring GX software (version 7.2) (Agilent Technologies). For each series, the raw data was obtained from GEO as $\log^2$ of normalized Cy5/Cy3 ratio, where tumor sample RNA and human universal reference RNA were labeled with Cy5 and Cy3, respectively.

The raw data were transformed from log2 to linear values followed by per-gene median normalization in GeneSpring. The expression levels of Cav-1 (-/-) MSF associated genes were clustered based on standard correlation as the similarity measurement. Subsequently, a condition tree based on distance correlation was created to order the tumor specimens. The patients exhibiting the highest expression level of the Cav-1 (-/-) MSF gene signature were utilized to define the impact of the Cav-I (-/-) MSF signature on disease outcome. For Kaplan-Meier analysis, statistical calculations were performed using GraphPad Prism 4.0 software. ES cell specific gene sets 11 and Estrogen-induced gene sets were as previously described.

**Example 12 -** Statistical Analysis of Overlapping Gene Sets. For determining the statistical significance of gene set overlap, we used p-values. We calculated the statistical significance for the transcriptome intersection by using hypergeometric probabilities for any two groups of genes. By considering the commonality between human and mouse platforms based upon identical transcript identifiers, we generated a p-value for the interesting sets. All comparisons were statistically significant at $p < 0.009$. In this case, the p-value is the probability of finding the number of overlapping genes in the two sets by pure chance.

This is determined by the equation:

$$p = 1 - \sum_{j=0}^{i-1} \frac{c(m, j)c(t - m, n - j)}{c(t, n)}.$$

p , where c(nj) is the number of combinations that one choose j objects from n objects, t is the total number of observable genes, i is the number of genes in the overlap, and m and n are the numbers of differentially expressed genes in the two sets. For the present comparisons (CAFs versus MSFs), t the number of observable genes was taken as number of common genes based on the Gene Symbol for the two chips MU74Av2 and HU133_2.0.

**Example 13 -** Other Statistical Considerations. The biological replicates demonstrate robust coherency within the phenotypic sample sets, inconsistent variation would result in an increased standard deviation and a high p-value. While we accept that based on a p-value < 0.05 the data set will have an inherent false positive element, we have compensated by using at least a two fold cut off. When the p-value of Cav-1 MSFs 832 transcript set is examined, we observe more than 3/4 of the differentially regulated genes have a p-value less than 0.005. A randomized Pearson correlation within biological replicates gave an r = 1, while between phenotypes gave an r = 0.6.

**Example 14 -** Target Validation by real-time PCR (RT-PCR). SYBR green real-time quantitative RTPCR. To independently quantify gene expression, 2000 ng of total RNA was reversetranscribed using random examers and Super-script-II reverse transcriptase (Invitrogen), according to the manufacturer's protocol. All primers were designed using Primer Express Software (Applied Biosystems, Foster City, CA) and validated for specificity. Real-time PCR was performed using the myIQ realtime PCR (biorad) according to the manufacturer's instructions. Reactions were conducted in triplicate and performed in a 25-$\mu$l volume with 50 nM of forward and reverse primer. The reaction cycles were: an initial incubation at 50°C for 2 min, denaturation at 95°C for 10 min, and 40 cycles of 95°C for 15 s and 60°C for 1 min. The SYBR green signal was continuously monitored. The amplified PCR products were analyzed in the linear range of amplification with standards. To confirm the amplification specificity, the PCR products were subjected to melting temperature dissociation curve analysis. In parallel, no amplification and no template controls was run to rule out the presence of fluorescence contaminants in the sample or in the thermal cycler heat block. Relative quantification of samples was assessed by arbitrarily setting the control cDNA value at 100, and changes in transcript levels of a sample are expressed as a multiple thereof (relative expression). Differences in the number of mRNA copies in each PCR reaction were normalized using mouse 18S rRNA transcript levels.

**Example 15 -** Target Validation by Western Blot or Immunofluorescence Analysis. As mRNA levels do not necessarily reflect protein expression.levels, we also performed target validation by Western Blot or immunofluorescence. Using this approach, we evaluated the protein expression status of a number of important genes including RB, phospho-RB, collagen I, CAPER, and HGF. Interestingly, total RB protein levels remained unchanged and HGF protein levels increased by ~10-fold in Cav-1 (-/-) MSFs. However, this is in contrast to our DNA microarray results, which showed that Rb1 transcripts decreased 2.0-fold and HGF transcripts decreased 2.1-fold. Thus, in these two cases, other gene or protein regulatory mechanisms may be operating. In contrast, analysis of phospho-RB, collagen I, and CAPER protein expression levels are concordant with the increased transcriptional expression of RB/E2F target genes (96 transcripts), as well as collagen I, and CAPER transcripts, in Cav-1 (-/-) MSFs. These results are also supported by other functional assays, such as BrdU incorporation and retraction/contraction analysis.

**Example 16 -** Assay for BrdU Incorporation. Cell proliferation was determined using a standard BrdU assay (Roche). The incorporation of a pyrimidine analogue (BrdU) was measured in WT and Cav-1 (-/-) MSFs, was suggested by the manufacturer. Briefly, fibroblasts were trypsinized and plated in a 96-well plate (Coming) at a density of 2,000 cells/well. After 72 hours, the cells were given a BrdU pulse of 2hrs at 37°C.

**Example 17-** Retraction/Contraction Assay. Passage 3 primary mammary fibroblasts were seeded at 50% confluency in 35 mm dishes and allowed to reach complete confluency in regular MSF growth media. Then, the cells were treated with ascorbic acid (40μg/ml). After 4 days, Cav-1 (-/-) MSFs showed a retraction phenotype, whereas WT cells remained completely attached to the plate.

**Example 18 -** Western Blot Analysis. Mammary fibroblast lysates were prepared by scraping the cells into Lysis Buffer (10mM TrisHCl pH 7.5, 50mM NaCl, 1% TritonX-100, 60mM octyl glucoside with Phosphatase Inhibitor cocktails (Sigma) and Protease Inhibitor Tablet). After rotation at 4°C for 40 min, cell lysates were spun for 10 min to remove insoluble material. Protein concentrations were assessed using the BCA assay kit. Cellular proteins were resolved by SDS-PAGE, and transferred to nitrocellulose membranes (Schleicher and Schuell, 0.2 μm). Blots were blocked for 1 hour in TBST (10 mM Tris-HCl, pH 8.0, 150 mM NaCl, 0.2% Tween 20) containing 1% bovine serum albumin (BSA) and 4% non-fat dry milk (Carnation). Then, membranes were incubated for 2 hours with primary antibodies in a 1%BSA/TBST solution. Membranes were then washed with TBST, and incubated for 40 min with the appropriate horseradish peroxidase-conjugated secondary antibodies (Pierce, diluted 5000-fold in 1 %BSA/ TBST). Signal was detected with an ECL detection kit (Pierce). Non-fat dry milk was omitted from the blocking solution when we employed phospho-specific antibodies.

**Example 19 -** Immunofluorescence. Cells were fixed for 30 min at RT in 2% PFA diluted in PBS, after which they were permeabilized for 10min at RT with IF Buffer (PBS + 0.2% BSA + 0.1% TritonX-100). Then, cells were incubated with NH4Cl in PBS to quench free aldheyde groups. Primary antibodies were incubated in IF Buffer overnight at RT. After washing with IF Buffer (3X, 10min each), cells were incubated for 30 min at RT with fluorocrome-conjugated secondary antibodies (Jackson Laboratories) diluted in IF Buffer. Finally slides were washed at RT with IF Buffer (3X, 10min each), and mounted with Slow-Fade Anti-fade Reagent (Molecular Probes). For Collagen I staining, passage 3 primary mammary fibroblasts were allowed to reach confluency, and were treated with ascorbic acid (40μg/ml) for 24 hours. Ascorbic acid treatment is required for collagen secretion. Then, cells were fixed and were immunostained with rabbit polyclonal antibodies against collagen I (Novus Biologicals, CO). Alternatively, a methanolfixation protocol was employed, as previously described. Methanol fixation was preferred for nuclear antigens, such phospho-RB and CAPER.

**Example 20 -** Preparation of Conditioned Media and 3D Mammary Culture Analysis. To prepare conditioned media, primary mammary fibroblasts were cultured until confluence. Confluent cultures were rinsed twice with serum-free medium and incubated in lowserum medium (DMEM, 10% Nu Serum, Glutamine, Pen-Strep) for 48 hours. Then, "MSF-conditioned media" was collected and incubated with 3D cultures of primary mammary epithelial cells. Primary mammary epithelial cells were purified, as we previously described. Briefly, after surgical and chemical isolation, mammary gland organoids were resuspended in Assay Media (DMEM/F12, 2% Horse Serum, 0.5 μg/ml Hydrocortisone, 100 ng/ml Cholera Toxin, 10 μg/ml Insulin, Pen/Strep). To wash away single cells, "organoid" pellets were subjected to repeated differential centrifugation (spun at 1,000 rpms for 45 sec; repeated for 10 cycles of pelleting and re-suspension). After the last wash, organoids were resuspended in 2 ml of Growth Media (DMEM/F12, 5% Horse Serum, 20 ng/ml EGF, 0.5 μg/ml Hydrocortisone, 100 ng/ml Cholera Toxin, 10 μg/ml Insulin, Pen/Strep), and disrupted by pipetting up and down 20-25 times with a sterile 1-ml-blue-pipette-tip. Organoids were plated and allowed to attach and spread as a monolayer. 4-5 days after purification, organoids attached to plastic dishes and grew as a mammary epithelial cell monolayer. Cell monolayers were then trypsinized. To obtain a single cell suspension, cells were passed 20-25X through a 1-ml-blue tip. This single cell suspension was then overlaid onto Matrigel, essentially as we previously described. Briefly, WT mammary epithelial cells were diluted in WT or Cav-1 (-/-) MSF-conditioned media supplemented with 2% Matrigel. Then, 5000 cells were overlaid on top of Matrigel (i.e., 8-well chamber slides, which were pre-coated with 40 μl of Matrigel). Chambers were placed in a standard cell culture incubator at 37°C. All experiments were performed with primary mammary epithelial cells that were passage.

**Example 21 -** Proteome Analysis of Secreted Proteins (ELISA). Levels of up to 40 different growth factors, cytokines, and chemokines in tissue culture supernatants were measured using SearchLight Proteome Arrays (Pierce Biotechnology, Woburn, MA). The SearchLight Proteome Array is a quantitative multiplexed sandwich ELISA containing different capture antibodies spotted on the bottom of a 96-well polystyrene microtiter plate. Each antibody captures specific protein present in the standards and samples added to the plate. The bound proteins are then detected with a biotinylated detection antibody, followed by the addition of streptavidin-horseradish peroxidase (HRP) and lastly, SuperSignal ELISA Femto Chemiluminescent substrate (U.S. Patent No. 6,432,662). The luminescent signal produced from the HRP-catalyzed oxidation of the substrate is measured by imaging the plate using the SearchLight Imaging System which is a cooled charge-coupled device (CCD) camera. The data is then analyzed using ArrayVision customized software. The amount of luminescent signal produced is proportional to the amount of each protein present in the original standard or sample. Concentrations are extrapolated from a standard curve.

**Example 22** - Identification of Endothelial and Pro-angiogenic Factors by RT-PCR. RNA was extracted from confluent mammary fibroblasts, grown in 10% FBS DMEM medium supplemented with 40 $\mu$g/ml ascorbic acid. RNA was extracted using RNAeasy columns (Quiagen) according to manufacturer's instructions including Dnase treatment to eliminate contamination, and retro-transcribed using RT2 first strand kit (Superarray) following the manufacturer's instructions. Mouse Angiogenesis and Cancer Pathway Finder RT2 Profiler PCR Arrays and RT2 Real-Timer SyBR Green/ROX PCR Mix were purchased from SuperArray Bioscience Corporation (Frederick, MD). For data analysis, the comparative Ct method was used employing the average of four housekeeping genes to normalize the values, according to Superarray's pre-developed software analysis; for each gene, fold-changes were calculated as the difference in gene expression between the average of expression of three different assays run on two separate preparations of WT and Cav-1 (-/-) MSFs.

**Example 23** - CD31 (Pecam1) Immunostaining. Mammary glands (inguinal) from six-month old virgin female WT and Cav-1 (-/-) mice were harvested and used to prepare frozen tissue sections. Frozen sections were subjected to fixation in acetone for 5 min at -20°C or 4 % paraformaldehyde in PBS for 10 min at 4°C. For immunohistochemical detection, a 3- step biotin-streptavidin-horseradish peroxidase method was employed after blocking with 10% rabbit serum. Tissue sections were then incubated overnight at 4 °C with rat antimouse CD31 (BD Biosciences, San Jose, CA) at a dilution of 1:200 (0.08 $\mu$g/ml) followed by biotinylated rabbit anti-rat IgG (1:200; Vector Labs, Burlingame, CA) and streptavidin-HRP (Dako, Carpinteria, CA). Immunoreactivity was revealed with 3, 3' diaminobenzidine. For immunofluorescence detection, CD31 antibody was used at a 1:50 dilution (0.3 $\mu$g/ml) after blocking with 10% goat serum. Sections were incubated for 1.5 hrs at room temperature and after washing, immunoreactivity was detected using goat anti-rat rhodamine red-X F(ab')2 (Jackson ImmunoResearch, West Grove, PA) at a 1:200 dilution (6.7 $\mu$g/ml). Images were collected with a Zeiss LSM510 meta confocal system using a 543 nm HeNe excitation laser and a detector band pass filter range of 560-615 nm.

Example 24 - Generation of Transgenic Animals Expressing Polypeptides as a Means for Testing Therapeutics. Caveolin-1 and/or caveolin-2 nucleic acids are used to generate genetically modified non-human animals, or site specific gene modifications thereof, in cell lines, for the study of function or regulation of prostate tumor-related genes, or to create animal models of diseases, including prostate cancer. The term "transgenic" is intended to encompass genetically modified animals having an exogenous caveolin-1 and/or caveolin-2 gene(s) that is stably transmitted in the host cells where the gene(s) may be altered in sequence to produce a modified protein, or having an exogenous LTR promoter operably linked to a reporter gene. Transgenic animals may be made through a nucleic acid construct randomly integrated into the genome. Vectors for stable integration include plasmids, retroviruses and other animal viruses, YACs, and the like. Of interest are transgenic mammals, e.g. cows, pigs, goats, horses, etc., and particularly rodents, e.g. rats, mice, etc.

The modified cells or animals are useful in the study of caveolin-1 and/or caveolin-2 gene function and regulation. For example, a series of small deletions and/or substitutions may be made in the caveolin-1 and/or caveolin-2 genes to determine the role of different genes in tumorigenesis. Specific constructs of interest include, but are not limited to, antisense constructs to block caveolin-1 and/or caveolin-2.gene expression, expression of dominant negative caveolin-1 and/or caveolin-2 gene mutations, and over-expression of a caveolin-1 and/or caveolin-2 gene. Expression of a caveolin-1 and/or caveolin-2 gene or variants thereof in cells or tissues where it is not normally expressed or at abnormal times of development is provided. In addition, by providing expression of proteins derived from caveolin-1 and/or caveolin-2 in cells in which it is otherwise not normally produced, changes in cellular behavior can be induced.

DNA constructs for random integration need not include regions of homology to mediate recombination. Conveniently, markers for positive and negative selection are included. For various techniques for transfecting mammalian cells, see Keown et al., Methods in Enzymology 185:527-537 (1990).

For embryonic stem (ES) cells, an ES cell line is employed, or embryonic cells are obtained freshly from a host, e.g. mouse, rat, guinea pig, etc. Such cells are grown on an appropriate fibroblast-feeder layer or grown in the presence of appropriate growth factors, such as leukemia inhibiting factor (LIF). When ES cells are transformed, they may be used to produce transgenic animals. After transformation, the cells are plated onto a feeder layer in an appropriate medium. Cells containing the construct may be detected by employing a selective medium. After sufficient time for colonies to grow, they are picked and analyzed for the occurrence of integration of the construct. Those colonies that are positive may then be used for embryo manipulation and blastocyst injection. Blastocysts are obtained from 4 to 6 week old superovulated females. The ES cells are trypsinized, and the modified cells are injected into the blastocoel of the blastocyst. After injection, the blastocysts are returned to each uterine horn of pseudopregnant females. Females are then allowed to go to term and the resulting chimeric animals screened for cells bearing the construct. By providing for a different phenotype of the blastocyst and the ES cells, chimeric progeny can be readily detected.

The chimeric animals are screened for the presence of the modified gene and males and females having the modification are mated to produce homozygous progeny. If the gene alterations cause lethality at some point in development, tissues or organs are maintained as allogeneic or congenic grafts or transplants, or in in vitro culture. The transgenic animals may be any non-human mammal, such as laboratory animals, domestic animals, etc. The transgenic animals are used in functional studies, drug screening, etc., e.g. to determine the effect of a candidate drug on prostate cancer, to test potential therapeutics or treatment regimens, etc.

**Claims**

1. An *in vitro* method for making a prognosis of disease course in a human neoplastic disease patient, the method comprising the step of determining the level of caveolin-1 and/or caveolin-2 protein expression in a sample of stromal cells obtained from stromal cells adjacent to a neoplasm; wherein said prognosis is made when the level of caveolin-1 and/or caveolin-2 protein expression in the stromal cells of the sample is lower than the level of caveolin-1 and/or caveolin-2 protein expression in a control.

2. The method of claim 1, wherein the human neoplastic disease patient has a neoplasm selected from the group consisting of breast, skin, kidney, lung, pancreas, rectum and colon, prostate, bladder, epithelial, non-epithelial, lymphomas, sarcomas, melanomas.

3. The method of any of claims 1-2, wherein the human neoplastic disease patient has a breast neoplasm subtype selected from the group consisting of ER(+), PR(+), HER2(+), triple-negative (ER(-)/PR(-)/HER2(-)), ER(-), PR(-), all tumor and nodal stages, and all tumor grades.

4. The method of any of claims 1-3, wherein the level of caveolin- 1 and/or caveolin-2 stromal expression is determined by immunohistochemical staining.

5. The method of any of claims 1-4, wherein the prognosis of disease course includes a risk for metastasis, recurrence and relapse of neoplastic disease.

6. The method of any of claims 1-5, wherein loss of stromal caveolin-1 and/or caveolin-2 predicts early disease recurrence, metastasis, survival, and tamoxifen-resistance at diagnosis.

7. The method of any of claims 1-6, wherein loss of stromal caveolin-1 and/or caveolin-2 predicts the prognosis of lymph-node positive (LN(+)) patients.

8. The method of any of claims 1-7, wherein loss or absence of stromal caveolin-1 and/or caveolin-2 is associated with a poor prognosis.

9. The method of any of claims 1-5, wherein the up-regulation or presence of stromal caveolin-1 and/or caveolin-2 is associated with a good prognosis.

10. The method of any of claims 1-9, wherein epithelial caveolin-1 expression is not predictive in any of the sub-types of breast neoplasm.

11. The method of any of claims 1-2, wherein the neoplasm is a pre-malignant lesion selected from the group consisting of ductal carcinoma in situ (DCIS) of the breast and myelodysplasia syndrome of the bone marrow.

12. The method of any of claims 1-11, wherein the prognosis of disease course includes staging malignant disease in a human neoplastic disease patient.

13. The method of claim 1, wherein (i) the patient is a LN(+) breast cancer patient, and (ii) the method further comprises determining that the patient's prognosis comprises an approximately 11.5-fold reduction in five-year survival, by determining that the patient lacks stromal Cav-1.

14. The method of claim 1, wherein (i) the patient is a breast cancer patient, and (ii) the method further comprises determining that the patient's prognosis comprises an approximately 3.6-fold reduction in progression-free survival, by determining that the patient lacks stromal Cav-1.

**Patentansprüche**

1. *In vitro*-Verfahren zum Erstellen einer Prognose für den Verlauf einer Erkrankung bei einem menschlichen Patienten mit einer neoplastischen Erkrankung, wobei das Verfahren den Schritt umfasst, den Spiegel der Caveolin-1- und Caveolin-2-Proteinexpression in einer Probe von Stromazellen zu bestimmen, die von an ein Neoplasma angrenzenden Stromazellen erhalten wurden; wobei die Prognose erstellt wird, wenn der Spiegel der Caveolin-1- und/oder der Caveolin-2-Proteinexpression in den Stromazellen der Probe niedriger ist als der Spiegel der Caveolin-1- und/oder Caveolin-2-Proteinexpression in einer Kontrolle.

2. Verfahren nach Anspruch 1, wobei der menschliche Patient mit einer neoplastischen Erkrankung ein Neoplasma hat, das ausgewählt ist aus der Gruppe bestehend aus Brust-, Haut-, Nieren-, Lungen-, Bauchspeicheldrüsen-, Rektum- und Colon-, Prostata-, Blasen-, Epithel- und Nicht-Epithelneoplasma, Lymphomen, Sarkomen, Melanomen.

3. Verfahren nach einem der Ansprüche 1 bis 2, wobei der menschliche Patient mit einer neoplastischen Erkrankung ein Brustneoplasma vom Subtyp hat, ausgewählt aus der Gruppe bestehend aus ER(+), PR(+), HER2(+), dreifachnegativem (ER(-)/PR(-)/HER2(-)), ER(-), PR(-), allen Tumor- und Knotenstadien und allen Tumorgraden.

4. Verfahren nach einem der Ansprüche 1 bis 3, wobei der Spiegel der stromalen Caveolin-1- und/oder Caveolin-2-Expression mittels immunhistochemischer Färbung bestimmt wird.

5. Verfahren nach einem der Ansprüche 1 bis 4, wobei die Prognose des Verlaufs der Erkrankung ein Risiko für Metastasierung, Wiederauftreten und Rückfall der neoplastischen Erkrankung einschließt.

6. Verfahren nach einem der Ansprüche 1 bis 5, wobei der Verlust von stromalem Caveolin-1 und/oder Caveolin-2 ein frühes Wiederauftreten der Erkrankung, Metastasierung, Überlebensrate und Tamoxifenresistenz bei Diagnosestellung prognostizieren.

7. Verfahren nach einem der Ansprüche 1 bis 6, wobei der Verlust an stromalem Caveolin-1 und/oder Caveolin-2 die Prognose von Lymphknoten-positiven (LN(+))-Patienten vorhersagt.

8. Verfahren nach einem der Ansprüche 1 bis 7, wobei der Verlust oder die Abwesenheit von stromalem Caveolin-1 und/oder Caveolin-2 mit einer schlechten Prognose in Zusammenhang steht.

9. Verfahren nach einem der Ansprüche 1 bis 5, wobei die Hochregulierung oder Anwesenheit von stromalem Caveolin-1 und/oder Caveolin-2 mit einer guten Prognose in Zusammenhang steht.

10. Verfahren nach einem der Ansprüche 1 bis 9, wobei die epitheliale Expression von Caveolin-1 in keinem der Brustneoplasmen-Subtypen prognostisch ist.

11. Verfahren nach einem der Ansprüche 1 bis 2, wobei das Neoplasma eine prämaligne Läsion ist, ausgewählt aus der Gruppe bestehend aus duktalem Karzinom in situ (DCIS, ductal carcinoma in situ) der Brust und myelodysplastischem Syndrom des Knochenmarks.

12. Verfahren nach einem der Ansprüche 1 bis 11, wobei die Prognose des Verlaufs der Erkrankung die Stadienbestimmung der malignen Erkrankung bei einem menschlichen Patienten mit einer neoplastischen Erkrankung umfasst.

13. Verfahren nach Anspruch 1, wobei (i) der Patient ein LN(+)-Brustkrebs-Patient ist und (ii) das Verfahren des Weiteren das Bestimmen, dass die Prognose des Patienten eine etwa 11,5-fache Verminderung der Fünf-Jahres-Überlebensrate umfasst, anhand der Bestimmung, dass der Patient einen Mangel an stromalem Cav-1 aufweist, umfasst.

14. Verfahren nach Anspruch 1, wobei (i) der Patient ein Brustkrebs-Patient ist und (ii) das Verfahren des Weiteren das Bestimmen, dass die Prognose des Patienten eine etwa 3,6-fache Verminderung der progressionsfreien Überlebensrate umfasst, anhand der Bestimmung, dass der Patient einen Mangel an stromalem Cav-1 aufweist, umfasst.

**Revendications**

1. Méthode *in vitro* pour faire un pronostic d'évolution d'une maladie chez un patient humain atteint d'une maladie néoplasique, la méthode comprenant l'étape de détermination du niveau d'expression de protéine cavéoline-1 et/ou cavéoline-2 dans un échantillon de cellules stromales obtenues à partir de cellules stromales adjacentes à un néoplasme ; dans laquelle ledit pronostic est fait quand le niveau d'expression de protéine cavéoline-1 et /ou cavéoline-2 dans les cellules stromales de l'échantillon est plus bas que le niveau d'expression de protéine cavéoline-1 et/ou cavéoline-2 dans un contrôle.

2.  Méthode selon la revendication 1, dans laquelle le patient humain atteint d'une maladie néoplasique a un néoplasme choisi dans le groupe constitué du sein, de la peau, du rein, du poumon, du pancréas, du rectum et du colon, de la prostate, de la vessie, épithélial, non épithélial, des lymphomes, des sarcomes, des mélanomes.

3.  Méthode selon l'une quelconque des revendications 1-2, dans laquelle le patient humain atteint d'une maladie néoplasique a un sous-type de néoplasme du sein choisi dans le groupe constitué de ER(+), PR(+), HER2(+), triple négatif (ER(-)/PR(-)/HER2(-)), ER(-), PR(-), tous les stades tumoraux et nodaux, et tous les grades tumoraux.

4.  Méthode selon l'une quelconque des revendications 1 à 3, dans laquelle le niveau d'expression stromale de cavéoline-1 et/ou cavéoline-2 est déterminé par coloration immunohistochimique.

5.  Méthode selon l'une quelconque des revendications 1 à 4, dans laquelle le pronostic d'évolution de la maladie inclut un risque de métastases, récidive et rechute de la maladie néoplasique.

6.  Méthode selon l'une quelconque des revendications 1 à 5, dans laquelle une perte de cavéoline-1 et/ou cavéoline-2 stromale prédit une récidive précoce de la maladie, une métastase, une survie, et une résistance au tamoxifène lors du diagnostic.

7.  Méthode selon l'une quelconque des revendications 1 à 6, dans laquelle une perte de cavéoline-1 et/ou cavéoline-2 stromale prédit le pronostic de patients positifs pour le noeud lymphatique (LN(+)).

8.  Méthode selon l'une quelconque des revendications 1 à 7, dans laquelle la perte ou l'absence de cavéoline-1 et/ou cavéoline-2 stromale est associée à un mauvais pronostic.

9.  Méthode selon l'une quelconque des revendications 1 à 5, dans laquelle la régulation ou la présence de cavéoline-1 et/ou cavéoline-2 stromale est associée à un bon pronostic.

10. Méthode selon l'une quelconque des revendications 1 à 9, dans laquelle l'expression épithéliale de cavéoline-1 n'est pas prédictive dans l'un quelconque des sous-types de néoplasme du sein.

11. Méthode selon l'une quelconque des revendications 1 à 2, dans laquelle le néoplasme est une lésion pré-maligne choisie dans le groupe constitué du carcinome canalaire in situ (CCIS) du sein et du syndrome myélodysplasique de la moelle osseuse.

12. Méthode selon l'une quelconque des revendications 1 à 11, dans laquelle le pronostic d'évolution de la maladie inclut la détermination du stade de la maladie maligne chez un patient humain atteint d'une maladie néoplasique.

13. Méthode selon la revendication 1, dans laquelle (i) le patient est un patient atteint d'un cancer du sein LN(+), et (ii) la méthode comprend en outre la détermination que le pronostic du patient comprend une réduction d'approximativement 11,5 fois de la survie à 5 ans, en déterminant que le patient manque de Cav-1 stromale.

14. Méthode selon la revendication 1, dans laquelle (i) le patient est un patient atteint d'un cancer du sein, et (ii) la méthode comprend en outre la détermination que le pronostic du patient comprend une réduction d'approximativement 3,6 fois de la survie sans progression, en déterminant que le patient manque de Cav-1 stromale.

Figure 1

Fig. 1B

Fig. 1A

Caveolin-1 WB

Up-regulated

Down-regulated

Fig. 1D

Fig. 1C

RB/E2F signature

Figure 2

Fig. 2A

Highest 33%

Fig. 2B

# Figure 3

## Fig. 3A

### Tamoxifen-Treated Patients

* p = 4.61 ×10⁻⁵; log rank test

## Fig. 3B

### No Tamoxifen Treatment

* p = 7.74 ×10⁻⁵; log rank test

EP 2 399 129 B1

Figure 4

Cav-1 (-/-) MSFs    CAF signature

325   55   63

Cav-1 (-/-) MEFs    CAF signature

445   8   58

EP 2 399 129 B1

# Figure 5

Fig. 5A

Fig. 5B

EP 2 399 129 B1

## Figure 6

Figure 7

Mammary Fibroblasts
WT  KO
HGF-β
β-actin

Figure 8

## Up-regulated Genes

Patient 1      Patient 2

p = 0.0040      p = 0.0085

158      91

202

82    Cav-1 (-/-) MSFs

p = 0.0054

Patient 3

### Overlapping Genes

$178/380 = 47\%$

## Down-regulated Genes

Patient 1      Patient 2

p = 0.0043      p = 0.0067

82      48

324

52    Cav-1 (-/-) MSFs

p = 0.0047

Patient 3

### Overlapping Genes

$127/452 = 28\%$

Figure 9

Total Patient Cohort

Fig. 9A

Stromal Cav-1

Fig. 9B

Epithelial Cav-1

\* p =1.77×10⁻⁹; log rank test

Figure 10

ER-Positive Patients

Fig. 10A

Stromal Cav-1

Fig. 10B

Epithelial Cav-1

* $p = 5.94 \times 10^{-7}$; log rank test

Figure 11

Fig. 11A

## PR-Positive Patients

Fig. 11B

### Stromal Cav-1

### Epithelial Cav-1

$^*$ p $=1.18\times10^{-5}$; log rank test

Figure 12

HER2-Positive Patients

Fig. 12A

Stromal Cav-1

Fig. 12B

Epithelial Cav-1

* p = 7.97×10⁻³; log rank test

## Figure 13

EP 2 399 129 B1

Fig. 13A                    Triple-Negative Patients                    Fig. 13B

### Stromal Cav-1                                         Epithelial Cav-1

* p = 2.01×10⁻²; log rank test

$$^* \ p = 2.01 \times 10^{-2}; \ \text{log rank test}$$

Figure 14

Stromal Cav-1 Status

Fig. 14A

LN-Negative

$* p = 6.44 \times 10^{-3}$; log rank test

Fig. 14B

LN-Positive

$* p = 1.14 \times 10^{-5}$; log rank test

# Figure 15

Figure 16

## Stromal Cav-1 Status

Fig. 16A

### ER-Negative

Fig. 16B

### PR-Negative

* p = 9.47 ×10$^{-3}$; log rank test

* p = 6.73 ×10$^{-4}$; log rank test

Figure 17

Fig. 17A    **Stromal Cav-1 Status**    Fig. 17B

T0/T1    T0/T1/T2

$^*$p = 2.94 ×10$^{-7}$; log rank test     $^*$p = 6.01 ×10$^{-7}$; log rank test

EP 2 399 129 B1

Figure 18

Stromal Cav-1 Status

Grade = 3 (Poorly-Differentiated)

* p = 9.32 ×10⁻⁵; log rank test

Figure 19

Cancer-Associated Fibroblast (CAF) Phenotype

Loss of Stromal Cav-1 Expression

↓

pRB Inactivation
(RB phosphorylation)

Myofibroblast
Proliferation

Growth Factor
Expression/Secretion

Tumor Angiogenesis

↓

Tumor Recurrence
and Metastasis

↓

Poor Clinical Outcome

Figure 20

Figure 20A

Figure 20B

Figure 20C

ER-Positive Patients

HER2-Positive Patients

Triple-Negative Patients

Figure 20D

Figure 20E

Figure 20F

Figure 21

Figure 21A

LN-Positive

Figure 21B

T0/T1

Figure 21C

Poorly-Differentiated

Figure 22

Stromal Classification of Human Breast Cancers

Cav-1 Immunostaining

⬇

Score Stromal Cav-1 Levels

Stromal Cav-1 (+)          Stromal Cav-1 (-)

⬇                          ⬇

Low-Risk                   High-Risk

⬇                          ⬇

Standard                   More Aggressive and/or
Therapy                    Anti-Angiogenic Therapy

Figure 23

Figure 23A                    Figure 23B

Figure 23C                    Figure 23D

Figure 24

Figure 24A

Figure 24B

Invasive recurrence

Invasive recurrence

Figure 25

Stromal Cav-1 Immuno-staining

Benign

PCa

Mets

Figure 26

Figure 27

Figure 28

Myo-fibroblast/
Cancer-Associated Fibroblast

Epithelial Cancer Cells
and Endothelia

Glycolysis

Glucose

PK-M2

Pyruvate

LDH

Lactate

MCT

Pyruvate
Lactate

Oxidative
Phosphorylation

TCA
Cycle

High ATP
Production

Pyruvate
Lactate

Energy
Production

Energy
Transfer

Tumor Growth and
Angiogenesis

Figure 29

Figure 29A

Figure 29B

**A** MDA-MB-231 Mammary Tumors

2.5-fold

Tumor Weight (grams)

Stromal Fibroblasts: WT Cav-1 KO

* p< 0.03 (Student© *t* test)
N = 8 tumors for each group

**B** WT Cav-1 KO

10X

20X

Figure 30

MDA-MB-231 Mammary Tumors

Vessel Area

p< 0.003 (Student's t test)
N = 4 tumors for each group

Figure 31

Cav-1

Vimentin

Figure 32

Figure 32A
**Fibro Alone**

Figure 32B
**Fibro + MCF-7**

Figure 32C

Figure 32D

Figure 33

MDA-MB-231 Mammary Tumors

Weight

4.5-fold

| Stromal Fibroblasts: | Cav-1 KO | Cav-1 KO |
| Treatment: | Vehicle Alone | 2-DG + DCA (200 mg/kg ea) |

* $p < 0.04$ (Student's t test)
N = 9 tumors for each group

Figure 34

## LDH-B

Figure 34A

Figure 34B

Figure 35

Figure 36

Figure 36A

**Control siRNA**

Figure 36B

**Cav-1 siRNA**

**Cav-1**

**Nitro-Tyrosine**

Figure 36C

Figure 36D

Figure 37

**Metformin + 2-DG Treatment**

Figure 38

Figure 38A

NFκB-Luc

Figure 38B

HIF-Luc

| Fold Change | 9.4 | 2.4 | -1.6 | -2.9 | | -- | -- | 1.9 | 4 |

Fibro-Luc Alone
Fibro-Luc + MCF-7

Figure 39

Figure 39A

Figure 39B

Figure 39C

Figure 40

Figure 41

Figure 42

MAMMARY FAT PAD INJECTION OF MET-1 CELLS

| Mammary Fat Pad | WT | WT | | KO | KO |
|---|---|---|---|---|---|
| Rapamycin Tx | -- | + | | -- | + |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5783182 A
- US 6252051 B
- US 5780454 A, Adams
- US 6066730 A
- US 6083903 A
- US 6297217 B
- US 6465433 B
- US 6548668 B
- US 6617317 B
- US 6747150 B
- WO 9930707 A, Siman
- WO 05021558 A, Bemareggi
- WO 05016859 A, Chatterjee
- US 20040167337 A, Furet
- US 02096933 A, Furet
- US 6018020 A, Attwood
- WO 04022070 A
- WO 04064755 A
- US 5693617 A, Stein
- WO 9113904 A, Siman
- WO 05105826 A, Iinuma
- US 6831099 B, Crews
- WO 05111008 A, Smyth
- WO 06045066 A, Bennett
- US 6310057 B, Chatterjee and Mallamo
- US 6096778 A
- US 6075150 A, Wang
- US 6781000 B
- WO 05030707 A, Baudy-Floc'h
- WO 03018557 A, Bonnemains
- US 20030166572 A, Furet
- WO 05115431 A, Papathanassiu
- US 5756764 A, Tenteany
- US 6147223 A
- US 6335358 B
- US 6645999 B
- WO 05003137 A, Fenical
- WO 05002572 A, Palladino
- WO 04071382 A, Stadler
- US 2005023162 A, Xiao and Patel
- WO 05099687 A, Corey
- US 20040186167 A, Dou
- US 5885530 A
- US 4981785 A
- US 6159750 A
- US 5358691 A
- US 5681702 A
- US 5597909 A
- US 5545730 A
- US 5594117 A
- US 5591584 A
- US 5571670 A
- US 5580731 A
- US 5624802 A
- US 5635352 A
- US 5594118 A
- US 5359100 A
- US 5124246 A
- US 5681697 A
- US 9701019 W
- US 6432662 B

**Non-patent literature cited in the description**

- **ISABELLE MERCIER et al.** Human breast cancer-associated fibroblasts (CAFs) show caveolin-1 down-regulation and RB tumor suppressor functional inactivation: Implications for the response to hormonal therapy. *CANCER BIOLOGY & THERAPY,* 01 August 2008, vol. 7 (8), 1212-1225
- **BEDDEK et al.** the proteomics of the lactating mammary gland, and is based on the KEGG pathway database. *Proteomics,* 2008, vol. 8, 1502-1515, www.genome.jp/kegg/pathway.html
- **LI et al.** *J. Biol. Chem.,* 1996, vol. 271, 29182-90
- **ANDERSON.** *Annu. Rev. Biochem.,* 1998, vol. 67, 199-255
- **ENGELMANN et al.** *FEBS letters,* 1998, vol. 436, 403-410
- **KOLESKE et al.** *Proc. Nad. Acad. Sci. USA,* 1995, vol. 92, 1381-1385
- **LEE, S. W. et al.** *Oncogene,* 1995, vol. 16, 1391-1397
- **RACINE C. et al.** *Biochem. Biophys. Res. Commun.,* 1999, vol. 255, 580-586
- *Prostate,* 15 February 2001, vol. 46 (3), 249-56
- *FEBS. Lett.,* 09 April 1999, vol. 448 (2-3), 221-30
- **BORING, C: C. et al.** *CA Cancer J. Chin.,* 1993, vol. 43, 7

- **WITKIEWICZ et al.** *Cell Cycle,* 2009, vol. 8, 1654-8
- **WITKIEWICZ et al.** *Cancer Biol Ther,* 2009, vol. 8, 1167-75
- **DI VIZIO et al.** *Cell Cycle,* 2009, vol. 8, 2420-4
- **LEI et al.** *Teratog Carcinog Mutagen,* 2002, vol. 22, 377-83
- **JOSEPH et al.** *J Biol Chem,* 2002, vol. 277, 6131-6
- **SALERNO et al.** *Clin Exp Metastasis,* 2003, vol. 20, 3-10
- **CHRISTOFK et al.** *Nature,* 2008, vol. 452, 230-3
- **CHRISTOFK et al.** *Nature,* 2008, vol. 452, 181-6
- **LEE et al.** *Int J Biochem Cell Biol,* 2008, vol. 40, 1043-54
- **KERBEL et al.** *Nature Reviews,* October 2002, vol. 2, 727
- **FRY, D.W. et al.** Specific inhibition of cyclin-dependent kinase 4/6 by PD 0332991 and associated antitumor activity in human tumor xenografts. *Mol Cancer Ther.,* 2004, vol. 3, 1427-1438
- **OIKAWA.** *Cancer Lett.,* 1988, vol. 43, 85
- **HARAGUCHI.** *Cancer Res.,* 1993, vol. 53, 5680-5682
- **YAYOI.** *Int J Oncol.,* 1994, vol. 5, 27-32
- **YAMAMOTO.** *Oncol Reports,* 1995, vol. 2, 793-796
- **IQBAL et al.** *J. Med. Chem.,* 1995, vol. 38, 2276-2277
- **SPALTENSTEIN et al.** *Tetrahedron Lett.,* 1996, vol. 37, 1343
- **MENG.** *Proc. Natl. Acad. Sci.,* 1999, vol. 96, 10403
- **MENG.** *Cancer Res.,* 1999, vol. 59, 2798
- **MARASTONI et al.** *J. Med. Chem.,* 2005, vol. 48, 5038
- **RYDZEWSKI et al.** *J. Med. Chem.,* 2006, vol. 49, 2953
- **BOGYO et al.** *Proc. Natl. Acad. Sci.,* 1997, vol. 94, 6629
- **BOUGET et al.** *Bioorg, Med. Chem.,* 2003, vol. 11, 4881
- **FENTEANY et al.** *Proc. Natl. Acad. Sci. USA,* 1994, vol. 91, 3358
- **KOGUCHI.** *Antibiot. (Tokyo),* 2000, vol. 53, 105
- **KROLL M.** *Chem. Biol.,* 1999, vol. 6, 689
- **NAM S.** *J. Biol. Chem.,* 2001, vol. 276, 13322
- **NAM et al.** *J. Biol. Chem.,* 2001, vol. 276, 13322
- Remington's Pharmaceutical Science. Mack Publishing Co,
- **O'REILLY et al.** Angiogenic Regulation of Metastatic Growth. *Cell,* 21 October 1994, vol. 79 (2), 315-328
- **YU et al.** *PNAS,* 2004, vol. 101 (21), 8005-8010
- **KOEHLER ; MILSTEIN.** *Nature,* 1975, vol. 256, 495
- **ZLOKARNIK et al.** *Science,* 1998, vol. 279, 84-8
- **HEID et al.** *Genome Res.,* 1996, vol. 6, 986-994
- **KEOWN et al.** *Methods in Enzymology,* 1990, vol. 185, 527-537